# EUROPEAN PATENT APPLICATION

(11) **EP 2 584 042 A1**
(43) Date of publication of application: **24.04.2013**
(21) Application number: 11008353.2
(22) Date of filing: 17.10.2011
(51) Int. Cl.: C12N 15/82, C12P 7/08

(54) **Production, storage and use of cell wall-degrading enzymes**

(71) Applicant: Nomad Bioscience GmbH, 80333 München (DE)
(72) Inventor: Hahn, Simone, 06114 Halle (DE); Giritch, Anatoli, 06108 Halle, (DE); Gleba, Yuri, 10117 Berlin (DE)
(74) Representative: Blodig, Wolfgang

(57) **Abstract**

A process of producing a preparation of cell wall-degrading enzymes, comprising: expressing in plants or plant parts one or more than one heterologous cell wall-degrading enzyme; harvesting said plants or plant part containing said one or more than one cell wall-degrading enzyme; and storing the harvest as a silage.

SEQUENCE LISTING

<110>  Nomad Bioscience GmbH

<120>  Production, storage and use of cell wall-degrading enzymes

<130>  EAD-15635

<160>  8

<170>  PatentIn version 3.3

<210>  1
<211>  10921
<212>  DNA
<213>  Artificial

<220>
<223>  T-DNA of 5' vector module pICH22455

<400>  1
tgatgggctg cctgtatcga gtggtgattt tgtgccgagc tgccggtcgg ggagctgttg      60

gctggctggt ggcaggatat attgtggtgt aaacaaattg acgcttagac aacttaataa     120

cacattgcgg acgtttttaa tgtactgggg tggatgcagg tcgatctagt aacatagatg     180

acaccgcgcg cgataattta tcctagtttg cgcgctatat tttgttttct atcgcgtatt     240

aaatgtataa ttgcgggact ctaatcataa aaacccatct cataaataac gtcatgcatt     300

acatgttaat tattacatgc ttaacgtaat tcaacagaaa ttatatgata atcatcgcaa     360

gaccggcaac aggattcaat cttaagaaac tttattgcca aatgtttgaa cgatctgctt     420

gactctagat ccagagtccc gctcagaaga actcgtcaag aaggcgatag aaggcgatgc     480

gctgcgaatc gggagcggcg ataccgtaaa gcacgaggaa gcggtcagcc cattcgccgc     540

caagctcttc agcaatatca cgggtagcca acgctatgtc ctgatagcgg tccgccacac     600

ccagccggcc acagtcgatg aatccagaaa agcggccatt ttccaccatg atattcggca     660

agcaggcatc gccatgagtc acgacgagat cctcgccgtc gggcatacgc gccttgagcc     720

tggcgaacag ttcggctggc gcgagcccct gatgctcttc gtccagatca tcctgatcga     780

caagaccggc ttccatccga gtacgtgctc gctcgatgcg atgtttcgct tggtggtcga     840

atgggcaggt agccggatca agcgtatgca gccgccgcat tgcatcagcc atgatggata     900

ctttctcggc aggagcaagg tgagatgaca ggagatcctg ccccggcact tcgcccaata     960

gcagccagtc ccttcccgct tcagtgacaa cgtcgagcac agctgcgcaa ggaacgcccg    1020

tcgtggccag ccacgatagc cgcgctgcct cgtcctggag ttcattcagg gcaccggaca    1080

ggtcggtctt gacaaaaaga accgggcgcc cctgcgctga cagccggaac acggcggcat    1140

cagagcagcc gattgtctgt tgtgcccagt catagccgaa tagcctctcc acccaagcgg    1200

ccggagaacc tgcgtgcaat ccatcttgtt caatcatgcg aaacgatcca gatccggtgc    1260

agattatttg gattgagagt gaatatgaga ctctaattgg ataccgaggg gaatttatgg    1320

aacgtcagtg gagcattttt gacaagaaat atttgctagc tgatagtgac cttaggcgac    1380

ttttgaacgc gcaataatgg tttctgacgt atgtgcttag ctcattaaac tccagaaacc 1440
cgcggctgag tggctccttc aacgttgcgg ttctgtcagt tccaaacgta aaacggcttg 1500
tcccgcgtca tcggcggggg tcataacgtg actcccttaa ttctccgctc atggtaccac 1560
gcgtttcgac aaaatttaga acgaacttaa ttatgatctc aaatacattg atacatatct 1620
catctagatc taggttatca ttatgtaaga aagttttgac gaatatggca cgacaaaatg 1680
gctagactcg atgtaattgg tatctcaact caacattata cttataccaa acattagtta 1740
gacaaaattt aaacaactat tttttatgta tgcaagagtc agcatatgta taattgattc 1800
agaatcgttt tgacgagttc ggatgtagta gtagccatta tttaatgtac atactaatcg 1860
tgaatagtga atatgatgaa acattgtatc ttattgtata aatatccata aacacatcat 1920
gaaagacact ttctttcacg gtctgaatta attatgatac aattctaata gaaaacgaat 1980
taaattacgt tgaattgtat gaaatctaat tgaacaagcc aaccacgacg acgactaacg 2040
ttgcctggat tgactcggtt taagttaacc actaaaaaaa cggagctgtc atgtaacacg 2100
cggatcgagc aggtcacagt catgaagcca tcaaagcaaa agaactaatc caagggctga 2160
gatgattaat tagtttaaaa attagttaac acgagggaaa aggctgtctg acagccaggt 2220
cacgttatct ttacctgtgg tcgaaatgat tcgtgtctgt cgattttaat tatttttttg 2280
aaaggccgaa aataaagttg taagagataa acccgcctat ataaattcat atattttcct 2340
ctccgctttg aagttttagt tttattgcaa caacaacaac aaattacaat aacaacaaac 2400
aaaatacaaa caacaacaac atggcacaat ttcaacaaac aattgacatg caaactctcc 2460
aagccgctgc gggacgcaac agcttggtga atgatttggc atctcgtcgc gtttacgata 2520
atgcagtcga ggagctgaat gctcgttcca gacgtcccaa ggtaatagga actttctgga 2580
tctactttat ttgctggatc tcgatcttgt tttctcaatt tccttgagat ctggaattaa 2640
ttcgtttaat ttggatctgt gaacctccac taaatctttt ggttttacta gaatcgatct 2700
aagttgaccg atcagttagc tcgattatag ctaccagaat ttggcttgac cttgatggag 2760
agatccatgt tcatgttacc tgggaaatga tttgtatatg tgaattgaaa tctgaactgt 2820
tgaagttaga ttgaatctga acactgtcaa tgttagattg aatctgaaca ctgtttaagg 2880
ttagatgaag tttgtgtata gattcttcga aactttagga tttgtagtgt cgtacgttga 2940
acagaaagct atttctgatt caatcagggt ttatttgact gtattgaact ctttttgtgt 3000
gtttgcaggt ccacttctcc aaggcagtgt ctacggaaca gaccctgatt gcaacaaacg 3060
catatccgga gttcgagatt tcctttactc atacgcaatc cgctgtgcac tccttggccg 3120
gaggccttcg gtcacttgag ttggagtatc tcatgatgca agttccgttc ggttctctga 3180
cgtacgacat cggcggtaac ttttccgcgc accttttcaa agggcgcgat tacgttcact 3240
gctgcatgcc taatctggat gtacgtgaca ttgctcgcca tgaaggacac aaggaagcta 3300
tttacagtta tgtgaatcgt ttgaaaaggc agcagcgtcc tgtgcctgaa taccagaggg 3360
cagctttcaa caactacgct gagaacccgc acttcgtcca ttgcgacaaa cctttccaac 3420

agtgtgaatt gacgacagcg tatggcactg acacctacgc tgtagctctc catagcattt 3480
atgatatccc tgttgaggag ttcggttctg cgctactcag gaagaatgtg aaaacttgtt 3540
tcgcggcctt tcatttccat gagaatatgc ttctagattg tgatacagtc acactcgatg 3600
agattggagc tacgttccag aaatcaggta acattcctta gttacctttc ttttcttttt 3660
ccatcataag tttatagatt gtacatgctt tgagattttt ctttgcaaac aatctcaggt 3720
gataacctga gcttcttctt ccataatgag agcactctca attacaccca cagcttcagc 3780
aacatcatca agtacgtgtg caagacgttc ttccctgcta gtcaacgctt cgtgtaccac 3840
aaggagttcc tggtcactag agtcaacact tggtactgca agttcacgag agtggatacg 3900
ttcactctgt tccgtggtgt gtaccacaac aatgtggatt gcgaagagtt ttacaaggct 3960
atggacgatg cgtggcacta caaaaagacg ttagcaatgc ttaatgccga gaggaccatc 4020
ttcaaggata acgctgcgtt aaacttctgg ttcccgaagg tgctcttgaa attggaagtc 4080
ttcttttgtt gtctaaacct atcaatttct ttgcggaaat ttatttgaag ctgtagagtt 4140
aaaattgagt cttttaaact tttgtaggtg agagacatgg ttatcgtccc tctctttgac 4200
gcttctatca caactggtag gatgtctagg agagaggtta tggtgaacaa ggacttcgtc 4260
tacacggtcc taaatcacat caagacctat caagctaagg cactgacgta cgcaaacgtg 4320
ctgagcttcg tggagtctat taggtctaga gtgataatta acggtgtcac tgccaggtaa 4380
gttgttactt atgattgttt tcctctctgc tacatgtatt ttgttgttca tttctgtaag 4440
atataagaat tgagttttcc tctgatgata ttattaggtc tgaatgggac acagacaagg 4500
caattctagg tccattagca atgacattct tcctgatcac gaagctgggt catgtgcaag 4560
atgaaataat cctgaaaaag ttccagaagt tcgacagaac caccaatgag ctgatttgga 4620
caagtctctg cgatgccctg atgggggtta ttccctcggt caaggagacg cttgtgcgcg 4680
gtggttttgt gaaagtagca gaacaagcct tagagatcaa ggttagtatc atatgaagaa 4740
atacctagtt tcagttgatg aatgctattt tctgacctca gttgttctct tttgagaatt 4800
atttcttttc taatttgcct gatttttcta ttaattcatt aggttcccga gctatactgt 4860
accttcgccg accgattggt actacagtac aagaaggcgg aggagttcca atcgtgtgat 4920
ctttccaaac ctctagaaga gtcagagaag tactacaacg cattatccga gctatcagtg 4980
cttgagaatc tcgactcttt tgacttagag gcgtttaaga ctttatgtca gcagaagaat 5040
gtggacccgg atatggcagc aaaggtaaat cctggtccac acttttacga taaaaacaca 5100
agattttaaa ctatgaactg atcaataatc attcctaaaa gaccacactt ttgttttgtt 5160
tctaaagtaa tttttactgt tataacaggt ggtcgtagca atcatgaagt cagaattgac 5220
gttgcctttc aagaaaccta cagaagagga aatctcggag tcgctaaaac caggagaggg 5280
gtcgtgtgca gagcataagg aagtgttgag cttacaaaat gatgctccgt tcccgtgtgt 5340
gaaaaatcta gttgaaggtt ccgtgccggc gtatggaatg tgtcctaagg gtggtggttt 5400

cgacaaattg gatgtggaca ttgctgattt ccatctcaag agtgtagatg cagttaaaaa 5460
gggaactatg atgtctgcgg tgtacacagg gtctatcaaa gttcaacaaa tgaagaacta 5520
catagattac ttaagtgcgt cgctggcagc tacagtctca aacctctgca aggtaagagg 5580
tcaaaaggtt tccgcaatga tccctctttt tttgtttctc tagtttcaag aatttgggta 5640
tatgactaac ttctgagtgt tccttgatgc atatttgtga tgagacaaat gtttgttcta 5700
tgttttaggt gcttagagat gttcacggcg ttgacccaga gtcacaggag aaatctggag 5760
tgtgggatgt taggagagga cgttggttac ttaaacctaa tgcgaaaagt cacgcgtggg 5820
gtgtggcaga agacgccaac cacaagttgg ttattgtgtt actcaactgg gatgacggaa 5880
agccggtttg tgatgagaca tggttcaggg tggcggtgtc aagcgattcc ttgatatatt 5940
cggatatggg aaaacttaag acgctcacgt cttgcagtcc aaatggtgag ccaccggagc 6000
ctaacgccaa agtaattttg gtcgatggtg ttcccggttg tggaaaaacg aaggagatta 6060
tcgaaaaggt aagttctgca tttggttatg ctccttgcat tttaggtgtt cgtcgctctt 6120
ccatttccat gaatagctaa gatttttttt ctctgcattc attcttcttg cctcagttct 6180
aactgtttgt ggtatttttg ttttaattat tgctacaggt aaacttctct gaagacttga 6240
ttttagtccc tgggaaggaa gcttctaaga tgatcatccg gagggccaac caagctggtg 6300
tgataagagc ggataaggac aatgttagaa cggtggattc cttcttgatg catccttcta 6360
gaagggtgtt taagaggttg tttatcgatg aaggactaat gctgcataca ggttgtgtaa 6420
atttcctact gctgctatct caatgtgacg tcgcatatgt gtatggggac acaaagcaaa 6480
ttccgttcat ttgcagagtc gcgaactttc cgtatccagc gcattttgca aaactcgtcg 6540
ctgatgagaa ggaagtcaga agagttacgc tcaggtaaag caactgtgtt ttaatcaatt 6600
tcttgtcagg atatatggat tataacttaa tttttgagaa atctgtagta tttggcgtga 6660
aatgagtttg ctttttggtt tctcccgtgt tataggtgcc cggctgatgt tacgtatttc 6720
cttaacaaga agtatgacgg ggcggtgatg tgtaccagcg cggtagagag atccgtgaag 6780
gcagaagtgg tgagaggaaa gggtgcattg aacccaataa ccttaccgtt ggagggtaaa 6840
attttgacct tcacacaagc tgacaagttc gagttactgg agaagggtta caaggtaaag 6900
tttccaactt tcctttacca tatcaaacta aagttcgaaa ctttttattt gatcaacttc 6960
aaggccaccc gatctttcta ttcctgatta atttgtgatg aatccatatt gacttttgat 7020
ggttacgcag gatgtgaaca ctgtgcacga ggtgcaaggg gagacgtacg agaagactgc 7080
tattgtgcgc ttgacatcaa ctccgttaga gatcatatcg agtgcgtcac ctcatgtttt 7140
ggtggcgctg acaagacaca caacgtgttg taaatattac accgttgtgt tggacccgat 7200
ggtgaatgtg atttcagaaa tggagaagtt gtccaatttc cttcttgaca tgtatagagt 7260
tgaagcaggt ctgtctttcc tatttcatat gtttaatcct aggaatttga tcaattgatt 7320
gtatgtatgt cgatcccaag actttcttgt tcacttatat cttaactctc tctttgctgt 7380
ttcttgcagg tgtccaatag caattacaaa tcgatgcagt attcagggga cagaacttgt 7440

ttgttcagac gcccaagtca ggagattggc gagatatgca attttactat gacgctcttc 7500
ttcccggaaa cagtactatt ctcaatgaat ttgatgctgt tacgatgaat ttgagggata 7560
tttccttaaa cgtcaaagat tgcagaatcg acttctccaa atccgtgcaa cttcctaaag 7620
aacaacctat tttcctcaag cctaaaataa gaactgcggc agaaatgccg agaactgcag 7680
gtaaaatatt ggatgccaga cgatattctt tcttttgatt tgtaactttt tcctgtcaag 7740
gtcgataaat tttatttttt ttggtaaaag gtcgataatt tttttttgga gccattatgt 7800
aattttccta attaactgaa ccaaaattat acaaaccagg tttgctggaa aatttggttg 7860
caatgatcaa aagaaacatg aatgcgccgg atttgacagg gacaattgac attgaggata 7920
ctgcatctct ggtggttgaa aagttttggg attcgtatgt tgacaaggaa tttagtggaa 7980
cgaacgaaat gaccatgaca agggagagct tctccaggta aggacttctc atgaatatta 8040
gtggcagatt agtgttgtta aagtctttgg ttagataatc gatgcctcct aattgtccat 8100
gttttactgg ttttctacaa ttaaaggtgg ctttcgaaac aagagtcatc tacagttggt 8160
cagttagcgg actttaactt tgtggatttg ccggcagtag atgagtacaa gcatatgatc 8220
aagagtcaac caaagcaaaa gttagacttg agtattcaag acgaatatcc tgcattgcag 8280
acgatagtct accattcgaa aaagatcaat gcgattttcg gtccaatgtt ttcagaactt 8340
acgaggatgt tactcgaaag gattgactct tcgaagtttc tgttctacac cagaaagaca 8400
cctgcacaaa tagaggactt cttttctgac ctagactcaa cccaggcgat ggaaattctg 8460
gaactcgaca tttcgaagta cgataagtca caaaacgagt tccattgtgc tgtagagtac 8520
aagatctggg aaaagttagg aattgatgag tggctagctg aggtctggaa acaaggtgag 8580
ttcctaagtt ccattttttt gtaatccttc aatgttattt taacttttca gatcaacatc 8640
aaaattaggt tcaattttca tcaaccaaat aatatttttc atgtatatat aggtcacaga 8700
aaaacgacct tgaaagatta tacggccgga atcaaaacat gtctttggta tcaaaggaaa 8760
agtggtgatg tgacaacctt tattggtaat accatcatca ttgccgcatg tttgagctca 8820
atgatcccca tggacaaagt gataaaggca gctttttgtg gagacgatag cctgatttac 8880
attcctaaag gtttagactt gcctgatatt caggcgggcg cgaacctcat gtggaacttc 8940
gaggccaaac tcttcaggaa gaagtatggt tacttctgtg gtcgttatgt tattcaccat 9000
gatagaggag ccattgtgta ttacgatccg cttaaactaa tatctaagtt aggttgtaaa 9060
catattagag atgttgttca cttagaagag ttacgcgagt ctttgtgtga tgtagctagt 9120
aacttaaata attgtgcgta tttttcacag ttagatgagg ccgttgccga ggttcataag 9180
accgcggtag gcggttcgtt tgctttttgt agtataatta agtatttgtc agataagaga 9240
ttgtttagag atttgttctt tgtttgataa tgtcgatagt ctcgtacgaa cctaaggtga 9300
gtgatttcct caatctttcg aagaaggaag agatcttgcc gaaggctcta acgaggttaa 9360
aaaccgtgtc tattagtact aaagatatta tatctgtcaa ggagtcggag actttgtgtg 9420

atatagattt gttaatcaat gtgccattag ataagtatag atatgtgggt atcctaggag 9480

ccgtttttac cggagagtgg ctagtgccag acttcgttaa aggtggagtg acgataagtg 9540

tgatagataa gcgtctggtg aactcaaagg agtgcgtgat tggtacgtac agagccgcag 9600

ccaagagtaa gaggttccag ttcaaattgg ttccaaatta ctttgtgtcc accgtggacg 9660

caaagaggaa gccgtggcag gtaaggattt ttatgatata gtatgcttat gtattttgta 9720

ctgaaagcat atcctgcttc attgggatat tactgaaagc atttaactac atgtaaactc 9780

acttgatgat caataaactt gattttgcag gttcatgttc gtatacaaga cttgaagatt 9840

gaggcgggtt ggcagccgtt agctctggaa gtagtttcag ttgctatggt caccaataac 9900

gttgtcatga agggtttgag ggaaaaggtc gtcgcaataa atgatccgga cgtcgaaggt 9960

ttcgaaggta agccatcttc ctgcttattt ttataatgaa catagaaata ggaagttgtg 10020

cagagaaact aattaacctg actcaaaatc taccctcata attgttgttt gatattggtc 10080

ttgtattttg caggtgtggt tgacgaattc gtcgattcgg ttgcagcatt taaagcggtt 10140

gacaacttta aaagaaggaa aaagaaggtt gaagaaaagg gtgtagtaag taagtataag 10200

tacagaccgg agaagtacgc cggtcctgat tcgtttaatt tgaaagaaga aaacgtctta 10260

caacattaca aacccgaatc agtaccagta tttcgataag aaacaagaaa ccatgggcag 10320

cagccaccac caccaccacc acagcagcgg cgacgacgac gacaaaggta agtttcattt 10380

tcataattac acaaatttag atttgatttt tgttgtgccc caactggggt aacctttgag 10440

ttctctcagt tgggggcgta gggcccagcg gccgctctag ctagagtcaa gcagatcgtt 10500

caaacatttg gcaataaagt ttcttaagat tgaatcctgt tgccggtctt gcgatgatta 10560

tcatataatt tctgttgaat tacgttaagc atgtaataat taacatgtaa tgcatgacgt 10620

tatttatgag atgggttttt atgattagag tcccgcaatt atacatttaa tacgcgatag 10680

aaaacaaaat atagcgcgca aactaggata aattatcgcg cgcggtgtca tctatgttac 10740

tagatcgacc agcttagatc agattgtcgt ttcccgcctt cagtttaaac tatcagtgtt 10800

tgacaggata tattggcggg taaacctaag agaaaagagc gtttattaga ataatcggat 10860

atttaaaagg gcgtgaaaag gtttatccgt tcgtccattt gtatgtgcca accacagggt 10920

t 10921

<210> 2
<211> 2568
<212> DNA
<213> Artificial

<220>
<223> T-DNA of 3' vector module pNMD231

<400> 2
cctgtggttg gcacatacaa atggacgaac ggataaacct tttcacgccc ttttaaatat 60

ccgattattc taataaacgc tcttttctct taggtttacc cgccaatata tcctgtcaaa 120

cactgatagt ttaaactgaa ggcgggaaac gacaatctga tctaagctag ggtacctcga 180

agccgcggtg cgggtgccag ggcgtgccct tgggctcccc gggcgcgtac tccacctcac 240
ccatctttta ttacatgttt gaacttcaac aatttatgac tttttgttct tattgttgca 300
ggtgcaggtg gcggatattg gcacacttct ggtagagaaa tcctagatgc aaacaatgtg 360
cctgttcgca tagctgggat taactggttc ggcttcgaga cttgtaacta cgtagtccac 420
ggactttgga gtcggactta tagaagcatg ctcgatcaaa tcaaaagcct gggatacaac 480
acgattcgac tgccatactc tgatgacatt ctgaaacctg gaaccatgcc gaactccatc 540
aacttctatc aaatgaacca agatctgcaa gggctgacta gcttgcaagt gatggataag 600
atagtagcct acgctggcca aattgggctt agaataatcc tcgatagaca tcgtcctgat 660
tgctctggac aatctgcact ctggtataca tcctctgtct ctgaagctac ctggatttcc 720
gatttgcaag cactagccca gagatacaaa ggaaatccaa ccgtagtcgg ctttgacttg 780
cataacgaac cacatgatcc tgcttgttgg ggatgtggtg atccatcaat tgactggcgt 840
ttagctgctg aaagagctgg aaacgctgtc ttatccgtga atcccaatct tctcatcttc 900
gtagagggag ttcagtcata caatggcgat tcttactggt ggggagggaa tctacaaggc 960
gctggacaat accctgtagt tctaaacgta cccaaccgat tggtgtattc tgcccatgac 1020
tatgcaacat ccgtttatcc gcaaacctgg ttttctgatc ccacatttcc gaacaatatg 1080
cctggcattt ggaacaagaa ctggggatat ctgttcaacc aaaacattgc tcccgtatgg 1140
cttggcgagt ttggaacaac tttacaaagc acaacggacc agacctggct taagacatta 1200
gtgcaatatc ttagaccgac cgcacaatat ggtgctgata gctttcaatg gacgttctgg 1260
agttggaatc ctgattctgg cgatacgggt ggaatactta aggatgactg gcaaaccgtt 1320
gacactgtaa aggacggata ccttgctccc ataaagtcta gcatctttga tcccgttggt 1380
gcatctgctt ctccaagttc acaaccaagt ccctctgttt caccaagtcc atcaccatca 1440
cctagtgctt ctagaacacc aactcctact ccaacaccaa ctgcatctcc aactcctaca 1500
ttgacaccta ccgcaacacc tacacctact gcttcaccta ctccatcacc tactgccgct 1560
tctggagcta gatgcactgc ttcttatcaa gtcaactctg attggggaaa cggctttact 1620
gtgaccgttg ctgtcacgaa ttctggaagt gtggcaacaa agacttggac tgtttcttgg 1680
acgtttgggg gaaatcaaac cattacgaac tcttggaatg ctgccgtaac acaaaatggg 1740
caatccgtta ccgctcggaa catgtcatac aataacgtca tccaacctgg gcaaaacaca 1800
acgttcggct ttcaagcctc ctataccggt tcaaatgctg cacctactgt tgcttgcgct 1860
gcatcttaag cttgaagact aggcgtggtg cgcacgatag cgcatagtgt ttttctctcc 1920
acttgaatcg aagagataga cttacggtgt aaatccgtag ggtggcgtaa accaaattta 1980
cgcaatgttt tgggttccat ttaaatcgaa accccttatt tcctggatca cctgttaacg 2040
cacgtttgac gtgtattaca gtgggaataa gtaaaagtga gaggttcgaa tcctccctaa 2100
ccccgggtag gggcccagcg gccgctctag ctagagtcaa gcagatcgtt caaacatttg 2160

gcaataaagt ttcttaagat tgaatcctgt tgccggtctt gcgatgatta tcatataatt 2220

tctgttgaat tacgttaagc atgtaataat taacatgtaa tgcatgacgt tatttatgag 2280

atgggttttt atgattagag tcccgcaatt atacatttaa tacgcgatag aaaacaaaat 2340

atagcgcgca aactaggata aattatcgcg cgcggtgtca tctatgttac tagatcgact 2400

agtgagctct gcatccaccc cagtacatta aaaacgtccg caatgtgtta ttaagttgtc 2460

taagcgtcaa tttgtttaca ccacaatata tcctgccacc agccagccaa cagctccccg 2520

accggcagct cggcacaaaa tcaccactcg atacaggcag cccatcag 2568

<210> 3
<211> 2493
<212> DNA
<213> Artificial

<220>
<223> T-DNA of 3' vector module pNMD241

<400> 3
cctgtggttg gcacatacaa atggacgaac ggataaacct tttcacgccc ttttaaatat 60

ccgattattc taataaacgc tcttttctct taggtttacc cgccaatata tcctgtcaaa 120

cactgatagt ttaaactgaa ggcgggaaac gacaatctga tctaagctag ggtacctcga 180

agccgcggtg cgggtgccag ggcgtgccct tgggctcccc gggcgcgtac tccacctcac 240

ccatctttta ttacatgttt gaacttcaac aatttatgac tttttgttct tattgttgca 300

ggtcaatctg cttgcactct gcaatccgaa acgcatccac ctttgacttg gcaaaagtgt 360

tcctctggtg gaacgtgtac ccagcaaacc ggttctgtag tgattgacgc aaattggaga 420

tggacacacg ctaccaatag ctctacgaac tgctatgatg ggaatacctg gtctagcaca 480

ctatgccctg ataacgaaac ctgtgcaaag aattgctgtc ttgatggtgc tgcctatgct 540

tcaacttacg gtgtcacaac tagcggcaat tctctttcca tcggcttcgt cacacaaagt 600

gcccaaaaga acgttggagc acggctttac cttatggctt ccgacacgac ctatcaagaa 660

ttcacgctgc ttgggaatga gttttccttt gatgtggacg tttctcaact cccatgcgga 720

cttaacggag cactttactt tgtgtctatg gacgctgatg gaggcgtttc taagtatccg 780

actaacactg ctggtgctaa gtacggaacc ggttactgcg attcacaatg ccctagagat 840

ctgaagttca tcaacggaca agcaaacgtc gagggctggg aaccttcttc caataacgca 900

aacactggca ttggtggcca cggatcatgt tgttctgaga tggacatttg ggaagcaaac 960

tcaatctccg aagcacttac cccacatccg tgtacaactg ttggccaaga aatctgtgag 1020

ggtgatggat gtggtggcac ttactctgac aatcgctatg gtggaacctg tgatccagat 1080

ggatgcgatt ggaaccccta taggcttgga aacacgtctt tctatggacc tggctctagc 1140

tttacccttg ataccactaa gaaactcacc gtggtaacgc aattcgagac tagcggagcc 1200

ataaaccgat attacgtaca gaatggcgtt acgtttcaac aacccaatgc cgagttgggc 1260

tcatactctg ggaatgagtt gaatgatgac tactgtactg ctgaagaggc tgaattcggt 1320

ggaagttcct ttagcgataa gggaggccta acccagttta agaaagcaac gtctggtggg 1380

atggttctcg tcatgagtct atgggatgac tactatgcta acatgctgtg gctcgattcc 1440

acatatccca ccaatgagac tagttcaact cctggagctg ttcgtggatc ctgttcaact 1500

agctctggtg ttcctgccca agtagaatct caaagtccca acgcaaaggt gaccttttcc 1560

aacataaagt tcggaccgat tggctcaact ggaaatccat ctgggggaaa tccacctggt 1620

ggcaatagag gaacaactac aactagaagg cctgctacta ccactggttc atctcctggt 1680

ccaactcaat ctcattatgg ccaatgcggt ggcattggct attctggtcc tactgtgtgt 1740

gctagtggaa ccacatgcca agttctcaac ccctattact cccaatgcct ctaagcttga 1800

agactaggcg tggtgcgcac gatagcgcat agtgtttttc tctccacttg aatcgaagag 1860

atagacttac ggtgtaaatc cgtaggggtg gcgtaaacca aattacgcaa tgttttgggt 1920

tccatttaaa tcgaaacccc ttatttcctg gatcacctgt taacgcacgt ttgacgtgta 1980

ttacagtggg aataagtaaa agtgagaggt tcgaatcctc cctaaccccg ggtaggggcc 2040

cagcggccgc tctagctaga gtcaagcaga tcgttcaaac atttggcaat aaagtttctt 2100

aagattgaat cctgttgccg gtcttgcgat gattatcata taatttctgt tgaattacgt 2160

taagcatgta ataattaaca tgtaatgcat gacgttattt atgagatggg tttttatgat 2220

tagagtcccg caattataca tttaatacgc gatagaaaac aaaatatagc gcgcaaacta 2280

ggataaatta tcgcgcgcgg tgtcatctat gttactagat cgactagtga gctctgcatc 2340

caccccagta cattaaaaac gtccgcaatg tgttattaag ttgtctaagc gtcaatttgt 2400

ttacaccaca atatatcctg ccaccagcca gccaacagct ccccgaccgg cagctcggca 2460

caaaatcacc actcgataca ggcagcccat cag 2493

<210> 4
<211> 2679
<212> DNA
<213> Artificial

<220>
<223> T-DNA of 3' vector module pNMD251

<400> 4
cctgtggttg gcacatacaa atggacgaac ggataaacct tttcacgccc ttttaaatat 60

ccgattattc taataaacgc tcttttctct taggtttacc cgccaatata tcctgtcaaa 120

cactgatagt ttaaactgaa ggcgggaaac gacaatctga tctaagctag ggtacctcga 180

agccgcggtg cgggtgccag ggcgtgccct tgggctcccc gggcgcgtac tccacctcac 240

ccatctttta ttacatgttt gaacttcaac aatttatgac tttttgttct tattgttgca 300

ggtgcaggct gttctgttga ttacaccgtg aattcttggg gaactggatt cacggctaac 360

gttaccatca cgaatcttgg ttctgccata aacggatgga cattagagtg ggacttccct 420

ggcaaccagc aagttaccaa cctgtggaat ggcacgtata cccagtctgg gcaacacgtt 480

tctgtttcta atgctcctta caacgcatct atccctgcaa atggcactgt cgaattcggc 540

tttaatggga gctattctgg aagcaacgac atacctagct cttttaagct gaacggtgta 600

acatgtgacg ggagcgacga tccagatcct gaaccttctc catcaccttc tccatctccc 660

agccctactg atcctgatga acctggtgga ccaaccaatc ctccaacaaa ccctggcgag 720

aaagtggaca atcccttcga gggtgccaaa ctttacgtaa accctgtatg gagtgctaaa 780

gctgcagctg aacctggtgg atctgctgta gccaatgaat ctactgctgt ttggctggat 840

agaatcggtg ccatagaggg aaacgattct cctacaactg ggtctatggg tcttagagat 900

catctcgagg aagccgtaag acaaagtggt ggcgatcccc ttaccatcca agttgtgatc 960

tacaatctac ctggtcgcga ctgtgctgca cttgcttcta atggagaatt gggacctgat 1020

gaactggata gatacaagtc cgagtacatt gatcccattg ctgacatcat gtgggacttc 1080

gctgactatg agaacctacg aatcgtcgcc atcattgaga tagactccct ccctaatcta 1140

gtcactaatg tcggaggcaa cggagggact gagctatgtg catacatgaa gcaaaacgga 1200

ggctacgtca atggagttgg ctatgccctt agaaagttgg gtgagattcc taacgtctac 1260

aactacatcg atgccgctca ccatggctgg ataggctggg attctaactt tggaccctcc 1320

gtggacatct tctacgaagc tgcaaatgcc tctggtagca ccgttgacta tgtgcatgga 1380

ttcatctcta acacggcaaa ctatagcgct actgttgaac cgtatctaga cgtaaacggc 1440

accgtgaatg ggcaactgat aaggcaatct aagtgggtcg actggaacca atacgttgat 1500

gagttgagtt tcgtccaaga cttgaggcaa gctctcatag ccaaagggtt tcgttctgat 1560

atcggcatgc tcatagacac ttctagaaac ggatggggag gccctaatcg tccaactgga 1620

cctagctcat ctacggacct taacacatac gttgacgagt cacgaattga tagacgcata 1680

caccctggaa actggtgcaa ccaagctggt gctggcttgg gtgaaagacc taccgtgaac 1740

ccagctcctg gtgttgacgc atacgtttgg gttaagcctc cgggagaatc tgatggagct 1800

tccgaggaaa tccctaatga tgaggggaag ggcttcgata gaatgtgcga tcctacttac 1860

caaggcaacg ctagaaacgg caacaatcca tctggtgctc tacctaatgc tcccatatct 1920

gggcactggt tttctgccca atttcgggag cttctagcta atgcttaccc tcccctctaa 1980

gcttgaagac taggcgtggt gcgcacgata gcgcatagtg tttttctctc cacttgaatc 2040

gaagagatag acttacggtg taaatccgta ggggtggcgt aaaccaaatt acgcaatgtt 2100

ttgggttcca tttaaatcga aaccccttat ttcctggatc acctgttaac gcacgtttga 2160

cgtgtattac agtgggaata agtaaaagtg agaggttcga atcctcccta accccgggta 2220

ggggcccagc ggccgctcta gctagagtca agcagatcgt tcaaacattt ggcaataaag 2280

tttcttaaga ttgaatcctg ttgccggtct tgcgatgatt atcatataat ttctgttgaa 2340

ttacgttaag catgtaataa ttaacatgta atgcatgacg ttatttatga gatgggtttt 2400

tatgattaga gtcccgcaat tatacattta atacgcgata gaaaacaaaa tatagcgcgc 2460

aaactaggat aaattatcgc gcgcggtgtc atctatgtta ctagatcgac tagtgagctc 2520

tgcatccacc ccagtacatt aaaaacgtcc gcaatgtgtt attaagttgt ctaagcgtca 2580

atttgtttac accacaatat atcctgccac cagccagcca acagctcccc gaccggcagc 2640

tcggcacaaa atcaccactc gatacaggca gcccatcag 2679

<210> 5
<211> 1978
<212> DNA
<213> Artificial

<220>
<223> T-DNA of 3' vector module pNMD910

<400> 5
cctgtggttg gcacatacaa atggacgaac ggataaacct tttcacgccc ttttaaatat 60

ccgattattc taataaacgc tcttttctct taggtttacc cgccaatata tcctgtcaaa 120

cactgatagt ttaaactgaa ggcgggaaac gacaatctga tctaagctag ggtacctcga 180

agccgcggtg cgggtgccag ggcgtgccct tgggctcccc gggcgcgtac tccacctcac 240

ccatctttta ttacatgttt gaacttcaac aatttatgac tttttgttct tattgttgca 300

ggtatgagcc taccacctga ctttaagtgg ggttttgcta ccgccgctta tcagatagaa 360

ggatctgtga atgaggatgg tagaggacct tctatttggg acaccttttg tgcaatccct 420

ggtaaaatag ctgacggaag ctcaggagct gttgcctgtg attcttacaa gcggacaaag 480

gaagatattg ccttgttaaa agagcttggt gctaactcct atagattttc catttcttgg 540

tcccgaatta tccctctggg cggaagaaat gaccctatca accaaaaagg catcgatcat 600

tacgtgaagt tcgtagacga tctaatcgag gccggaatta ctccattcat tactctcttc 660

cactgggatc ttcccgatgc attggataag aggtacgggg gctttctaaa caaagaagag 720

ttcgctgcag atttcgagaa ttatgcaagg attatgttta aagcaatccc taagtgcaag 780

cactggatta cctttaacga accatggtgt agtgccattc tcggatataa tacaggctat 840

ttcgcacctg gacacacttc tgatagatct aagagtccgg tgggtgactc agctcgtgaa 900

ccgtggatag ttgggcacaa catactaatt gcccatgcta gggcagttaa ggcttatcgt 960

gaagatttca aaccaacgca aggtggagaa ataggtataa cgctgaacgg tgatgctact 1020

cttccgtggg atccagagga tcccgctgac attgaagcat gtgataggaa aatcgaattt 1080

gccataagct ggttcgcaga tccgatctac ttcggcaaat atccggactc aatgagaaag 1140

cagttagggg atcgactccc agaattcacg cctgaagagg tggctctagt gaaaggtagt 1200

aacgatttct acgggatgaa tcactatacg gctaattaca tcaaacacaa gactggtgtt 1260

cctcccgaag acgatttcct tggaaacctt gagacactct tttataacaa atatggtgat 1320

tgcattggcc ctgagacaca gtcattctgg ttaagaccac atgctcaagg gtttcgagat 1380

ttgctgaatt ggctttcaaa gaggtacggg taccctaaga tctacgtcac tgaaaacgga 1440

accagtctga agggtgagaa tgacatgcca ctagaacaag tcctcgagga cgatttccga 1500

gttaagtatt tcaatgatta cgttcgtgca atggccgctg cagtagctga agatggttgc 1560

aacgtaagag gctacttggc ctggagtcta ctggataatt ttgaatgggc tgagggatac 1620

gagacacggt tcggtgttac ttatgtcgac tatgccaacg atcagaagcg ctatccaaag 1680

aaatctgcaa agtccttaaa gcccctgttt gatagcctta ttcgtaaaga gtaagcttga 1740

agactaggcg tggtgcgcac gatagcgcat agtgtttttc tctccacttg aatcgaagag 1800

atagacttac ggtgtaaatc cgtaggggtg gcgtaaacca aattacgcaa tgttttgggt 1860

tccatttaaa tcgaaacccc ttatttcctg gatcacctgt taacgcacgt ttgacgtgta 1920

ttacagtggg aataagtaaa agtgagaggt tcgaatcctc cctaaccccg ggtagggg 1978

<210> 6
<211> 4807
<212> DNA
<213> Artificial

<220>
<223> T-DNA of PhiC31 integrase expressing construct

<400> 6
ctgatgggct gcctgtatcg agtggtgatt ttgtgccgag ctgccggtcg gggagctgtt 60

ggctggctgg tggcaggata tattgtggtg taaacaaatt gacgcttaga caacttaata 120

acacattgcg gacgttttta atgtactggg gtggatgcag gtcgatctag taacatagat 180

gacaccgcgc gcgataattt atcctagttt gcgcgctata ttttgttttc tatcgcgtat 240

taaatgtata attgcgggac tctaatcata aaaacccatc tcataaataa cgtcatgcat 300

tacatgttaa ttattacatg cttaacgtaa ttcaacagaa attatatgat aatcatcgca 360

agaccggcaa caggattcaa tcttaagaaa ctttattgcc aaatgtttga acgatctgct 420

tgactctaga tccagagtcc cgctcagaag aactcgtcaa gaaggcgata gaaggcgatg 480

cgctgcgaat cgggagcggc gataccgtaa agcacgagga agcggtcagc ccattcgccg 540

ccaagctctt cagcaatatc acgggtagcc aacgctatgt cctgatagcg gtccgccaca 600

cccagccggc cacagtcgat gaatccagaa aagcggccat tttccaccat gatattcggc 660

aagcaggcat cgccatgagt cacgacgaga tcctcgccgt cgggcatacg cgccttgagc 720

ctggcgaaca gttcggctgg cgcgagcccc tgatgctctt cgtccagatc atcctgatcg 780

acaagaccgg cttccatccg agtacgtgct cgctcgatgc gatgtttcgc ttggtggtcg 840

aatgggcagg tagccggatc aagcgtatgc agccgccgca ttgcatcagc catgatggat 900

actttctcgg caggagcaag gtgagatgac aggagatcct gccccggcac ttcgcccaat 960

agcagccagt cccttcccgc ttcagtgaca acgtcgagca cagctgcgca aggaacgccc 1020

gtcgtggcca gccacgatag ccgcgctgcc tcgtcctgga gttcattcag ggcaccggac 1080

aggtcggtct tgacaaaaag aaccgggcgc ccctgcgctg acagccggaa cacggcggca 1140

tcagagcagc cgattgtctg ttgtgcccag tcatagccga atagcctctc cacccaagcg 1200

gccggagaac ctgcgtgcaa tccatcttgt tcaatcatgc gaaacgatcc agatccggtg 1260

cagattattt ggattgagag tgaatatgag actctaattg gataccgagg ggaatttatg 1320
gaacgtcagt ggagcatttt tgacaagaaa tatttgctag ctgatagtga ccttaggcga 1380
cttttgaacg cgcaataatg gtttctgacg tatgtgctta gctcattaaa ctccagaaac 1440
ccgcggctga gtggctcctt caacgttgcg gttctgtcag ttccaaacgt aaaacggctt 1500
gtcccgcgtc atcggcgggg gtcataacgt gactccctta attctccgct catgaattgg 1560
taccgcttat gcagccgatg gtagattcga agacgcagct aaagctagaa aattgcttcg 1620
agaccgaggg gaaaagaagg aaacagggct aagttgggtt gaagaaagga acaaggttca 1680
tacatttgca gctggagaaa gacgtcatga gaaaagcaag gagatatatg agaagctggc 1740
tgagttaggg gaagagatgg agaaagcggg ttacattgca gacacaagct atgttctgag 1800
agaagtggat ggtgacgaga agaatcaaac cataaggtat catagtgaga gattagctat 1860
tgcttttgga ctgatcacgt ttccagctga tagaccgatc cgagtaatga agaatctgcg 1920
tgtttgcggt gattgccata acgcgatcaa gttcatgtcc gtatgtacga gacgagtgat 1980
cattgtgaga gataataatc ggtttcatcg gtttgaagat ggcaagtgtt cttgtaatga 2040
ctattggtga agaagacaaa tgagagttgg tttatattta accataattt cattcagttc 2100
acactgaacc ggcgaaattt ctttgccaga cctattcgga attgaaacaa gtggagtctc 2160
gaaacgaaaa gaactttctg gaattcgttt gctcacaaag ctaaaaacgg ttgatttcat 2220
cgaaatacgg cgtcgttttc aaagaacaat ccagaaatca ctggttttcc tttatttcaa 2280
aagaagagac tagaacttta tttctcctct ataaaatcac tttgtttttt cctctcttct 2340
tcataaatca acaaaacaat cacaaatctc tcgaaacgct ctcgaagttc caaattttct 2400
cttagcattc tctttcgttt ctcgtttgcg ttgaatcaaa gttcgttgcg atggctcgag 2460
tctgcaggac aatggacacg tacgcgggtg cttacgaccg tcagtcgcgc gagcgcgaga 2520
attcgagcgc agcaagccca gcgacacagc gtagcgccaa cgaagacaag gcggccgacc 2580
ttcagcgcga agtcgagcgc gacgggggcc ggttcaggtt cgtcgggcat ttcagcgaag 2640
cgccgggcac gtcggcgttc gggacggcgg agcgcccgga gttcgaacgc atcctgaacg 2700
aatgccgcgc cgggcggctc aacatgatca ttgtctatga cgtgtcgcgc ttctcgcgcc 2760
tgaaggtcat ggacgcgatt ccgattgtct cggaattgct cgccctgggc gtgacgattg 2820
tttccactca ggaaggcgtc ttccggcagg gaaacgtcat ggacctgatt cacctgatta 2880
tgcggctcga cgcgtcgcac aaagaatctt cgctgaagtc ggcgaagatt ctcgacacga 2940
agaaccttca gcgcgaattg ggcgggtacg tcggcgggaa ggcgccttac ggcttcgagc 3000
ttgtttcgga gacgaaggag atcacgcgca acggccgaat ggtcaatgtc gtcatcaaca 3060
agcttgcgca ctcgaccact ccccttaccg gacccttcga gttcgagccc gacgtaatcc 3120
ggtggtggtg gcgtgagatc aagacgcaca aacaccttcc cttcaagccg ggcagtcaag 3180
ccgccattca cccgggcagc atcacggggc tttgtaagcg catggacgct gacgccgtgc 3240

cgacccgggg cgagacgatt gggaagaaga ccgcttcaag cgcctgggac ccggcaaccg 3300

ttatgcgaat ccttcgggac ccgcgtattg cgggcttcgc cgctgaggtg atctacaaga 3360

agaagccgga cggcacgccg accacgaaga ttgagggtta ccgcattcag cgcgacccga 3420

tcacgctccg gccggtcgag cttgattgcg gaccgatcat cgagcccgct gagtggtatg 3480

agcttcaggc gtggttggac ggcagggggc gcggcaaggg gctttcccgg gggcaagcca 3540

ttctgtccgc catggacaag ctgtactgcg agtgtggcgc cgtcatgact tcgaagcgcg 3600

gggaagaatc gatcaaggac tcttaccgct gccgtcgccg gaaggtggtc gacccgtccg 3660

cacctgggca gcacgaaggc acgtgcaacg tcagcatggc ggcactcgac aagttcgttg 3720

cggaacgcat cttcaacaag atcaggcacg ccgaaggcga cgaagagacg ttggcgcttc 3780

tgtgggaagc cgcccgacgc ttcggcaagc tcactgaggc gcctgagaag agcggcgaac 3840

gggcgaacct tgttgcggag cgcgccgacg ccctgaacgc ccttgaagag ctgtacgaag 3900

accgcgcggc aggcgcgtac gacggacccg ttggcaggaa gcacttccgg aagcaacagg 3960

cagcgctgac gctccggcag caaggggcgg aagagcggct tgccgaactt gaagccgccg 4020

aagccccgaa gcttcccctt gaccaatggt tccccgaaga cgccgacgct gacccgaccg 4080

gccctaagtc gtggtggggg cgcgcgtcag tagacgacaa gcgcgtgttc gtcgggctct 4140

tcgtagacaa gatcgttgtc acgaagtcga ctacgggcag gggcagggga acgcccatcg 4200

agaagcgcgc ttcgatcacg tgggcgaagc cgccgaccga cgacgacgaa gacgacgccc 4260

aggacggcac ggaagacgta gcggcgtaga ccccctaaga agaagaggaa ggtttaggat 4320

ccaccggaga tccctggccg gccccgatcg ttcaaacatt tggcaataaa gtttcttaag 4380

attgaatcct gttgccggtc ttgcgatgat tatcatataa tttctgttga attacgttaa 4440

gcatgtaata attaacatgt aatgcatgac gttatttatg agatgggttt ttatgattag 4500

agtcccgcaa ttatacattt aatacgcgat agaaaacaaa atatagcgcg caaactagga 4560

taaattatcg cgcgcggtgt catctatgtt actagatccg atgataagct gtcaaacatg 4620

agaattgggc cccaagctta tgatcagatt gtcgtttccc gccttcagtt taaactatca 4680

gtgtttgaca ggatatattg gcgggtaaac ctaagagaaa agagcgttta ttagaataat 4740

cggatattta aaagggcgtg aaaaggttta tccgttcgtc catttgtatg tgcatgccaa 4800

ccacagg 4807

<210> 7
<211> 11053
<212> DNA
<213> Artificial

<220>
<223> T-DNA of assembled vector pNMD1201

<400> 7
cctgtggttg gcacatacaa atggacgaac ggataaacct tttcacgccc ttttaaatat 60

ccgattattc taataaacgc tcttttctct taggtttacc cgccaatata tcctgtcaaa 120

cactgatagt ttaaactgaa ggcgggaaac gacaatctga tctaagctag cttggaattg 180

gtaccacgcg tttcgacaaa atttagaacg aacttaatta tgatctcaaa tacattgata 240

catatctcat ctagatctag gttatcatta tgtaagaaag ttttgacgaa tatggcacga 300

caaaatggct agactcgatg taattggtat ctcaactcaa cattatactt ataccaaaca 360

ttagttagac aaaatttaaa caactatttt ttatgtatgc aagagtcagc atatgtataa 420

ttgattcaga atcgttttga cgagttcgga tgtagtagta gccattattt aatgtacata 480

ctaatcgtga atagtgaata tgatgaaaca ttgtatctta ttgtataaat atccataaac 540

acatcatgaa agacactttc tttcacggtc tgaattaatt atgatacaat tctaatagaa 600

aacgaattaa attacgttga attgtatgaa atctaattga acaagccaac cacgacgacg 660

actaacgttg cctggattga ctcggtttaa gttaaccact aaaaaaacgg agctgtcatg 720

taacacgcgg atcgagcagg tcacagtcat gaagccatca aagcaaaaga actaatccaa 780

gggctgagat gattaattag tttaaaaatt agttaacacg agggaaaagg ctgtctgaca 840

gccaggtcac gttatcttta cctgtggtcg aaatgattcg tgtctgtcga ttttaattat 900

ttttttgaaa ggccgaaaat aaagttgtaa gagataaacc cgcctatata aattcatata 960

ttttcctctc cgctttgaag ttttagtttt attgcaacaa caacaacaaa ttacaataac 1020

aacaaacaaa atacaaacaa caacaacatg gcacaatttc aacaaacaat tgacatgcaa 1080

actctccaag ccgctgcggg acgcaacagc ttggtgaatg atttggcatc tcgtcgcgtt 1140

tacgataatg cagtcgagga gctgaatgct cgttccagac gtcccaaggt aataggaact 1200

ttctggatct actttatttg ctggatctcg atcttgtttt ctcaatttcc ttgagatctg 1260

gaattcgttt aatttggatc tgtgaacctc cactaaatct tttggtttta ctagaatcga 1320

tctaagttga ccgatcagtt agctcgatta tagctaccag aatttggctt gaccttgatg 1380

gagagatcca tgttcatgtt acctgggaaa tgatttgtat atgtgaattg aaatctgaac 1440

tgttgaagtt agattgaatc tgaacactgt caatgttaga ttgaatctga acactgttta 1500

aggttagatg aagtttgtgt atagattctt cgaaacttta ggatttgtag tgtcgtacgt 1560

tgaacagaaa gctatttctg attcaatcag ggtttatttg actgtattga actctttttg 1620

tgtgtttgca ggtccacttc tccaaggcag tgtctacgga acagaccctg attgcaacaa 1680

acgcatatcc ggagttcgag atttccttta ctcatacgca atccgctgtg cactccttgg 1740

ccggaggcct tcggtcactt gagttggagt atctcatgat gcaagttccg ttcggttctc 1800

tgacgtacga catcggcggt aacttttccg cgcacctttt caaagggcgc gattacgttc 1860

actgctgcat gcctaatctg gatgtacgtg acattgctcg ccatgaagga cacaaggaag 1920

ctatttacag ttatgtgaat cgtttgaaaa ggcagcagcg tcctgtgcct gaataccaga 1980

gggcagcttt caacaactac gctgagaacc cgcacttcgt ccattgcgac aaacctttcc 2040

aacagtgtga attgacgaca gcgtatggca ctgacaccta cgctgtagct ctccatagca 2100

tttatgatat ccctgttgag gagttcggtt ctgcgctact caggaagaat gtgaaaactt 2160

gtttcgcggc ctttcatttc catgagaata tgcttctaga ttgtgataca gtcacactcg 2220

atgagattgg agctacgttc cagaaatcag gtaacattcc ttagttacct ttcttttctt 2280

tttccatcat aagtttatag attgtacatg ctttgagatt tttctttgca aacaatctca 2340

ggtgataacc tgagcttctt cttccataat gagagcactc tcaattacac ccacagcttc 2400

agcaacatca tcaagtacgt gtgcaagacg ttcttccctg ctagtcaacg cttcgtgtac 2460

cacaaggagt tcctggtcac tagagtcaac acttggtact gcaagttcac gagagtggat 2520

acgttcactc tgttccgtgg tgtgtaccac aacaatgtgg attgcgaaga gttttacaag 2580

gctatggacg atgcgtggca ctacaaaaag acgttagcaa tgcttaatgc cgagaggacc 2640

atcttcaagg ataacgctgc gttaaacttc tggttcccga aggtgctctt gaaattggaa 2700

gtcttctttt gttgtctaaa cctatcaatt tctttgcgga aatttatttg aagctgtaga 2760

gttaaaattg agtcttttaa acttttgtag gtgagagaca tggttatcgt ccctctcttt 2820

gacgcttcta tcacaactgg taggatgtct aggagagagg ttatggtgaa caaggacttc 2880

gtctacacgg tcctaaatca catcaagacc tatcaagcta aggcactgac gtacgcaaac 2940

gtgctgagct tcgtggagtc tattaggtct agagtgataa ttaacggtgt cactgccagg 3000

taagttgtta cttatgattg ttttcctctc tgctacatgt attttgttgt tcatttctgt 3060

aagatataag aattgagttt tcctctgatg atattattag gtctgaatgg gacacagaca 3120

aggcaattct aggtccatta gcaatgacat tcttcctgat cacgaagctg ggtcatgtgc 3180

aagatgaaat aatcctgaaa aagttccaga agttcgacag aaccaccaat gagctgattt 3240

ggacaagtct ctgcgatgcc ctgatggggg ttattccctc ggtcaaggag acgcttgtgc 3300

gcggtggttt tgtgaaagta gcagaacaag ccttagagat caaggttagt atcatatgaa 3360

gaaataccta gtttcagttg atgaatgcta ttttctgacc tcagttgttc tcttttgaga 3420

attatttctt ttctaatttg cctgattttt ctattaattc attaggttcc cgagctatac 3480

tgtaccttcg ccgaccgatt ggtactacag tacaagaagg cggaggagtt ccaatcgtgt 3540

gatctttcca aacctctaga agagtcagag aagtactaca acgcattatc cgagctatca 3600

gtgcttgaga atctcgactc ttttgactta gaggcgttta agactttatg tcagcagaag 3660

aatgtggacc cggatatggc agcaaaggta aatcctggtc cacactttta cgataaaaac 3720

acaagatttt aaactatgaa ctgatcaata atcattccta aaagaccaca cttttgtttt 3780

gtttctaaag taatttttac tgttataaca ggtggtcgta gcaatcatga agtcagaatt 3840

gacgttgcct ttcaagaaac ctacagaaga ggaaatctcg gagtcgctaa aaccaggaga 3900

ggggtcgtgt gcagagcata aggaagtgtt gagcttacaa aatgatgctc cgttcccgtg 3960

tgtgaaaaat ctagttgaag gttccgtgcc ggcgtatgga atgtgtccta agggtggtgg 4020

tttcgacaaa ttggatgtgg acattgctga tttccatctc aagagtgtag atgcagttaa 4080

aaagggaact atgatgtctg cggtgtacac agggtctatc aaagttcaac aaatgaagaa 4140

ctacatagat tacttaagtg cgtcgctggc agctacagtc tcaaacctct gcaaggtaag 4200

aggtcaaaag gtttccgcaa tgatccctct tttttgtttt ctctagtttc aagaatttgg 4260

gtatatgact aacttctgag tgttccttga tgcatatttg tgatgagaca aatgtttgtt 4320

ctatgtttta ggtgcttaga gatgttcacg gcgttgaccc agagtcacag gagaaatctg 4380

gagtgtggga tgttaggaga ggacgttggt tacttaaacc taatgcgaaa agtcacgcgt 4440

ggggtgtggc agaagacgcc aaccacaagt tggttattgt gttactcaac tgggatgacg 4500

gaaagccggt ttgtgatgag acatggttca gggtggcggt gtcaagcgat tccttgatat 4560

attcggatat gggaaaactt aagacgctca cgtcttgcag tccaaatggt gagccaccgg 4620

agcctaacgc caaagtaatt ttggtcgatg gtgttcccgg ttgtggaaaa acgaaggaga 4680

ttatcgaaaa ggtaagttct gcatttggtt atgctccttg cattttaggt gttcgtcgct 4740

cttccatttc catgaatagc taagattttt tttctctgca ttcattcttc ttgcctcagt 4800

tctaactgtt tgtggtattt ttgttttaat tattgctaca ggtaaacttc tctgaagact 4860

tgattttagt ccctgggaag gaagcttcta agatgatcat ccggagggcc aaccaagctg 4920

gtgtgataag agcggataag gacaatgtta gaacggtgga ttccttcttg atgcatcctt 4980

ctagaagggt gtttaagagg ttgtttatcg atgaaggact aatgctgcat acaggttgtg 5040

taaatttcct actgctgcta tctcaatgtg acgtcgcata tgtgtatggg gacacaaagc 5100

aaattccgtt catttgcaga gtcgcgaact ttccgtatcc agcgcatttt gcaaaactcg 5160

tcgctgatga gaaggaagtc agaagagtta cgctcaggta aagcaactgt gttttaatca 5220

atttcttgtc aggatatatg gattataact taatttttga gaaatctgta gtatttggcg 5280

tgaaatgagt ttgctttttg gtttctcccg tgttataggt gcccggctga tgttacgtat 5340

ttccttaaca agaagtatga cggggcggtg atgtgtacca gcgcggtaga gagatccgtg 5400

aaggcagaag tggtgagagg aaagggtgca ttgaacccaa taaccttacc gttggagggt 5460

aaaattttga ccttcacaca agctgacaag ttcgagttac tggagaaggg ttacaaggta 5520

aagtttccaa ctttccttta ccatatcaaa ctaaagttcg aaacttttta tttgatcaac 5580

ttcaaggcca cccgatcttt ctattcctga ttaatttgtg atgaatccat attgactttt 5640

gatggttacg caggatgtga acactgtgca cgaggtgcaa ggggagacgt acgagaagac 5700

tgctattgtg cgcttgacat caactccgtt agagatcata tcgagtgcgt cacctcatgt 5760

tttggtggcg ctgacaagac acacaacgtg ttgtaaatat tacaccgttg tgttggaccc 5820

gatggtgaat gtgatttcag aaatggagaa gttgtccaat ttccttcttg acatgtatag 5880

agttgaagca ggtctgtctt tcctatttca tatgtttaat cctaggaatt tgatcaattg 5940

attgtatgta tgtcgatccc aagactttct tgttcactta tatcttaact ctctctttgc 6000

tgtttcttgc aggtgtccaa tagcaattac aaatcgatgc agtattcagg ggacagaact 6060

tgtttgttca gacgcccaag tcaggagatt ggcgagatat gcaattttac tatgacgctc 6120

ttcttcccgg aaacagtact attctcaatg aatttgatgc tgttacgatg aatttgaggg 6180

atatttcctt aaacgtcaaa gattgcagaa tcgacttctc caaatccgtg caacttccta 6240

aagaacaacc tattttcctc aagcctaaaa taagaactgc ggcagaaatg ccgagaactg 6300

caggtaaaat attggatgcc agacgatatt ctttcttttg atttgtaact ttttcctgtc 6360

aaggtcgata aattttattt tttttggtaa aaggtcgata attttttttt ggagccatta 6420

tgtaattttc ctaattaact gaaccaaaat tatacaaacc aggtttgctg gaaaatttgg 6480

ttgcaatgat caaaagaaac atgaatgcgc cggatttgac agggacaatt gacattgagg 6540

atactgcatc tctggtggtt gaaaagtttt gggattcgta tgttgacaag gaatttagtg 6600

gaacgaacga aatgaccatg acaagggaga gcttctccag gtaaggactt ctcatgaata 6660

ttagtggcag attagtgttg ttaaagtctt tggttagata atcgatgcct cctaattgtc 6720

catgttttac tggttttcta caattaaagg tggctttcga aacaagagtc atctacagtt 6780

ggtcagttag cggactttaa ctttgtggat ttgccggcag tagatgagta caagcatatg 6840

atcaagagtc aaccaaagca aaagttagac ttgagtattc aagacgaata tcctgcattg 6900

cagacgatag tctaccattc gaaaaagatc aatgcgattt tcggtccaat gttttcagaa 6960

cttacgagga tgttactcga aaggattgac tcttcgaagt ttctgttcta caccagaaag 7020

acacctgcac aaatagagga cttcttttct gacctagact caacccaggc gatggaaatt 7080

ctggaactcg acatttcgaa gtacgataag tcacaaaacg agttccattg tgctgtagag 7140

tacaagatct gggaaaagtt aggaattgat gagtggctag ctgaggtctg gaaacaaggt 7200

gagttcctaa gttccatttt tttgtaatcc ttcaatgtta ttttaacttt tcagatcaac 7260

atcaaaatta ggttcaattt tcatcaacca aataatattt ttcatgtata tataggtcac 7320

agaaaaacga ccttgaaaga ttatacggcc ggaatcaaaa catgtctttg gtatcaaagg 7380

aaaagtggtg atgtgacaac ctttattggt aataccatca tcattgccgc atgtttgagc 7440

tcaatgatcc ccatggacaa agtgataaag gcagcttttt gtggagacga tagcctgatt 7500

tacattccta aaggtttaga cttgcctgat attcaggcgg gcgcgaacct catgtggaac 7560

ttcgaggcca aactcttcag gaagaagtat ggttacttct gtggtcgtta tgttattcac 7620

catgatagag gagccattgt gtattacgat ccgcttaaac taatatctaa gttaggttgt 7680

aaacatatta gagatgttgt tcacttagaa gagttacgcg agtctttgtg tgatgtagct 7740

agtaacttaa ataattgtgc gtatttttca cagttagatg aggccgttgc cgaggttcat 7800

aagaccgcgg taggcggttc gtttgctttt tgtagtataa ttaagtattt gtcagataag 7860

agattgttta gagatttgtt ctttgtttga taatgtcgat agtctcgtac gaacctaagg 7920

tgagtgattt cctcaatctt tcgaagaagg aagagatctt gccgaaggct ctaacgaggt 7980

taaaaaccgt gtctattagt actaaagata ttatatctgt caaggagtcg gagactttgt 8040

gtgatataga tttgttaatc aatgtgccat tagataagta tagatatgtg ggtatcctag 8100

gagccgtttt taccggagag tggctagtgc cagacttcgt taaaggtgga gtgacgataa 8160

gtgtgataga taagcgtctg gtgaactcaa aggagtgcgt gattggtacg tacagagccg 8220

cagccaagag taagaggttc cagttcaaat tggttccaaa ttactttgtg tccaccgtgg 8280

acgcaaagag gaagccgtgg caggtaagga tttttatgat atagtatgct tatgtatttt 8340

gtactgaaag catatcctgc ttcattggga tattactgaa agcatttaac tacatgtaaa 8400

ctcacttgat gatcaataaa cttgattttg caggttcatg ttcgtataca agacttgaag 8460

attgaggcgg gttggcagcc gttagctctg gaagtagttt cagttgctat ggtcaccaat 8520

aacgttgtca tgaagggttt gagggaaaag gtcgtcgcaa taaatgatcc ggacgtcgaa 8580

ggtttcgaag gtaagccatc ttcctgctta tttttataat gaacatagaa ataggaagtt 8640

gtgcagagaa actaattaac ctgactcaaa atctaccctc ataattgttg tttgatattg 8700

gtcttgtatt ttgcaggtgt ggttgacgaa ttcgtcgatt cggttgcagc atttaaagcg 8760

gttgacaact ttaaaagaag gaaaaagaag gttgaagaaa agggtgtagt aagtaagtat 8820

aagtacagac cggagaagta cgccggtcct gattcgttta atttgaaaga agaaaacgtc 8880

ttacaacatt acaaacccga atcagtacca gtatttcgat aagaaacaag aaaccatgag 8940

cctaccacct gactttaagt ggggttttgc taccgccgct tatcagatag aaggatctgt 9000

gaatgaggat ggtagaggac cttctatttg ggacaccttt tgtgcaatcc ctggtaaaat 9060

agctgacgga agctcaggag ctgttgcctg tgattcttac aagcggacaa aggaagatat 9120

tgccttgtta aaagagcttg gtgctaactc ctatagattt tccatttctt ggtcccgaat 9180

tatccctctg ggcggaagaa atgaccctat caaccaaaaa ggcatcgatc attacgtgaa 9240

gttcgtagac gatctaatcg aggccggaat tactccattc attactctct tccactggga 9300

tcttcccgat gcattggata agaggtacgg gggctttcta aacaaagaag agttcgctgc 9360

agatttcgag aattatgcaa ggattatgtt taaagcaatc cctaagtgca agcactggat 9420

tacctttaac gaaccatggt gtagtgccat tctcggatat aatacaggct atttcgcacc 9480

tggacacact tctgatagat ctaagagtcc ggtgggtgac tcagctcgtg aaccgtggat 9540

agttgggcac aacatactaa ttgcccatgc tagggcagtt aaggcttatc gtgaagattt 9600

caaaccaacg caaggtggag aaataggtat aacgctgaac ggtgatgcta ctcttccgtg 9660

ggatccagag gatcccgctg acattgaagc atgtgatagg aaaatcgaat ttgccataag 9720

ctggttcgca gatccgatct acttcggcaa atatccggac tcaatgagaa agcagttagg 9780

ggatcgactc ccagaattca cgcctgaaga ggtggctcta gtgaaaggta gtaacgattt 9840

ctacgggatg aatcactata cggctaatta catcaaacac aagactggtg ttcctcccga 9900

agacgatttc cttggaaacc ttgagacact cttttataac aaatatggtg attgcattgg 9960

ccctgagaca cagtcattct ggttaagacc acatgctcaa gggtttcgag atttgctgaa 10020

ttggctttca aagaggtacg ggtaccctaa gatctacgtc actgaaaacg gaaccagtct 10080

gaagggtgag aatgacatgc cactagaaca agtcctcgag gacgatttcc gagttaagta 10140

tttcaatgat tacgttcgtg caatggccgc tgcagtagct gaagatggtt gcaacgtaag 10200

aggctacttg gcctggagtc tactggataa ttttgaatgg gctgagggat acgagacacg 10260

gttcggtgtt acttatgtcg actatgccaa cgatcagaag cgctatccaa agaaatctgc 10320

aaagtcctta aagcccctgt ttgatagcct tattcgtaaa gagtaagctt actagagcgt 10380

ggtgcgcacg atagcgcata gtgtttttct ctccacttga atcgaagaga tagacttacg 10440

gtgtaaatcc gtaggggtgg cgtaaaccaa attacgcaat gttttgggtt ccatttaaat 10500

cgaaacccct tatttcctgg atcacctgtt aacgcacgtt tgacgtgtat tacagtggga 10560

ataagtaaaa gtgagaggtt cgaatcctcc ctaaccccgg gtaggggccc agcggccgct 10620

ctagctagag tcaagcagat cgttcaaaca tttggcaata aagtttctta agattgaatc 10680

ctgttgccgg tcttgcgatg attatcatat aatttctgtt gaattacgtt aagcatgtaa 10740

taattaacat gtaatgcatg acgttattta tgagatgggt ttttatgatt agagtcccgc 10800

aattatacat ttaatacgcg atagaaaaca aaatatagcg cgcaaactag gataaattat 10860

cgcgcgcggt gtcatctatg ttactagatc gacctgcatc caccccagta cattaaaaac 10920

gtccgcaatg tgttattaag ttgtctaagc gtcaatttgt ttacaccaca atatatcctg 10980

ccaccagcca gccaacagct ccccgaccgg cagctcggca caaaatcacc actcgataca 11040

ggcagcccat cag 11053

<210> 8
<211> 11377
<212> DNA
<213> Artificial

<220>
<223> T-DNA of assembled viral vector pNMD1161

<400> 8
cctgtggttg gcacatacaa atggacgaac ggataaacct tttcacgccc ttttaaatat 60

ccgattattc taataaacgc tcttttctct taggtttacc cgccaatata tcctgtcaaa 120

cactgatagt ttaaactgaa ggcgggaaac gacaatctga tctaagctag cttggaattg 180

gtaccacgcg tttcgacaaa atttagaacg aacttaatta tgatctcaaa tacattgata 240

catatctcat ctagatctag gttatcatta tgtaagaaag ttttgacgaa tatggcacga 300

caaaatggct agactcgatg taattggtat ctcaactcaa cattatactt ataccaaaca 360

ttagttagac aaaatttaaa caactatttt ttatgtatgc aagagtcagc atatgtataa 420

ttgattcaga atcgttttga cgagttcgga tgtagtagta gccattattt aatgtacata 480

ctaatcgtga atagtgaata tgatgaaaca ttgtatctta ttgtataaat atccataaac 540

acatcatgaa agacactttc tttcacggtc tgaattaatt atgatacaat tctaatagaa 600

aacgaattaa attacgttga attgtatgaa atctaattga acaagccaac cacgacgacg 660

actaacgttg cctggattga ctcggtttaa gttaaccact aaaaaaacgg agctgtcatg 720

taacacgcgg atcgagcagg tcacagtcat gaagccatca aagcaaaaga actaatccaa 780

gggctgagat gattaattag tttaaaaatt agttaacacg agggaaaagg ctgtctgaca 840
gccaggtcac gttatcttta cctgtggtcg aaatgattcg tgtctgtcga ttttaattat 900
ttttttgaaa ggccgaaaat aaagttgtaa gagataaacc cgcctatata aattcatata 960
ttttcctctc cgctttgaag ttttagtttt attgcaacaa caacaacaaa ttacaataac 1020
aacaaacaaa atacaaacaa caacaacatg gcacaatttc aacaaacaat tgacatgcaa 1080
actctccaag ccgctgcggg acgcaacagc ttggtgaatg atttggcatc tcgtcgcgtt 1140
tacgataatg cagtcgagga gctgaatgct cgttccagac gtcccaaggt aataggaact 1200
ttctggatct actttatttg ctggatctcg atcttgtttt ctcaatttcc ttgagatctg 1260
gaattcgttt aatttggatc tgtgaacctc cactaaatct tttggtttta ctagaatcga 1320
tctaagttga ccgatcagtt agctcgatta tagctaccag aatttggctt gaccttgatg 1380
gagagatcca tgttcatgtt acctgggaaa tgatttgtat atgtgaattg aaatctgaac 1440
tgttgaagtt agattgaatc tgaacactgt caatgttaga ttgaatctga acactgttta 1500
aggttagatg aagtttgtgt atagattctt cgaaacttta ggatttgtag tgtcgtacgt 1560
tgaacagaaa gctatttctg attcaatcag ggtttatttg actgtattga actctttttg 1620
tgtgtttgca ggtccacttc tccaaggcag tgtctacgga acagaccctg attgcaacaa 1680
acgcatatcc ggagttcgag atttccttta ctcatacgca atccgctgtg cactccttgg 1740
ccggaggcct tcggtcactt gagttggagt atctcatgat gcaagttccg ttcggttctc 1800
tgacgtacga catcggcggt aacttttccg cgcacctttt caaagggcgc gattacgttc 1860
actgctgcat gcctaatctg gatgtacgtg acattgctcg ccatgaagga cacaaggaag 1920
ctatttacag ttatgtgaat cgtttgaaaa ggcagcagcg tcctgtgcct gaataccaga 1980
gggcagcttt caacaactac gctgagaacc cgcacttcgt ccattgcgac aaacctttcc 2040
aacagtgtga attgacgaca gcgtatggca ctgacaccta cgctgtagct ctccatagca 2100
tttatgatat ccctgttgag gagttcggtt ctgcgctact caggaagaat gtgaaaactt 2160
gtttcgcggc ctttcatttc catgagaata tgcttctaga ttgtgataca gtcacactcg 2220
atgagattgg agctacgttc cagaaatcag gtaacattcc ttagttacct ttcttttctt 2280
tttccatcat aagtttatag attgtacatg ctttgagatt tttctttgca aacaatctca 2340
ggtgataacc tgagcttctt cttccataat gagagcactc tcaattacac ccacagcttc 2400
agcaacatca tcaagtacgt gtgcaagacg ttcttccctg ctagtcaacg cttcgtgtac 2460
cacaaggagt tcctggtcac tagagtcaac acttggtact gcaagttcac gagagtggat 2520
acgttcactc tgttccgtgg tgtgtaccac aacaatgtgg attgcgaaga gttttacaag 2580
gctatggacg atgcgtggca ctacaaaaag acgttagcaa tgcttaatgc cgagaggacc 2640
atcttcaagg ataacgctgc gttaaacttc tggttcccga aggtgctctt gaaattggaa 2700
gtcttctttt gttgtctaaa cctatcaatt tctttgcgga aatttatttg aagctgtaga 2760

gttaaaattg agtcttttaa acttttgtag gtgagagaca tggttatcgt ccctctcttt 2820

gacgcttcta tcacaactgg taggatgtct aggagagagg ttatggtgaa caaggacttc 2880

gtctacacgg tcctaaatca catcaagacc tatcaagcta aggcactgac gtacgcaaac 2940

gtgctgagct tcgtggagtc tattaggtct agagtgataa ttaacggtgt cactgccagg 3000

taagttgtta cttatgattg ttttcctctc tgctacatgt attttgttgt tcatttctgt 3060

aagatataag aattgagttt tcctctgatg atattattag gtctgaatgg gacacagaca 3120

aggcaattct aggtccatta gcaatgacat tcttcctgat cacgaagctg ggtcatgtgc 3180

aagatgaaat aatcctgaaa aagttccaga agttcgacag aaccaccaat gagctgattt 3240

ggacaagtct ctgcgatgcc ctgatggggg ttattccctc ggtcaaggag acgcttgtgc 3300

gcggtggttt tgtgaaagta gcagaacaag ccttagagat caaggttagt atcatatgaa 3360

gaaataccta gtttcagttg atgaatgcta ttttctgacc tcagttgttc tcttttgaga 3420

attatttctt ttctaatttg cctgattttt ctattaattc attaggttcc cgagctatac 3480

tgtaccttcg ccgaccgatt ggtactacag tacaagaagg cggaggagtt ccaatcgtgt 3540

gatctttcca aacctctaga agagtcagag aagtactaca acgcattatc cgagctatca 3600

gtgcttgaga atctcgactc ttttgactta gaggcgttta agactttatg tcagcagaag 3660

aatgtggacc cggatatggc agcaaaggta aatcctggtc cacactttta cgataaaaac 3720

acaagatttt aaactatgaa ctgatcaata atcattccta aaagaccaca cttttgtttt 3780

gtttctaaag taatttttac tgttataaca ggtggtcgta gcaatcatga agtcagaatt 3840

gacgttgcct ttcaagaaac ctacagaaga ggaaatctcg gagtcgctaa aaccaggaga 3900

ggggtcgtgt gcagagcata aggaagtgtt gagcttacaa aatgatgctc cgttcccgtg 3960

tgtgaaaaat ctagttgaag gttccgtgcc ggcgtatgga atgtgtccta agggtggtgg 4020

tttcgacaaa ttggatgtgg acattgctga tttccatctc aagagtgtag atgcagttaa 4080

aaagggaact atgatgtctg cggtgtacac agggtctatc aaagttcaac aaatgaagaa 4140

ctacatagat tacttaagtg cgtcgctggc agctacagtc tcaaacctct gcaaggtaag 4200

aggtcaaaag gtttccgcaa tgatccctct ttttttgttt ctctagtttc aagaatttgg 4260

gtatatgact aacttctgag tgttccttga tgcatatttg tgatgagaca aatgtttgtt 4320

ctatgtttta ggtgcttaga gatgttcacg gcgttgaccc agagtcacag gagaaatctg 4380

gagtgtggga tgttaggaga ggacgttggt tacttaaacc taatgcgaaa agtcacgcgt 4440

ggggtgtggc agaagacgcc aaccacaagt tggttattgt gttactcaac tgggatgacg 4500

gaaagccggt ttgtgatgag acatggttca gggtggcggt gtcaagcgat tccttgatat 4560

attcggatat gggaaaactt aagacgctca cgtcttgcag tccaaatggt gagccaccgg 4620

agcctaacgc caaagtaatt ttggtcgatg gtgttcccgg ttgtggaaaa acgaaggaga 4680

ttatcgaaaa ggtaagttct gcatttggtt atgctccttg cattttaggt gttcgtcgct 4740

cttccatttc catgaatagc taagattttt tttctctgca ttcattcttc ttgcctcagt 4800

tctaactgtt tgtggtattt ttgttttaat tattgctaca ggtaaacttc tctgaagact 4860
tgattttagt ccctgggaag gaagcttcta agatgatcat ccggagggcc aaccaagctg 4920
gtgtgataag agcggataag gacaatgtta gaacggtgga ttccttcttg atgcatcctt 4980
ctagaagggt gtttaagagg ttgtttatcg atgaaggact aatgctgcat acaggttgtg 5040
taaatttcct actgctgcta tctcaatgtg acgtcgcata tgtgtatggg gacacaaagc 5100
aaattccgtt catttgcaga gtcgcgaact ttccgtatcc agcgcatttt gcaaaactcg 5160
tcgctgatga gaaggaagtc agaagagtta cgctcaggta aagcaactgt gttttaatca 5220
atttcttgtc aggatatatg gattataact taatttttga gaaatctgta gtatttggcg 5280
tgaaatgagt ttgctttttg gtttctcccg tgttataggt gcccggctga tgttacgtat 5340
ttccttaaca agaagtatga cggggcggtg atgtgtacca gcgcggtaga gagatccgtg 5400
aaggcagaag tggtgagagg aaagggtgca ttgaacccaa taaccttacc gttggagggt 5460
aaaattttga ccttcacaca agctgacaag ttcgagttac tggagaaggg ttacaaggta 5520
aagtttccaa ctttccttta ccatatcaaa ctaaagttcg aaacttttta tttgatcaac 5580
ttcaaggcca cccgatcttt ctattcctga ttaatttgtg atgaatccat attgactttt 5640
gatggttacg caggatgtga acactgtgca cgaggtgcaa ggggagacgt acgagaagac 5700
tgctattgtg cgcttgacat caactccgtt agagatcata tcgagtgcgt cacctcatgt 5760
tttggtggcg ctgacaagac acacaacgtg ttgtaaatat tacaccgttg tgttggaccc 5820
gatggtgaat gtgatttcag aaatggagaa gttgtccaat ttccttcttg acatgtatag 5880
agttgaagca ggtctgtctt tcctatttca tatgtttaat cctaggaatt tgatcaattg 5940
attgtatgta tgtcgatccc aagactttct tgttcactta tatcttaact ctctctttgc 6000
tgtttcttgc aggtgtccaa tagcaattac aaatcgatgc agtattcagg ggacagaact 6060
tgtttgttca gacgcccaag tcaggagatt ggcgagatat gcaattttac tatgacgctc 6120
ttcttcccgg aaacagtact attctcaatg aatttgatgc tgttacgatg aatttgaggg 6180
atatttcctt aaacgtcaaa gattgcagaa tcgacttctc caaatccgtg caacttccta 6240
aagaacaacc tattttcctc aagcctaaaa taagaactgc ggcagaaatg ccgagaactg 6300
caggtaaaat attggatgcc agacgatatt ctttcttttg atttgtaact ttttcctgtc 6360
aaggtcgata aattttattt tttttggtaa aaggtcgata attttttttt ggagccatta 6420
tgtaattttc ctaattaact gaaccaaaat tatacaaacc aggtttgctg gaaaatttgg 6480
ttgcaatgat caaaagaaac atgaatgcgc cggatttgac agggacaatt gacattgagg 6540
atactgcatc tctggtggtt gaaaagtttt gggattcgta tgttgacaag gaatttagtg 6600
gaacgaacga aatgaccatg acaaggagag cttctccag gtaaggactt ctcatgaata 6660
ttagtggcag attagtgttg ttaaagtctt tggttagata atcgatgcct cctaattgtc 6720
catgttttac tggttttcta caattaaagg tggctttcga aacaagagtc atctacagtt 6780

ggtcagttag cggactttaa ctttgtggat ttgccggcag tagatgagta caagcatatg 6840
atcaagagtc aaccaaagca aaagttagac ttgagtattc aagacgaata tcctgcattg 6900
cagacgatag tctaccattc gaaaaagatc aatgcgattt tcggtccaat gttttcagaa 6960
cttacgagga tgttactcga aaggattgac tcttcgaagt ttctgttcta caccagaaag 7020
acacctgcac aaatagagga cttcttttct gacctagact caacccaggc gatggaaatt 7080
ctggaactcg acatttcgaa gtacgataag tcacaaaacg agttccattg tgctgtagag 7140
tacaagatct gggaaaagtt aggaattgat gagtggctag ctgaggtctg gaaacaaggt 7200
gagttcctaa gttccatttt tttgtaatcc ttcaatgtta ttttaacttt tcagatcaac 7260
atcaaaatta ggttcaattt tcatcaacca aataatattt ttcatgtata tataggtcac 7320
agaaaaacga ccttgaaaga ttatacggcc ggaatcaaaa catgtctttg gtatcaaagg 7380
aaaagtggtg atgtgacaac ctttattggt aataccatca tcattgccgc atgtttgagc 7440
tcaatgatcc ccatggacaa agtgataaag gcagcttttt gtggagacga tagcctgatt 7500
tacattccta aaggtttaga cttgcctgat attcaggcgg gcgcgaacct catgtggaac 7560
ttcgaggcca aactcttcag gaagaagtat ggttacttct gtggtcgtta tgttattcac 7620
catgatagag gagccattgt gtattacgat ccgcttaaac taatatctaa gttaggttgt 7680
aaacatatta gagatgttgt tcacttagaa gagttacgcg agtctttgtg tgatgtagct 7740
agtaacttaa ataattgtgc gtatttttca cagttagatg aggccgttgc cgaggttcat 7800
aagaccgcgg taggcggttc gtttgctttt tgtagtataa ttaagtattt gtcagataag 7860
agattgttta gagatttgtt ctttgtttga taatgtcgat agtctcgtac gaacctaagg 7920
tgagtgattt cctcaatctt tcgaagaagg aagagatctt gccgaaggct ctaacgaggt 7980
taaaaaccgt gtctattagt actaaagata ttatatctgt caaggagtcg gagactttgt 8040
gtgatataga tttgttaatc aatgtgccat tagataagta tagatatgtg ggtatcctag 8100
gagccgtttt taccggagag tggctagtgc cagacttcgt taaaggtgga gtgacgataa 8160
gtgtgataga taagcgtctg gtgaactcaa aggagtgcgt gattggtacg tacagagccg 8220
cagccaagag taagaggttc cagttcaaat tggttccaaa ttactttgtg tccaccgtgg 8280
acgcaaagag gaagccgtgg caggtaagga tttttatgat atagtatgct tatgtatttt 8340
gtactgaaag catatcctgc ttcattggga tattactgaa agcatttaac tacatgtaaa 8400
ctcacttgat gatcaataaa cttgattttg caggttcatg ttcgtataca agacttgaag 8460
attgaggcgg gttggcagcc gttagctctg gaagtagttt cagttgctat ggtcaccaat 8520
aacgttgtca tgaagggttt gagggaaaag gtcgtcgcaa taaatgatcc ggacgtcgaa 8580
ggtttcgaag gtaagccatc ttcctgctta tttttataat gaacatagaa ataggaagtt 8640
gtgcagagaa actaattaac ctgactcaaa atctaccctc ataattgttg tttgatattg 8700
gtcttgtatt ttgcaggtgt ggttgacgaa ttcgtcgatt cggttgcagc atttaaagcg 8760
gttgacaact ttaaaagaag gaaaaagaag gttgaagaaa agggtgtagt aagtaagtat 8820

aagtacagac cggagaagta cgccggtcct gattcgttta atttgaaaga agaaaacgtc 8880
ttacaacatt acaaacccga atcagtacca gtatttcgat aagaaacaag aaaccatggc 8940
actcaagaca cagttgttgt ggtcattcgt ggttgtgttc gttgtgtcct tcagtacaac 9000
ttcatgctca ggtgcaggct gttctgttga ttacaccgtg aattcttggg gaactggatt 9060
cacggctaac gttaccatca cgaatcttgg ttctgccata aacggatgga cattagagtg 9120
ggacttccct ggcaaccagc aagttaccaa cctgtggaat ggcacgtata cccagtctgg 9180
gcaacacgtt tctgtttcta atgctcctta caacgcatct atccctgcaa atggcactgt 9240
cgaattcggc tttaatggga gctattctgg aagcaacgac atacctagct cttttaagct 9300
gaacggtgta acatgtgacg ggagcgacga tccagatcct gaaccttctc catcaccttc 9360
tccatctccc agccctactg atcctgatga acctggtgga ccaaccaatc ctccaacaaa 9420
ccctggcgag aaagtggaca atcccttcga gggtgccaaa ctttacgtaa accctgtatg 9480
gagtgctaaa gctgcagctg aacctggtgg atctgctgta gccaatgaat ctactgctgt 9540
ttggctggat agaatcggtg ccatagaggg aaacgattct cctacaactg ggtctatggg 9600
tcttagagat catctcgagg aagccgtaag acaaagtggt ggcgatcccc ttaccatcca 9660
agttgtgatc tacaatctac ctggtcgcga ctgtgctgca cttgcttcta atggagaatt 9720
gggacctgat gaactggata gatacaagtc cgagtacatt gatcccattg ctgacatcat 9780
gtgggacttc gctgactatg agaacctacg aatcgtcgcc atcattgaga tagactccct 9840
ccctaatcta gtcactaatg tcggaggcaa cggagggact gagctatgtg catacatgaa 9900
gcaaaacgga ggctacgtca atggagttgg ctatgccctt agaaagttgg gtgagattcc 9960
taacgtctac aactacatcg atgccgctca ccatggctgg ataggctggg attctaactt 10020
tggaccctcc gtggacatct tctacgaagc tgcaaatgcc tctggtagca ccgttgacta 10080
tgtgcatgga ttcatctcta acacggcaaa ctatagcgct actgttgaac cgtatctaga 10140
cgtaaacggc accgtgaatg ggcaactgat aaggcaatct aagtgggtcg actggaacca 10200
atacgttgat gagttgagtt tcgtccaaga cttgaggcaa gctctcatag ccaaagggtt 10260
tcgttctgat atcggcatgc tcatagacac ttctagaaac ggatggggag gccctaatcg 10320
tccaactgga cctagctcat ctacggacct taacacatac gttgacgagt cacgaattga 10380
tagacgcata caccctggaa actggtgcaa ccaagctggt gctggcttgg gtgaaagacc 10440
taccgtgaac ccagctcctg gtgttgacgc atacgtttgg gttaagcctc cgggagaatc 10500
tgatggagct tccgaggaaa tccctaatga tgaggggaag ggcttcgata gaatgtgcga 10560
tcctacttac caaggcaacg ctagaaacgg caacaatcca tctggtgctc tacctaatgc 10620
tcccatatct gggcactggt tttctgccca atttcgggag cttctagcta atgcttaccc 10680
tcccctctaa gcttactaga gcgtggtgcg cacgatagcg catagtgttt ttctctccac 10740
ttgaatcgaa gagatagact tacggtgtaa atccgtaggg gtggcgtaaa ccaaattacg 10800

caatgttttg ggttccattt aaatcgaaac cccttatttc ctggatcacc tgttaacgca 10860

cgtttgacgt gtattacagt gggaataagt aaaagtgaga ggttcgaatc ctccctaacc 10920

ccgggtaggg gcccagcggc cgctctagct agagtcaagc agatcgttca aacatttggc 10980

aataaagttt cttaagattg aatcctgttg ccggtcttgc gatgattatc atataatttc 11040

tgttgaatta cgttaagcat gtaataatta acatgtaatg catgacgtta tttatgagat 11100

gggtttttat gattagagtc ccgcaattat acatttaata cgcgatagaa aacaaaatat 11160

agcgcgcaaa ctaggataaa ttatcgcgcg cggtgtcatc tatgttacta gatcgacctg 11220

catccacccc agtacattaa aaacgtccgc aatgtgttat taagttgtct aagcgtcaat 11280

ttgtttacac cacaatatat cctgccacca gccagccaac agctccccga ccggcagctc 11340

ggcacaaaat caccactcga tacaggcagc ccatcag 11377

## Description

### Field of the Invention

The present invention relates to a process of producing a preparation of enzymes capable of degrading plant cell walls or components thereof. The invention also relates to a process of storing cell wall-degrading enzymes and to silage containing one or more than one active cell wall-degrading enzymes. The preparation of cell wall-degrading enzymes can be used for degrading plant cell wall components of plant material, such as in the production of bioethanol from plant material, notably from cellulose.

### Background of the Invention

Production of bioalcohol, mostly of bioethanol, is widely used throughout the world as an additive to fuel and gasoline as an at least partial replacement of fossil sources for fuels. Concerns, whether justified or not, with climate change aggravated by fossil fuel-derived carbon dioxide have led to a steady increase of bioethanol production in recent years. However, bioethanol production based on plants grown on farm fields consumes enormous areas of limited agricultural land and thus competes with food production and leads to increased food prices.

Conventional processes for bioethanol production rely on fermentation of glucose derived from starch. Although cellulose that is a major component of plant cell walls also consists of glucose units, cellulose-based bioethanol (cellulosic bioethanol) has so far not played a significant role in bioethanol production, since breakdown of cellulose to glucose is difficult on an industrial scale. For example, chemical hydrolysis steps to break down cellulose to cellulose fragments or glucose are frequently used before microbial processes are employed. For these reasons, cellulose, albeit abundantly produced by plants, is difficult to convert to bioethanol. Improvements in the use of cellulose for bioalcohol production are therefore highly desired.

In order to avoid chemical hydrolysis or pre-hydrolysis steps in the breakdown of cellulose, enzymatic processes are widely pursued (see e.g. Bansal et al., Biotechnology Advances 27 (2009) 833-848). However, multiple enzymes are required to break down cellulose or plant cell wall material to glucose. Further, the enzymes required for the breakdown need to be produced in production processes independent from the glucose fermentation process, and represent a high cost factor for the production cellulosic bioalcohol. Further, these production processes are sophisticated processes requiring expert knowledge and equipment as found in the biotech industry, but cannot be carried out decentralized such as by farmers on farms where the plant material to be converted to bioalcohol is produced, which makes the entire bioalcohol production process from cellulose complex and non-economical.

Departing from the prior art, it is a problem of the present invention to provide plant cell wall-degrading enzymes in an economical way. It is a further problem of the invention to provide a process for producing and/or storing plant cell wall-degrading enzymes that can be carried out easily such as by farmers.

### Summary of the Invention

These problems are solved by
(1) A process of producing a preparation of cell wall-degrading enzymes, comprising: (i) expressing in plants or plant parts one or more than one heterologous cell wall-degrading enzyme; (ii) harvesting said plants or plant part containing said one or more than one cell wall-degrading enzyme; and (iii) storing the harvest as a silage.
(2) A process of producing a preparation of cell wall-degrading enzymes, comprising: (i) transfecting plants or parts thereof with a nucleic acid molecule comprising a nucleic acid construct containing a nucleotide sequence encoding one or more than one cell wall-degrading enzyme and expressing in said plants or parts thereof said one or more than one cell wall-degrading enzyme; (ii) harvesting said plants or parts containing said one or more than one cell wall-degrading enzyme; and (iii) storing the harvest as a silage.
(3) The process according to (1) or (2), wherein said one or more than one cell wall-degrading enzyme is a cellulolytic enzyme such as endoglucanases, exoglucanases, exocellulases, β-glucosidases, or combinations thereof; hemicellulases; pectinases; and/or lignin degrading enzymes.
(4) The process according to any one of (1) to (3), wherein said cell wall-degrading enzymes are secretory enzymes in the organism from which they are derived.
(5) The process according to any one of (1) to (4), wherein step (iii) comprises storing said harvested plants or parts thereof for at least 1 month in an atmosphere of reduced oxygen at ambient temperature.
(6) The process according to any one of (1) to (5), wherein step (iii) comprises lactic acid bacteria fermentation or acidification to produce acidic conditions in said silage.
(7) The process according to any one of (1) to (6), wherein said cell wall-degrading enzymes expressed in the cytosol of cells of said plants or parts thereof, or said cell wall-degrading enzymes are targeted to the apoplast or to plastids of said plant or plant parts.
(8) The process according to (1), wherein step (i) comprises transfecting said plants or parts thereof with a nucleic acid molecule comprising a nucleic acid construct containing a DNA sequence encoding said one or more cell wall-degrading enzyme(s).
(9) The process according to any one of (1) to (8), wherein step (i) comprises spraying aerial parts of said plant with an aqueous suspension containing cells of an *Agrobacterium* strain comprising a DNA molecule comprising a nucleic acid construct containing a DNA sequence encoding said one or more than one cell wall-degrading enzyme.
(10) The process according to any one of (1) to (8), wherein step (i) comprises spraying aerial parts of said plant with an aqueous suspension containing cells of an *Agrobacterium* strain comprising a DNA molecule comprising a nucleic acid construct encoding a DNA or RNA replicon encoding said one or more than one cell wall-degrading enzyme.
(11) The process according to any one of (1) to (10), wherein step (iii) comprises storing the harvested plants or plant parts in a closed container or bag under conditions of reduced oxygen content for enabling lactic acid fermentation.
(12) A process of storing plant cell wall-degrading enzymes, comprising storing plants or plant parts containing expressed cell wall-degrading enzymes in a silage, preferably under conditions as defined in (11) above; said plants or plant parts may contain said cell wall-degrading enzymes targeted into the apoplast or into plastids of said plant or plant parts.
(13) Silage containing one or more than one active cell wall-degrading enzyme, said silage obtainable by storing plant or plant parts containing expressed cell wall-degrading enzymes under reduced oxygen content for allowing lactic acid fermentation, wherein said one or more than one cell wall-degrading enzyme may be heterologous to said plant or said plant part.
(14) A process of degrading cell wall components of plant material, comprising treating said plant material in an aqueous reaction medium with the silage of (13) or with the silage obtainable according to any one of (1) to (11).
(15) The process according to (14), wherein said silage contains cell wall-degrading enzymes selected from endoglucanases, exoglucanases, exocellulases, hemicellulases, β-glucosidases, pectinases, lignin-degrading enzymes and combinations thereof.
(16) The process according to (14) or (15), wherein a silage containing an endoglucanase, a silage containing an exoglucanase, a silage containing an exocellulase, and a silage containing a β-glucosidase are mixed in desired ratio before treatment of said plant material to obtain a desired ratio of said cellulolytic enzymes, and treating said plant material in an aqueous reaction medium with the mixtures of silages.
(17) A process of producing a bioalcohol such as bioethanol from plant material, comprising: (a) treating said plant material, or a product obtained in pre-treatment of said plant material, in an aqueous reaction medium with the silage of (13) or with the silage obtainable according to any one of (1) to (11) for producing glucose from cellulose contained in said plant material, (b) fermenting the product obtained in the previous step for producing said bioalcohol from said glucose, (c) optionally isolating said bioalcohol from the reaction medium of step (b).

The inventors have surprisingly found that cell wall-degrading enzymes when expressed in plants, notably when expressed to a high level, can be stored as a silage of these plants over extended periods of time with little loss of enzymatic activity. Thus, expressed cell wall-degrading enzymes do not need to be purified from other plant components, such as proteases, for maintaining activity, which saves substantial costs. The inventors have further found that enzymes that degrade plant cell wall material are of exceptional stability upon storage of the plant or plant parts having expressed the enzymes. The stability under these conditions is higher than that of many native proteins. Accordingly, storage in silage leads to an enrichment of these enzymes in total soluble protein. In the form of the silage of the plants or plant parts containing expressed cell wall-degrading enzymes, the cell wall-degrading enzymes can be stored until the enzymes are required, e.g. for a process of degrading plant cell wall components of plant material in a process of producing a bioalcohol. Upon use, the silage can be added, in a reaction medium, to the plants or plant material cell wall material of which should be degraded. The invention has the advantage that plants containing cell wall-degrading enzymes can be easily produced and stored on a farm using techniques generally known to a farmer, as ensiling of forage is a widely used method for preserving animal feed over periods where fresh forage is limited. Purification of pant cell wall-degrading enzymes and costs associated therewith can be avoided. In the production of bioalcohol, the silage also enters the saccharification and alcohol fermentation process. Thus, the invention is of unprecedented efficiency and cost-effectiveness.

### Brief Description of the Figures

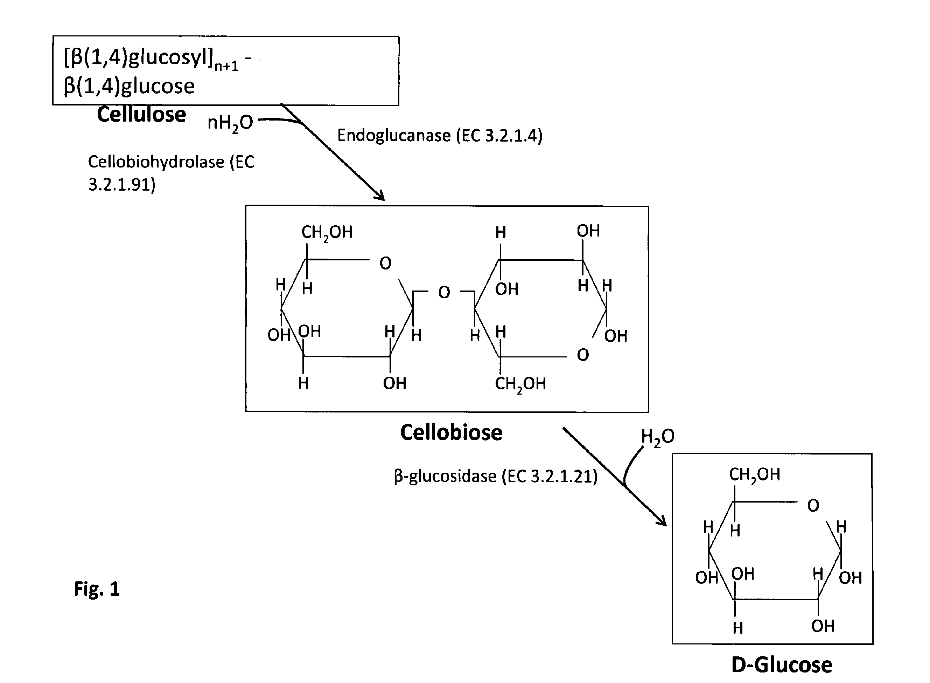

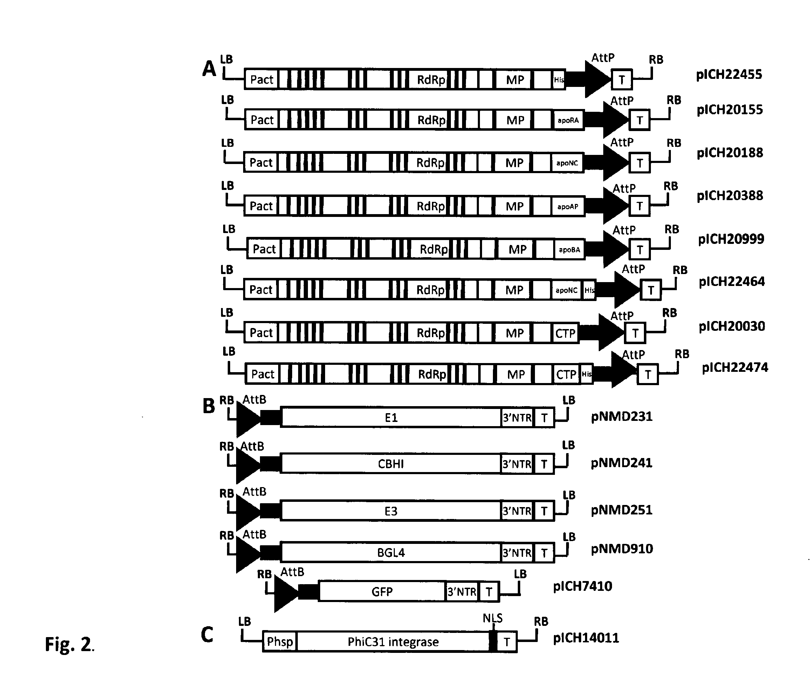

Fig. 1 outlines the relationship between the enzyme activities involved in the hydrolysis of cellulose. Endoglucanases cleave cellulose chains internally, providing free reducing and nonreducing chain termini that are subject to cellobiohydrolase (exoglucanase) cleavage. Cellobiohydrolases cleave cellulose chains termini releasing cellooligosaccharides, preferentially cellobiose. β-glucosidase hydrolyses cellobiose to glucose.
Fig. 2 shows schematically vectors used for the optimization of transient expression of cell wall degrading enzymes.
   (A) TMV-based 5'-provectors modules: pICH22455 construct for cytosolic expression of proteins of interest with N-terminal His (6)-tag and enterokinase cleavage site (His-EK); constructs for apoplast targeting pICH20155, pICH20188, pICH20388 and pICH20999 containing apoplast targeting presequences from rice amylase (apoRA), *Nicotiana plumbaginifolia* calreticulin (apoNC), apple pectinase (apoAP) and barley amylase (apoBA), respectively. Plasmid pICH22464 contains apoplast targeting presequence from *Nicotiana plumbaginifolia* calreticulin followed by His(6)-tag and enterokinase cleavage site (His-EK). pICH20030 construct for chloroplast targeting of expressed protein of interest contains chloroplast targeting presequence (CTP).
   (*B*) TMV-based 3'-provector modules: 3' modules for the expression of endoglucanase E1 from *Acidothermus cellulolyticus* (pNMD231), Exoglucanase 1 (CBHI) from *Trichoderma reesei* (pNMD241), exocellulase E3 from *Thermobifida fusca* (pNMD251), β-glucosidase BGL4 from *Humicola grisea* (pNMD910) and GFP (pICH7410). (*C*) Integrase module pICH14011. LB and RB, binary left and right borders, respectively; Pact, *Arabidopsis* actin 2 promoter; Phsp, *Arabidopsis* heat shock protein hsp 81.1 promoter; T, *nos* terminator; RdRp, RNA-dependent RNA polymerase; MP, movement protein; int, intron; AttP and AttB, recombination sites; apoRA, apoNC, apoAP and apoBA, apoplast targeting presequences from rice α-amylase, *Nicotiana plumbaginifolia* calreticulin, apple pectinase and barley amylase, respectively; CTP, chloroplast targeting presequence; NLS, nuclear localization signal.

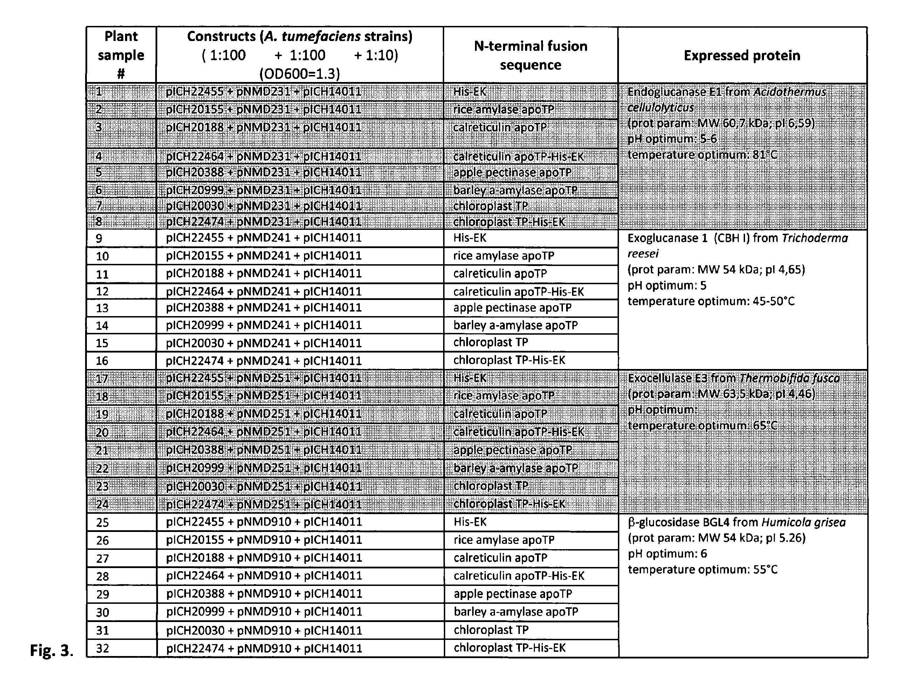

Fig. 3 provides an overview over agrobacterial inoculation for the optimisation of transient expression of cellulases in *Nicotiana benthamiana* as translational fusions to different N-terminal sequences. For each fusion protein, samples were inoculated into 3 different plants; plant material was harvested at 7 or 8 dpi (days post inoculation).

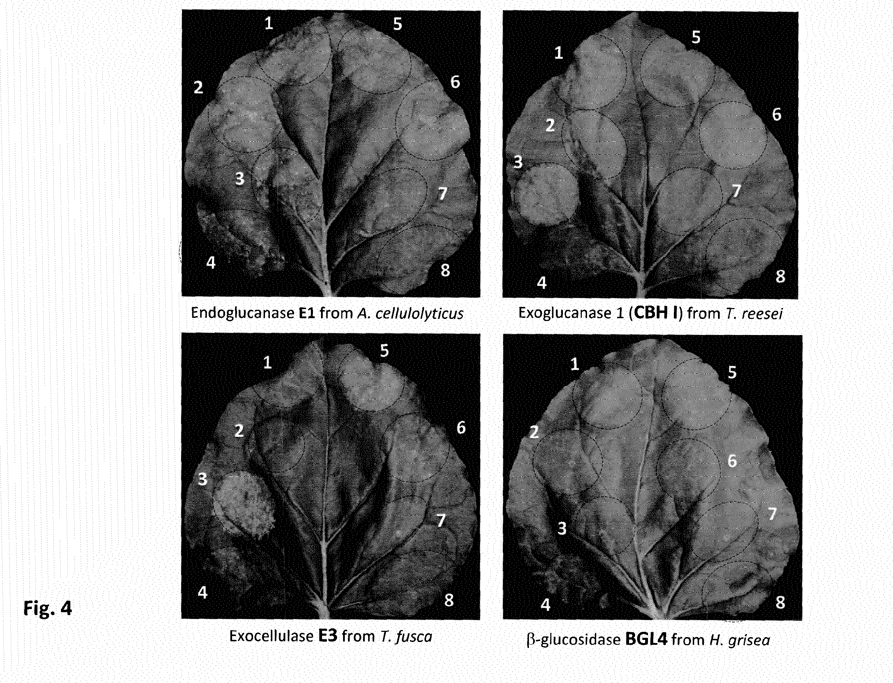

Fig. 4 photographs showing phenotypes of *Nicotiana benthamiana* leaves transiently expressing endoglucanase E1 from *Acidothermus cellulolyticus,* exoglucanase 1 (CBHI) from *Trichoderma reesei,* exocellulase E3 from *Thermobifida fusca* and β-glucosidase Bgl4 from *Humicola grisea* as fusion to different sequences at 8 dpi. N-terminal fusion sequences:
   1 - His(6)-tag and enterokinase cleavage site (His-EK);
   2 - rice amylase apoplast targeting presequence;
   *3 - Nicotiana plumbaginifolia* calreticulin apoplast targeting presequence;
   *4 - Nicotiana plumbaginifolia* calreticulin apoplast targeting presequence followed by His(6)-tag and enterokinase cleavage site (His-EK);
   5 - apple pectinase apoplast targeting presequence;
   6 - barley α-amylase apoplast targeting presequence;
   7 - chloroplast targeting presequence;
   8 - chloroplast targeting presequence followed by His(6)-tag and enterokinase cleavage site (His-EK).

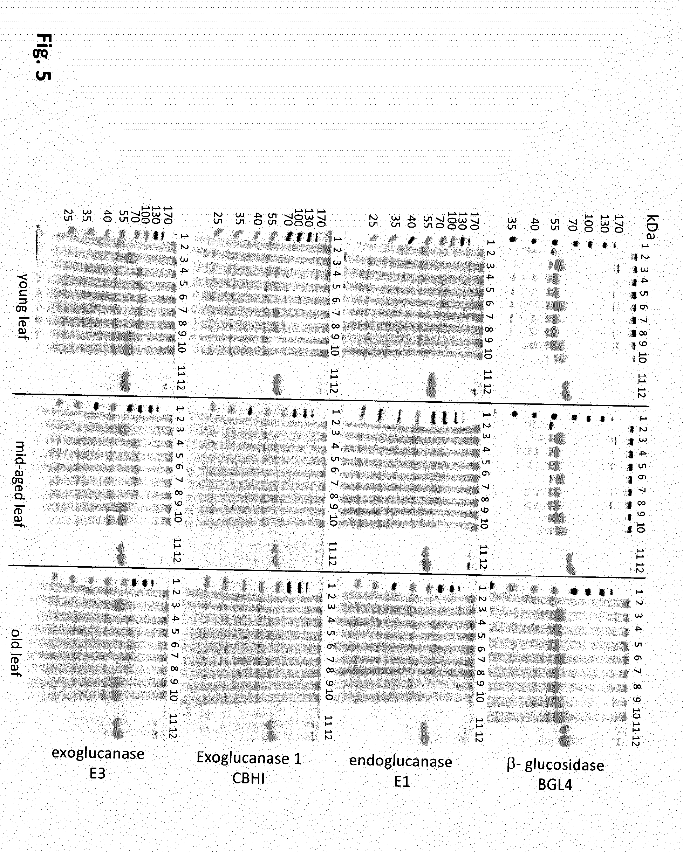

Fig. 5 shows results of optimisation of transient expression of cellulases via screening cellulase fusions to different N-terminal sequences for highest expression using TMV provectors. 1:100 dilutions of *Agrobacterium* cultures were inoculated by syringe; plant material was harvested at 11 dpi. Protein extracts containing 10 µg TSP were separated in 10% SDS-PAGE and stained using Coomassie. Leaves of different ages (young, mid-aged and old) were analyzed for each plant. The lanes are as follows:
   1 - PageRuler^{™} Prestained Protein Ladder (#SM0671, Fermentas);
   2 - uninfected leaf tissue;
   3 - fusion with His(6)-tag followed by enterokinase cleavage site (His-EK);
   4 - fusion with rice amylase apoplast targeting presequence;
   5 - fusion with *Nicotiana plumbaginifolia* calreticulin apoplast targeting presequence;
   6 - fusion with *Nicotiana plumbaginifolia* calreticulin apoplast targeting presequence followed by His(6)-tag and enterokinase cleavage site (His-EK);
   7 - fusion with apple pectinase apoplast targeting presequence;
   8 - fusion with barley α-amylase apoplast targeting presequence;
   9 - fusion with chloroplast targeting presequence;
   10 - fusion with chloroplast targeting presequence followed by His(6)-tag and enterokinase cleavage site (His-EK);
   11 - Bovine Serum Albumin (Sigma, A4503);
   12 - Bovine Serum Albumin (A4503, Sigma).

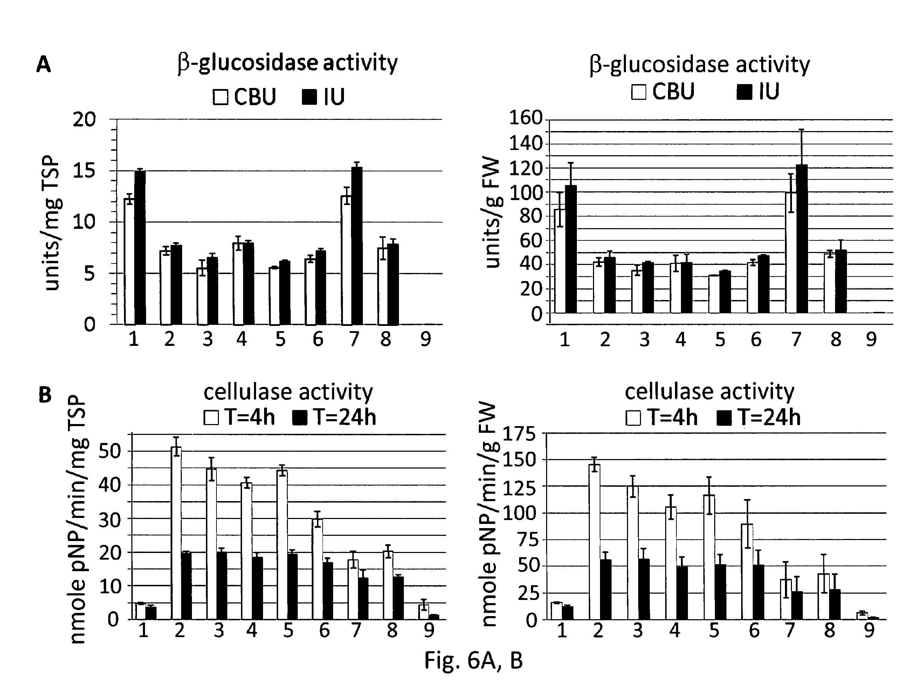

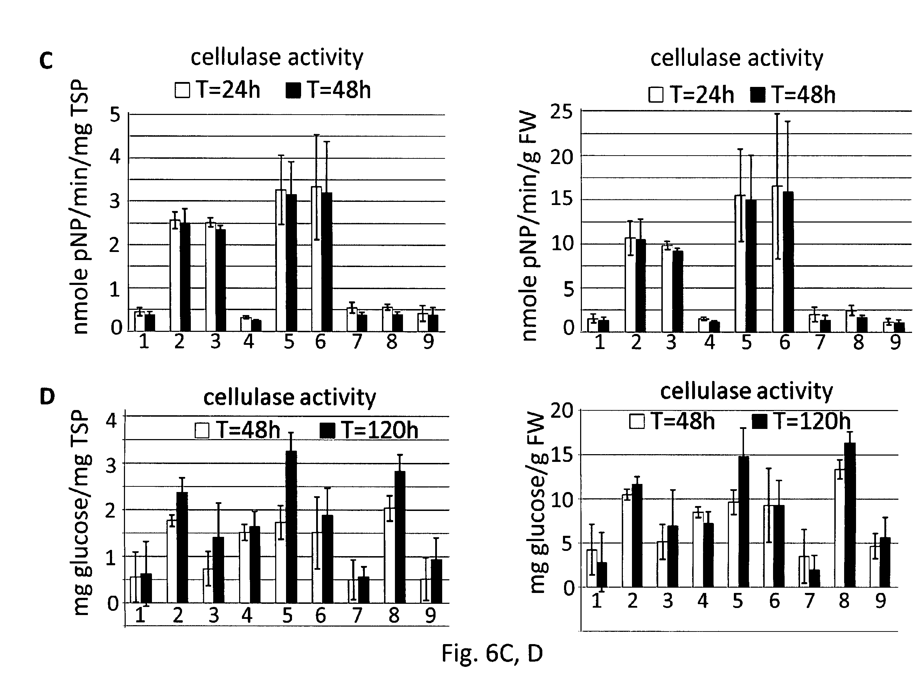

Fig. 6 shows the optimisation of transient expression of cellulases via screening cellulase fusions to different N-terminal sequences for highest enzymatic activity per mg TSP and grams fresh weight of plant tissue using TMV provectors.
   (*A*) β-glucosidase Bgl4, (*B*) endoglucanase E1, (*C*) exoglucanase CBHI, (*D*) exoglucanase E3. TSP (total soluble protein) extracts are prepared from plant material expressing the following cellulase fusions:
      1 - His(6)-tag and enterokinase cleavage site (His-EK);
      2 - rice amylase apoplast targeting presequence;
      3 - calreticulin apoplast targeting presequence;
      4 - calreticulin apoplast targeting presequence followed by His(6)-tag and enterokinase cleavage site (His-EK);
      5 - apple pectinase apoplast targeting presequence;
      6 - barley α-amylase apoplast targeting presequence;
      7 - chloroplast targeting presequence;
      8 - chloroplast targeting presequence followed by His(6)-tag and enterokinase cleavage site (His-EK);
      9 - TSP samples prepared from non-infected plant material.

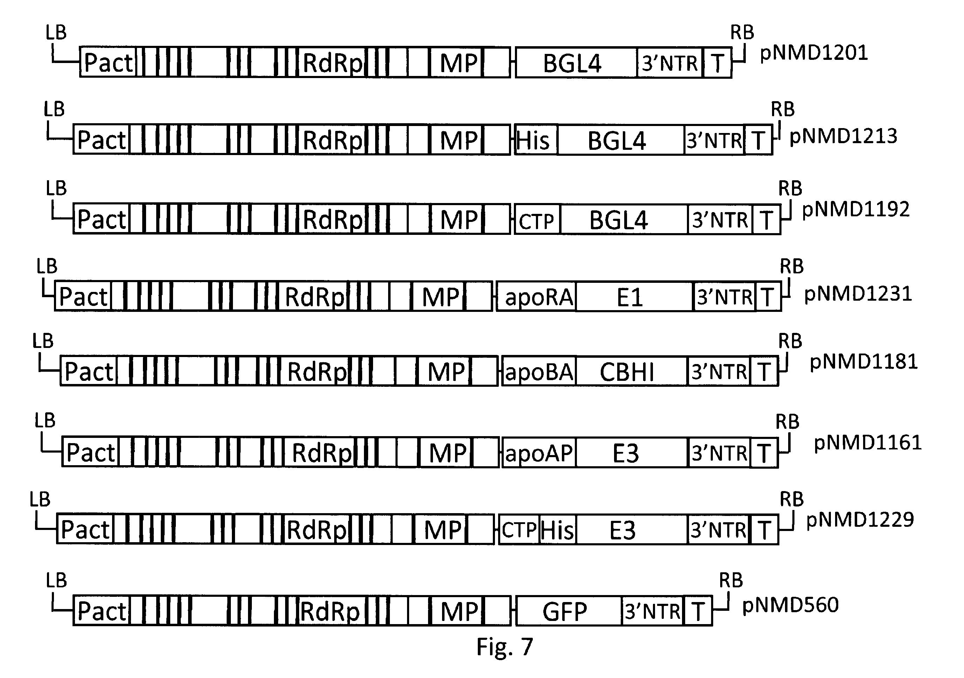

Fig. 7 shows schematically TMV-based assembled vectors used for the preparative large-scale production of cell wall degrading enzymes:
   pNMD1201 construct for cytosolic expression of β-glucosidase BGL4 from *Humicola grisea,*
   pNMD1213 construct for the expression of BGL4 with N-terminal His (6)-tag and enterokinase cleavage site (His-EK),
   pNMD1192 construct for the expression of BGL4 with chloroplast targeting presequence, pNMD1231 construct for the expression of endoglucanase E1 from *Acidothermus cellulolyticus* as a fusion with apoplast targeting presequence from rice amylase,
   pNMD1181 construct for the expression of exoglucanase 1 (CBHI) from *Trichoderma reesei* as a fusion with barley amylase apoplast targeting presequence,
   pNMD1161 construct for the expression of exocellulase E3 from *Thermobifida fusca* as a fusion with apple pectinase apoplast targeting presequence,
   pNMD1129 construct for the expression of E3 as a fusion with chloroplast targeting presequence followed by His (6)-tag and enterokinase cleavage site (His-EK). For the annotation of construct elements, see the Fig. 2 description.

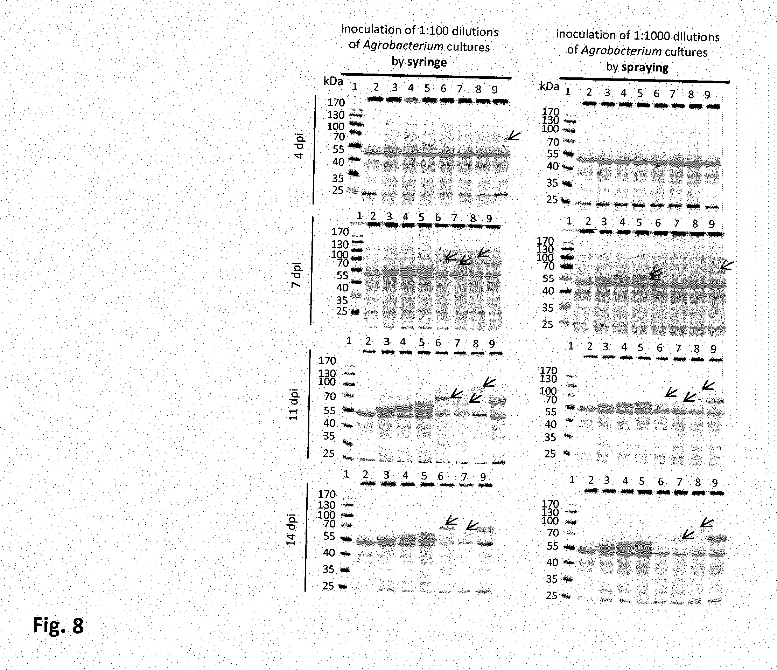

Fig. 8 depicts the analysis of different inoculation methods for highest cellulase expression of selected cellulase fusions: syringe infiltration vs spraying with suspension of agrobacteria harboring assembled TMV constructs. SDS-PAGE analysis of cellulase expression in crude extracts. Arrows indicate the bands of the cellulase expressed in cases where the cellulase is not strongly expressed compared to other bands.
   1 - PageRuler^{™} Prestained Protein Ladder (#SM0671, Fermentas);
   2 - uninfected leaf tissue;
   3- β-glucosidase BGL4 from *Humicola grisea* expressed in cytosol (pNMD1201 construct);
   4 - Bgl4 expressed in cytosol as a fusion with His(6)-tag followed by enterokinase cleavage site (His-EK) (pNMD1213 construct);
   5 - Bgl4 targeted to chloroplast (pNMD1192 construct);
   6 - endoglucanase E1 from *Acidothermus cellulolyticus* targeted to the apoplast via fusion with rice amylase presequence (pNMD1231);
   7 - exoglucanase 1 (CBHI) from *Trichoderma reesei* targeted to apoplast via fusion with barley α-amylase presequence (pNMD1181);
   8 - exocellulase E3 from *Thermobifida fusca* targeted to apoplast via fusion with apple pectinase presequence (pNMD1161);
   9 - E3 targeted to chloroplast (chloroplast targeting presequence followed by His(6)-tag and enterokinase cleavage site (His-EK)) (pNMD1129).

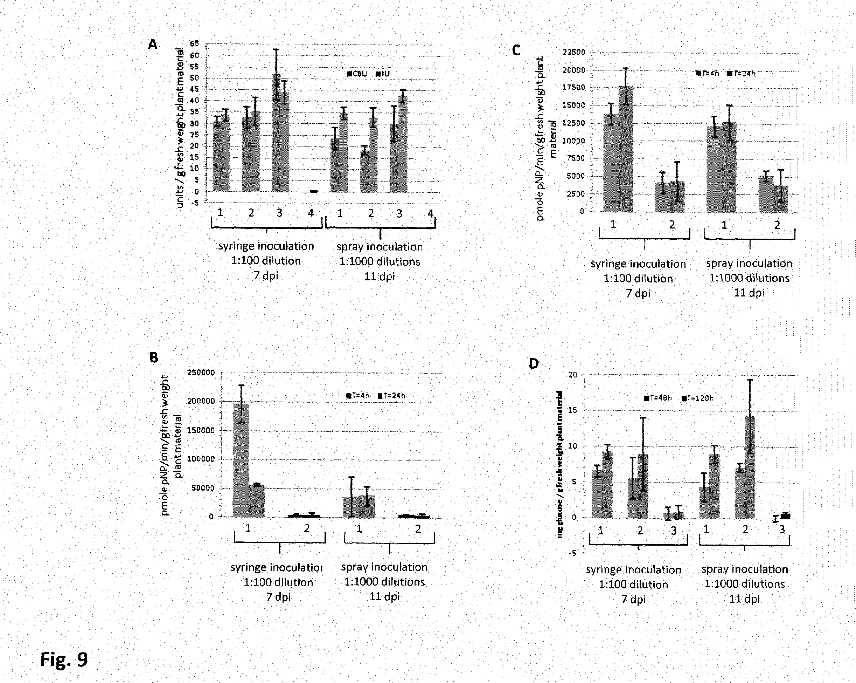

Fig. 9 shows results of a comparison of different inoculation methods for the highest cellulase expression of selected cellulase fusions: syringe infiltration vs. spraying with suspension of agrobacteria harboring assembled TMV constructs. Analysis of the enzymatic activity for the *Nicotiana benthamiana* leaves inoculated using a syringe (7 dpi, 1:100 dilution of agrofbacterial culture OD600=1.3) and by spraying (11 dpi, 1:1000 dilution of agrobacterialagrofbacterial culture OD600=1.3).
   (A) β-glucosidase BGL4 from *Humicola grisea* Bgl4. 1 - Bgl4 expressed in cytosol (pNMD1201); 2 - Bgl4 expressed in cytosol as a fusion with His(6)-tag followed by enterokinase cleavage site (His-EK) (pNMD1213); 3 - Bgl4 targeted to chloroplast (pNMD1192); 4 - uninfected leaf tissue.
   (B) endoglucanase E1 from *Acidothermus cellulolyticus.* 1 - E1 fusion with rice amylase presequence (pNMD1231); 2 - uninfected leaf tissue.
   (C) Exoglucanase 1 (CBHI) from *Trichoderma reesei.* 1 - CBHI fusion with barley α-amylase presequence (pNMD1181); 2 - uninfected leaf tissue.
   (D) Exocellulase E3 from *Thermobifida fusca.* 1 - E3 fusion with apple pectinase presequence (pNMD1161); 2 - E3 fusion with chloroplast targeting presequence followed by His(6)-tag and enterokinase cleavage site (His-EK) (pNMD1129); 3 - uninfected leaf tissue.

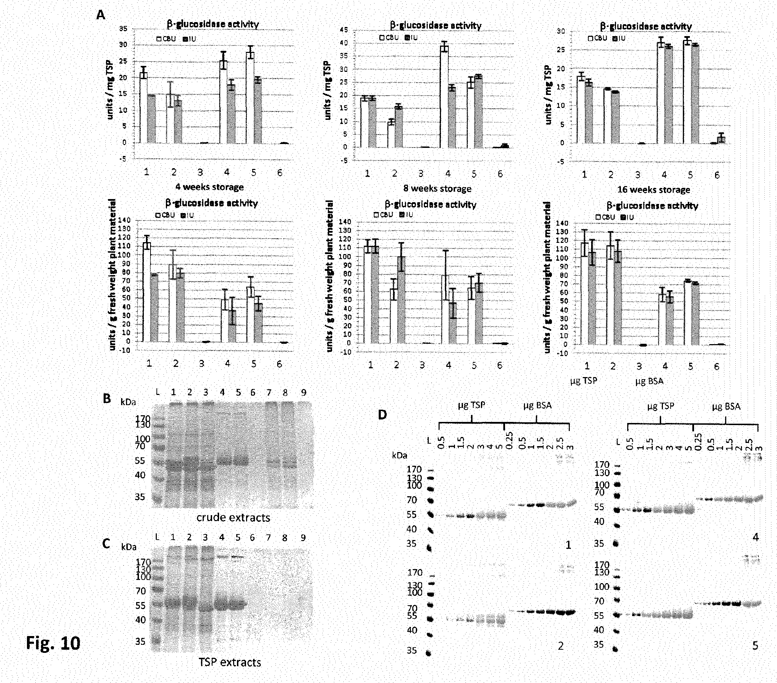

Fig. 10 shows results of an analysis of storage conditions for plant material expressing selected *Humicola grisea* β-glucosidase Bgl4cellulase fusions.
   (A) Enzymatic activity of TSP samples containing *Humicola grisea* β-glucosidase Bgl4 fusions extracted from plant material and stored for different periods of time at different conditions; analysis of crude protein extracts (B) and TSP extracts (C) by SDS-PAGE with Coomassie staining;
   (D) estimation of percentage of TSP of recombinant Bgl4 fusions (4 weeks storage time). L - PageRuler^{™} Prestained Protein Ladder (#SM0671, Fermentas);
      1 to 3 - plant material stored at -80°C;
      4 to 6 - plant material stored as a silage;
      7 to 9 - plant material stored as a dry mass;
      1, 4 and 7 - plant material expressing cytosolic Bgl4;
      2, 5 and 8 - plant material expressing chloroplast targeted Bgl4;
      3, 6 and 9 - non-infected plant material.

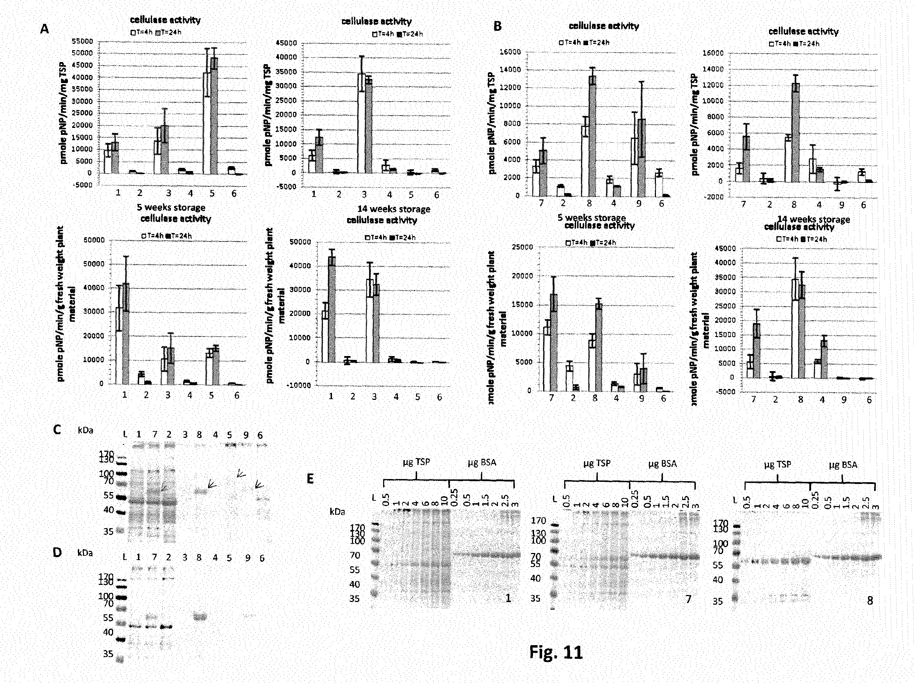

Fig. 11 shows the results of an analysis of storage conditions for plant material expressing selected *Acidothermus cellulolyticus* endoglucanase E1 and *Trichoderma reesei* exoglucanase CBHI fusions.
   (*A*) Enzymatic activity of TSP samples containing endoclucanase E1 fusions extracted from plant material and stored for different periods of time (left: 5 weeks; right: 14 weeks) at different conditions. 1, 3 and 5 - plant material expressing E1 from *Acidothermus cellulolyticus* targeted to the apoplast via fusion with rice amylase presequence (pNMD1231); 2, 4 and 6 - uninfected plant material;
   (*B*) Enzymatic activity of TSP samples containing containing *Trichoderma reesei* exoglucanase CBHI fusions extracted from plant material stored for different periods of time at different conditions. 7, 8 and 9 - plant material expressing apoplast targeted CBHI (fusion with barley α-amylase presequence, pNMD1181 construct); 2, 4 and 6 - non-infected plant material;
      Analysis of crude protein extracts (*C*) and TSP extracts (*D*) using SDS-PAGE;
      (*E*) estimation of percentage of TSP of recombinant E1 and CBHI fusions (4 weeks storage time);
      1, 2 and 7 - plant material stored at -80°C;
      3, 4 and 8 - plant material stored as silage;
      5, 6 and 9 - plant material stored as dry mass.

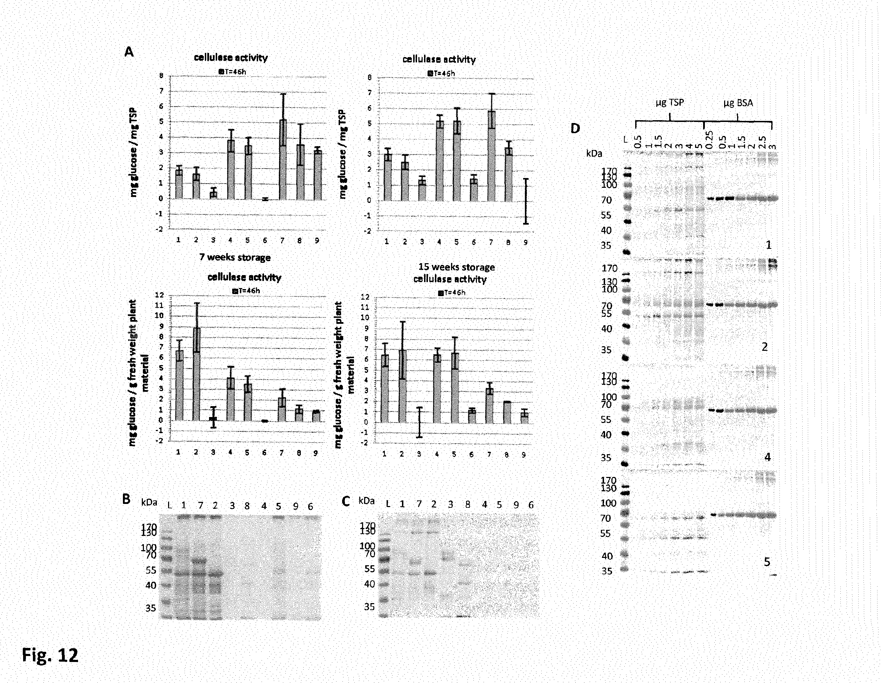

Fig. 12 shows the analysis of storage conditions for plant material expressing selected *Thermobifida fusca* exocellulase E3 fusions.
   (*A*) Enzymatic activity of TSP samples containing exoglucanase E3 fusions extracted from plant material stored for different periods of time at different conditions, 1, 4 and 7 - plant material expressing E3 targeted to apoplast with apple pectinase TP; 2, 5 and 8 - plant material expressing chloroplast TP-His-EK-E3 fusion; 3, 6 and 9 - uninfected plant material. Analysis of crude protein extracts (*B*) and TSP extracts (*C*) by SDS-PAGE with Coomassie staining (7 weeks storage time); (*E*) Estimation of percentage of TSP of recombinant E3 fusions (7 weeks storage time).
      1, 2 and 7 - plant material stored at -80°C;
      3, 4 and 8 - plant material stored as a silage;
      5, 6 and 9 - plant material stored as a dry mass.

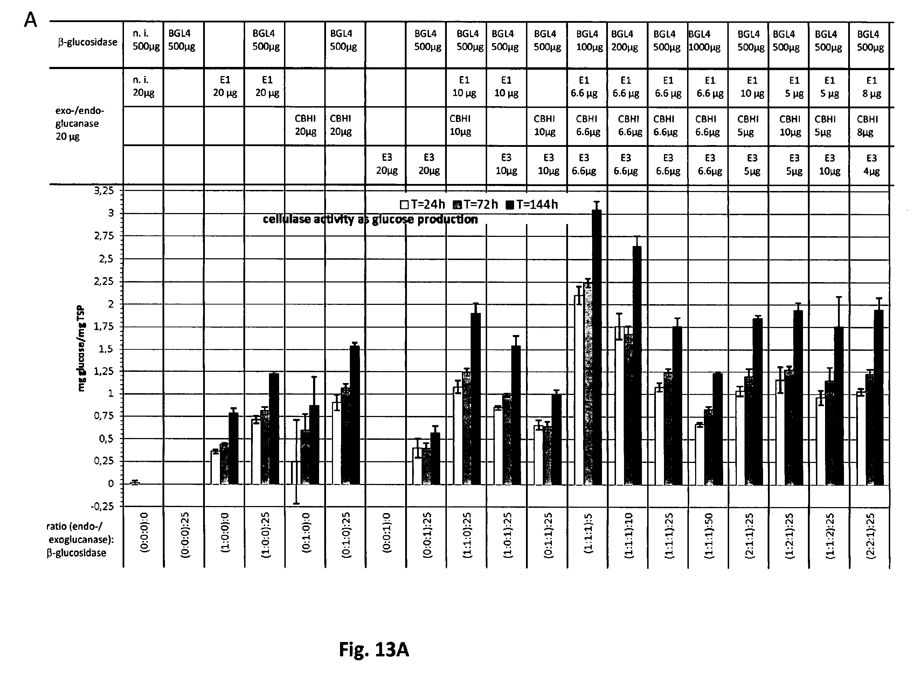

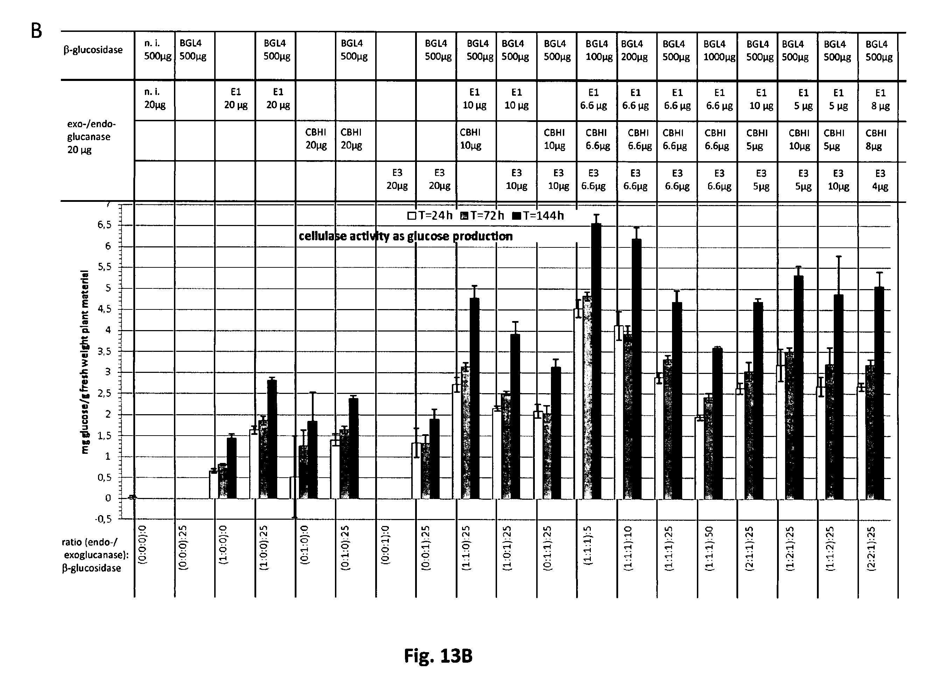

Fig. 13 shows results of screening for optimal ratios between plant-produced cellulases providing most efficient conversion of cellulose to glucose using a mixture of TSP samples containing recombinant β-glucosidase BGL4, endoglucanase E1, exoglucanase 1 (CBHI) and exocellulase E3. (*A*) Enzymatic activity is represented in mg glucose/mg TSP of enzyme-containing plant extracts. (*B*) Enzymatic activity is represented in mg glucose/g fresh weight of enzyme containing plant material. β-glucosidase BGL4 from *Humicola grisea* expressed in cytosol (pNMD1201), apoplast targeted endoglucanase E1 from *Acidothermus cellulolyticus* expressed as a fusion with rice amylase presequence (pNMD1231), apoplast targeted exoglucanase 1 (CBHI) from *Trichoderma reesei* expressed as a fusion with barley α-amylase presequence (pNMD1181) and chloroplast targeted exocellulase E3 from *Thermobifida fusca* (pNMD1129) were used for the screening.

### Detailed Description of the Invention

The process of producing a preparation of plant cell wall-degrading enzymes of the invention involves expression, in plants or parts thereof, the one or more than one plant cell wall-degrading enzyme. A part of a plant may be a plant part attached to a complete living plant such as leaves or may be detached from a complete plant, wherein the former possibility is preferred. It is generally desired to achieve high expression levels of these enzymes, since high expression levels can contribute to the stability of enzymatic activity of the expressed enzymes in the storing step. Methods of high level expression of proteins in plants have been established over the last two decades and are known to the plant biotechnologist. It is possible to use transgenic plants stably encoding the one or more than one plant cell wall-degrading enzyme in the nuclear or plastid genome. Inducible expression systems may be used to induce expression at a desired growth state of the plant. WO 2007137788 describes an inducible expression system using (viral) replicons expressed in transgenic plants. A method of controlling gene expression in plastids is disclosed in WO 2005/054481.

In one embodiment, transient expression is used, wherein plants or plant parts are transfected at a suitable point in time with a nucleic acid molecule (vector) comprising a nucleic acid construct containing a nucleic acid sequence encoding said one or more than one cell wall-degrading enzyme(s). This embodiment has the advantage that a decision on which enzyme to express can be delayed until shortly before the point of transfection, whereby the user can react on short notice to demands. The process of the invention therefore fits to modern market needs such as "trade on demand". The term "transient" means that no selection methods are used for selecting cells or plants transfected with the nucleic acid molecule (vector) or the nucleic acid construct in the background of non-transfected cells or plants using, e.g. selectable agents and selectable marker genes capable of detoxifying the selectable agents. As a result, the transfected nucleic acid is generally not stably introduced into plant chromosomal DNA. Instead, transient expression methods make use of the effect of transfection in the very plants transfected.

An established way of achieving high level expression is the use of self-replicating (viral) replicons containing a nucleotide sequence encoding said one or more cell wall-degrading enzyme(s). Plant viral expression systems have been described in many publications both for the use of DNA viral vectors and for the use of RNA viral vectors, such as in W02008028661, W02006003018, W02005071090, WO2005049839, WO2006012906, WO02101006 or WO02068664 and many more publications are cited in these documents.

Various methods for introducing a nucleic acid molecule into a plant or plant part for transient expression are known. Agrobacteria can be used for transfecting plants with a DNA molecule (vector) or construct of interest e.g. by agroinfiltration. A system and method for large scale infiltration of plants by agrobacteria is described in WO2009095183. In another embodiment, plants or plant parts are sprayed with an aqueous suspension containing cells of an *Agrobacterium* strain, which is well suitable for large scale applications to many plants such as to plants on a farm field. Such spray transfection processes are described in detail in international patent application PCT/EP2011/002279.

The *Agrobacterium* strain may belong to the species *Agrobacterium tumefaciens* or *Agrobacterium rhizogenes* that are commonly used for plant transformation and transfection and which are known to the skilled person from general knowledge. The *Agrobacterium* strain comprises a DNA molecule (Ti-plasmid) comprising a nucleic acid construct containing a DNA sequence of interest. The DNA sequence of interest encodes one or more than one enzyme to be expressed in plants. The nucleic acid construct is typically present in T-DNA of Ti-plasmids for introduction of the nucleic construct into plant cells by the secretory system of the *Agrobacterium* strain. On at least one side or on both sides, the nucleic acid construct is flanked by a T-DNA border sequence for allowing transfection of said plant(s) and introduction into cells of said plant of said nucleic acid construct. In the nucleic acid construct, the DNA sequence of interest is present such as to be expressible in plant cells. For this purpose, the DNA sequence of interest may be, in said nucleic acid construct, under the control of a promoter active in plant cells. Examples of the DNA sequence of interest are a DNA sequence encoding a DNA viral replicon or an RNA viral replicon or a gene to be expressed. The gene encodes the enzyme to be expressed in cells of the plant(s). Also the viral replicons encode the enzyme to be expressed in cells of the plant(s). The DNA construct may comprise, in addition to the DNA sequence of interest, other sequences such as regulatory sequences for expression of the DNA sequence of interest, such as a transcription promoter and terminator. Agrobacterium-mediated gene transfer and vectors therefor are known to the skilled person, e.g. from the references cited above or from text books on plant biotechnology such as Slater, Scott and Fowler, Plant Biotechnology, second edition, Oxford University Press, 2008.

In embodiments wherein strong expression of a protein is desired, the nucleic acid construct may encode a viral vector (referred to herein as "replicon") that can replicate in plant cells. In order to be replicating, the replicon contains an origin of replication that can be recognized by a nucleic acid polymerase present in plant cells, such as by the viral polymerase expressed from the replicon. In case of RNA viral vectors (referred to as "RNA replicons"), the replicons may be formed by transcription under the control of a plant promoter, from the DNA construct after the latter has been introduced into plant cell nuclei. In case of DNA replicons, the replicons may be formed by recombination between two recombination sites flanking the sequence encoding the viral relicon in the DNA construct, e.g. as described in WO00/17365 and WO 99/22003. If the replicon are encoded by the DNA construct, RNA replicons are preferred. Use of DNA and RNA viral vectors (DNA or RNA replicons) has been extensively described in the literature over the years. Some examples are the following patent publications: WO2008028661, W02007137788, WO 2006003018, WO2005071090, WO2005049839, WO02097080, WO02088369, W002068664. An example of DNA viral vectors are those based on geminiviruses. For the present invention, viral vectors or replicons based on plant RNA viruses, notably those based on plus-sense single-stranded RNA viruses are preferred. Accordingly, the viral replicon may be a plus-sense single-stranded RNA replicon. Examples of such viral vectors are tobacco mosaic virus (TMV) and potex virus X (PVX). Potexvirus-based viral vectors and expression systems are described in EP2061890. Many other plant viral replicons are described in the patent publications mentioned above.

In one embodiment, step (i) comprises spraying aerial parts of said plant with an aqueous suspension containing cells of an *Agrobacterium* strain comprising a DNA molecule comprising a nucleic acid construct encoding a DNA or, preferably, an RNA replicon encoding said one or more than one cell wall-degrading enzyme.

The aqueous suspension that may be used for spraying plants or plant parts in the process of the invention may have a concentration of *Agrobacterium* cells of at most 1.1·10⁹ cfu/ml, which corresponds approximately to an *Agrobacterium* culture in LB-medium of an optical density at 600 nm of 1. Due to the high transfection efficiency achievable, notably if spraying is done with the use of an abrasive and/or a surfactant or wetting agent (see below), much lower concentrations may, however, be used, which allows treatment of many plants such as entire farm fields without the need for huge fermenters for *Agrobacterium* production. Thus, the concentration may be at most 2.2·10⁷ cfu/ml, preferably at most 1.1·10⁷ cfu/ml, more preferably at most 4.4·10⁶ cfu/ml. In one embodiment, the concentration is at most 1.1·10⁶ cfu/ml of the suspension. For avoiding determination of cell concentrations in terms of cfu/ml, concentrations of agrobacterial suspensions are frequently assessed by measuring the apparent optical density at 600 nm using a spectrophotometer. Herein, the concentration of 1.1·10⁷ cfu/ml corresponds to a calculated optical density at 600 nm of 0.01, whereby the calculated optical density is defined by a 100-fold dilution with water or buffer of a suspension having an optical density of 1.0 at 600 nm. Similarly, the concentrations of 4.4·10⁶ cfu/ml and 1.1·10⁶ cfu/ml correspond to a calculated optical density at 600 nm of 0.004 and 0.001, respectively, whereby the calculated optical densities are defined by a 250-fold or 1000-fold, respectively, dilution with water or buffer of a suspension having an optical density of 1.0 at 600 nm.

The aqueous suspension containing cells of an *Agrobacterium* strain used for spraying the plants or parts of a plant may contain at least one abrasive suspended in said suspension for improving transfection efficiency. The abrasive that may be used is a particulate material that is essentially insoluble in the aqueous suspension of *Agrobacterium* cells. The abrasive is believed to weaken, notably if used together with a wetting agent, the surface of plant tissue such as leaves, and thereby facilitates penetration of *Agrobacterium* cells into the intercellular space of plant tissue. As a result, the transfection efficiency is high.

The particulate material to be used as the abrasive of the invention may be carrier material as commonly used as carriers in wettable powder (WP) of pesticide formulations. In the context of wettable powders, these carriers are also referred to in the field of pesticide formulations as "fillers" or "inert fillers". Wettable powder formulations are part of the general knowledge in the field of plant protection. Reference is made to the handbook PESTICIDE SPECIFICATIONS, "Manual for Development and Use of FAO and WHO Specifications for Pesticides", edited by the World Health Organisation (WHO) and the FOOD and Agriculture Organization of the United States, Rome, 2002, ISBN 92-5-104857-6. Wettable powder formulations for plant protection are for example described in EP 1810569, EP1488697, EP1908348 and EP0789510. The abrasive may be a mineral material, typically an inorganic material. Examples of such carrier materials are diatomaceous earth, talc, clay, calcium carbonate, bentonite, acid clay, attapulgite, zeolite, sericite, sepiolite or calcium silicate. It is also possible to use quartz powder such as the highly pure quartz powder described in WO02/087324. Preferred examples are silica, such as precipitated and fumed hydrophilic silica, and carborundum. The abrasive properties of diluents or fillers such as silica used in wettable powders are known (see "Pesticide Application Methods" by G.A. Matthews, third edition, Blackwell Science, 2000, on page 52 thereof).

As commercial products of particulate inorganic materials for use as abrasives, the hydrophilic silica Sipernat™ 22S and Sipernat™ 50 S, manufactured by Evonic Degussa may be mentioned. Other products are "Hi-Sil™ 257", a synthetic, amorphous, hydrated silica produced by PPG Industries Taiwan Ltd. or "Hubersorb 600™", a synthetic calcium silicate, manufactured by Huber Corporation. A commercial sub-micron sized silica is Hi-Sil™ 233 (PPG Industries) having an average particle size of around 0.02 µm.

The abrasive may have a median particle size between 0.01 and 40, preferably between 0.015 and 30, more preferably between 0.05 and 30, even more preferably between 0.1 and 30, even more preferably between 0.1 and 20, even more preferably between 0.5 and 20, and most preferably between 1.0 and 16 µm. In one embodiment, the median particle size is between 0.015 and 1 or between 0.02 and 0.5 µm. The median particle size is the volume median particle size that can be measured by laser diffraction using a Mastersizer™ from Malvern Instruments, Ltd. In order to avoid clogging of spraying nozzles, the maximum particle size of the largest particles contained in the abrasive should be at most 45 µm, preferably at most 40 µm, which may be determined by sieving. This condition is considered fulfilled, if the sieve residue is below 1.5 % by weight (following ISO 3262-19). The abrasive may have a D90 value of at most 40 µm, preferably of at most 30 µm, measured by laser diffraction as described above. Typically, the particle sizes above relate to primary particle sizes. The content of the abrasive in the aqueous suspension of the invention may be between 0.01 and 3, preferably between 0.02 and 2, more preferably between 0.05 and 1 and even more preferably between 0.1 and 0.5 % by weight of said suspension.

The aqueous suspension usable for spraying parts of the plants preferably contains an agricultural spray adjuvant. The spray adjuvant may be a surfactant or wetting agent. The surfactant or wetting agent has multiple advantages in the present invention. It reduces the surface tension of the water of the aqueous suspension and makes the waxy surface of plant leaves more permeable for agrobacteria. It further improves the stability of the suspension and reduces settling of the abrasive in the suspension. Surfactants used in the present invention are not particularly limited. However, nonionic surfactants are preferred. A measurement frequently used to describe surfactants is the HLB (hydrophilic/lipophilic balance). The HLB describes the ability of the surfactant to associate with hydrophilic and lipophilic compounds. Surfactants with a high HLB balance associate better with water soluble compounds than with oil soluble compounds. Herein, the HLB value should be 12 or greater, preferably at least 13. As noninionic surfactants, organo-silicone surfactants such as polyalkyleneoxide-modified heptamethyltrisiloxane are most preferred in the present invention. A commercial product is Silwet L77™ spray adjuvant from GE Advanced Materials.

The aqueous suspension of agrobacteria may be produced as follows. In one method, the *Agrobacterium* strain to be used in the process of the invention is inoculated into culture medium and grown to a high cell concentration. Larger cultures may be inoculated with small volumes of a highly concentrated culture medium for obtaining large amounts of the culture medium. Agrobacteria are generally grown up to a cell concentration corresponding to an OD at 600 nm of at least 1, typically of about 1.5. Such highly concentrated agrobacterial suspensions are then diluted to achieve the desired cell concentration. For diluting the highly concentrated agrobacterial suspensions, water is used. The water may contain a buffer. The water may further contain the surfactant mentioned above. Alternatively, the concentrated agrobacterial suspensions may be diluted with water, and any additives such as the surfactant and the optional buffer substances are added after or during the dilution process. The abrasive may be added before, during or after dilution. It is however preferred to agitate the suspension during addition of the abrasive to uniformly disperse the abrasive in the agrobacterial suspension. The step of diluting the concentrated agrobacterial suspension may be carried out in the spray tank of the sprayer used for spraying the diluted suspensions.

The sprayer to be used for transfecting plants or plant parts in step (i) of the process of the invention mainly depends on the number of plants or the area to be sprayed. For one or a small number of plants to be sprayed, pump sprayers as widely used in household and gardening can be used. These may have volumes of the spray tank of between 0.5 and 2 liters. For applications on a medium scale, manually operated hydraulic sprayers such as lever-operated knapsack sprayers or manually operated compression sprayers may be used. However, the high transfection efficiency achieved in the invention has its full potential in the transfection of many plants such as plants growing on a farm field or in a greenhouse. For this purpose, power-operated hydraulic sprayers such as tractor-mounted hydraulic sprayers equipped with spray booms can be used. Aerial application techniques using helicopters or airplanes are also possible for large fields. All these types of sprayers are known in the art and are described for example in the book "Pesticide Application Methods" by G.A. Matthews, third edition, Blackwell Science, 2000. In order to ensure a homogeneous suspension in the spray tanks of the sprayers, small or medium size sprayers may be shaken at regular intervals or continuously during spraying. Large sprayers such as the tractor-mounted sprayers should be equipped with an agitator in the spray tank.

Considering the presence of agrobacterial cells and optionally abrasive in the suspensions to be sprayed, sprayers used in the invention should produce spray of a droplet size at least of fine spray. Also, medium spray or coarse spray in the classification of sprays used in the above-mentioned book by G.A. Matthews, page 74, may be used. The main purpose of the spraying in the invention is wetting of plant tissue with the suspension. Thus, the exact droplet size is not critical. However, the transfection efficiency may be further improved by providing the spray to plant surfaces with increased pressure.

In the process of producing the preparation of the invention, the one or more than one cell wall degrading enzyme may be expressed in multi-cellular plants, notably, higher plants. Both monocot and dicot plants can be used. Common crop plants for the use in present invention include alfalfa, barley, beans, canola, cowpeas, cotton, corn, clover, lotus, lentils, lupine, millet, oats, peas, peanuts, rice, rye, sweet clover, sunflower, sweetpea, soybean, sorghum triticale, yam beans, velvet beans, vetch, wheat, wisteria, and nut plants. The plant species preferred for practicing this invention include, but not restricted to, representatives of Gramineae, Compositeae, Solanaceae and Rosaceae. Preferred plants are plants that do not normally enter the food chain such as *Nicotiana* species such as *N. tabacum* and *N. benthamiana* may be used.

It is possible to express the one or more than one cell wall-degrading enzyme in the cytosol of said plants or plant parts. In this case, no signal peptide directing the enzyme into a particular compartment is added to the enzyme. A natural signal peptide present in the enzyme for secreting the enzyme from the native organism from which the enzyme originates may be omitted for expression according to the present invention. However, since signal peptides of secretory proteins from bacteria are generally not functional or of low reliability in plants, it is not necessary to omit the natural signal peptide when enzymes from bacteria or achaea are expressed in plants according to this invention.

In one embodiment, the enzyme expressed in the plants or parts thereof is targeted to a desired cell compartment. Preferred compartments are plastids or the apoplast. Targeting to these compartments has the additional advantage that the expressed and targeted cell wall-degrading enzyme(s) is (are) removed from the cytoplasm where most proteases reside, which can contributes to stability of the enzymes upon storage. Targeting of an enzyme expressed in a plant to a cell compartment can be achieved by fusing a suitable signal peptide to the enzyme to form a fusion protein comprising the signal peptide and the enzyme. Targeting of protein expressed in plants to the apoplast is known e.g. from WO03020938, and may be achieved by fusing a signal peptide to the enzyme, wherein the signal peptide targets the enzyme into the endoplasmatic reticulum (ER) and the secretory pathway. The polypeptide capable of binding the fusion protein to a cell wall component used in WO03020938 may be omitted from the fusion protein of this reference. All signal peptides of proteins known to be secreted or targeted by plants to the apoplast may be used for targeting the enzyme to the apoplast. Preferred examples are the signal peptides of tobacco calreticulin or of rice α-amylase. Further examples are the signal peptides of pectin methylesterase or of *N. tabacco* tyrosine and lysine rich protein (NtTLPR). A further example is the signal peptide of apoplastic isoperoxidase from zucchini (APRX) (Carpin et al., 2001, The Plant Cell, 13, 511-520). The signal peptide has to be comprised in the fusion protein such that it is functional for said targeting. This condition may be fulfilled at any location in the fusion protein. In general, however, the signal peptide is positioned at the N-terminus of the enzyme for efficient targeting of the enzyme to the apoplast.

Targeting of plant-expressed proteins to plastids by fusing a plastid transit peptide to the protein or enzyme is also known from many publications such as WO2004101797 and references cited therein. Details of the cleavage sites of natural plastid-targeted plant proteins have been investigated (Gavel & Von Heijne 1990, FEBS Lett., 261, 455-458). Various chloroplast transit peptide (TP) are used in the examples of this document.

The cell wall-degrading enzymes expressed and used in the present invention are generally heterologous enzymes to the plant or plant part in which they are expressed, i.e. the plant or its part does not produce the enzyme naturally. In many cases, the enzymes may originate from bacteria, achaea or from fungi. It is possible to optimize codon usage of genes encoding these enzymes for expression in plants, notably in the plant employed for expressing them. Codon optimization is a standard method for increasing expression yields. Codon-optimized genes and nucleic acids can be ordered from commercial sources such as Entelechon GmbH, Regensburg, Germany.

Herein, a cell wall-degrading enzyme is an enzyme that can degrade at least one polymeric component of plant cell walls or an oligomeric degradation product of such polymeric component. Plant cell walls contain cellulose, hemicellulose, β-1,4-glycan, pectin and lignin, all of which are polymers. Except for lignin, these polymers are polysaccharides. Enzymes capable of degrading plant cell wall polymers or oligomeric degradation products thereof are produced by several organisms that can use these polymeric compounds as carbon source or that grow on decaying plants. Generally, these are microorganisms belonging to bacteria, archaea and fungi, but also some protozoa and termites produce cellulases. Ruminants rely on cellulase-expressing microorganisms to break down cellulose. Since plant cell wall biopolymers are polymeric, they cannot be degraded within cells of these organisms. Accordingly, cell wall-degrading enzymes are generally secreted into the environment and degrade plant cell wall components, and/or oligomeric degradation products of plant cell wall components, in the extracellular medium. Small molecular degradation products, mostly di- or monosaccharides such as D-glucose, may be taken up by the organisms and used as nutrients.

Herein, the terms "oligomer" or "oligomeric degradation product" refer to compounds made up of at least 2 monomer moieties. Thus, as far as saccharidic oligomers or oligomeric degradation product of polysaccharides are concerned, disaccharides are oligosaccharides. As an example, cellobiose is an oligosaccharide.

In contrast to many intracellular proteins expression and degradation of which is tightly regulated by organisms, secretory cell wall degrading enzymes are optimized by evolution to be rather stable enzymes that can relatively well withstand a variety of environmental conditions in the extracellular medium in order to be effective for extracellular plant cell wall degradation. The inventors assume that these circumstances are the reason for the surprisingly high stability of the cell wall degrading enzymes upon storage in silage, as found in the present invention.

Regarding cell wall degrading enzymes, the invention is not limited. Generally, the one or more than one cell wall degrading enzyme is heterologous to the plant or part in which it is expressed in the invention. The enzymes are generally of microbial origin, notably of bacterial, archaebacterial or fungal origin. Modifications may be made to such enzymes using general methods of genetic engineering. Enzymes involved in degradation of any of the main plant cell wall components may be used in the invention. Thus, cellulolytic enzymes, hemicellulases, pectinases and/or lignin degrading enzymes may be used. In an important embodiment, cellulolytic enzymes, i.e. enzymes that cleave β-14-glycosidic bonds between glucose moieties in compounds containing β-1,4-glycosidically linked glucose moieties are used. Cellulolytic enzymes are also referred to herein as "cellulases". Cellulose consists of D-glucose units that are linked by β-1,4-glycosidic bonds, and cellulolytic enzymes (cellulases) are preferred for use in the invention. Several enzymes are involved in the enzymatic breakdown of cellulose, and cellulolytic enzymes comprises multiple groups including:
- endoglucanases (β-1,4-glucanglucanohydrolases, EC3.2.1.4) that cause randomly cleave internal (away from chain ends) β-1,4-glycosidic bonds of cellulose;
- exoclucanases or cellobiohydrolases (EC3.2.1.91) that produce cellobiose by attacking cellulose from reducing chain ends (cellobiohydrolase I) or by attacking cellulose from the non-reducing ends of cellulose chains (cellobiohydrolase II); and
- β-glucosidases (EC 3.2.1.21) that convert cellobiose to glucose.

There is an enormous body of literature on all aspects of cellulolytic enzymes from many different sources, and the prospect of deriving fuel from cellulose has led to a boost of research in this field over the last decade. The invention is not limited with regard to the cellulytic enzyme to be used. In an interesting embodiment, enzymes derived from extremophilic organisms such as thermophiles or acidophiles are used, since these are of particularly high stability. Table 1 shows enzymes from each of the above listed cellulase groups that were used in the examples.

**Table 1. Cellulases used for transient expression**

| **N r** | **Name** | **EC number** | **Source organism** | **Accession number** | **Molecular weight, kDa** | **pl** | **pH optimum** | **Temp. optimum, °C** |
|---|---|---|---|---|---|---|---|---|
| 1 | Endoglucanase E1 | 3.2.1.4 | *Acidothermus cellulolyticus* | Swiss-Prot: P54583.1 | 60,7 | 6.59 | 5-6 | 81 |
| 2 | Exoglucanase 1 (CBH I) | 3.2.1.91 | *Trichoderma reesei*/ *Hypocrea jecorina* | Swiss-Prot: P62694.1 | 54 | 4.65 | 5 | 45-50 |
| 3 | Exocellulase E3 | 3.2.1.91 | *Thermobifida fusca* | GenBank: AAA62211 .1 | 63,5 | 4,46 | 7-8 | 65 |
| 4 | β-glucosidase BGL4 | 3.2.1.21 | *Humicola grisea* | GenBank: BAA74958 .1 | 54 | 5.26 | 6 | 60 |

Since enzymes from several groups are required to degrade cellulose to glucose, it is preferred to provide at least one enzyme from each of the above groups when plant cell wall-containing plant material is to be degraded. This may be achieved by expressing two or more such enzymes in one plant, or by expressing one enzyme per plant. In the latter case, two or more plants having expressed different cellulolytic enzymes can be mixed in a desired ratio before the storing step (iii) and stored together in step (iii). Alternatively, two or more silages each obtained from a plant having expressed an enzyme can be combined when performing a process of degrading plant cell wall components of plant materials. In one embodiment, a silage containing an endoglucanase, a cellobiohydrolase I and a cellobiohydrolase II is used. In another embodiment, a silage containing an endoglucanase, a cellobiohydrolase I, a cellobiohydrolase II, and a β-glucosidase is used.

Hemicelluloses are polysaccharidic heteropolymers such as arabinoxylans, present along with cellulose in almost all plant cell walls. Hemicellulose has a random, amorphous structure with little strength. It is easily hydrolyzed by dilute acid or base as well as many hemicellulase enzymes. Hemicellulose contains several different sugar monomers. For instance, besides glucose, sugar monomers in hemicellulose can include xylose, mannose, galactose, rhamnose, and arabinose. Hemicelluloses contain most of the D-pentose sugars. Xylose is generally the sugar monomer present in the largest amount, but mannuronic acid and galacturonic acid also tend to be present. Unlike cellulose, hemicellulose consists of shorter chains, 500-3,000 sugar units as opposed to 7,000 - 15,000 glucose molecules per polymer seen in cellulose. In addition, hemicellulose is a branched polymer, while cellulose is unbranched. Microbial xylanases usable in the present invention are reviewed by Beg et al. in Applied Microbiology and Biotechnology, Vol. 56, No. 3-4, 326-338. Hemicellulose conversion is described for example by Saba in J. Ind. Microbiol. Biotechnol. (2003) 30: 279-291.

Pectin is another major cell wall polymer found in all land plants (Willats et al., 2001, Plant Mol Biol, 47, 9-27). Pectin is a complex polymeric network of polysaccharides like homogalacturonan and rhamnogalacturonan. The main monomeric component of this network is galacturonic acid and not glucose like in the case of cellulose. Enzymes for degrading pectin are known in the art. An example is polygalacturonase, the most extensively studied among the family of pectinolytic enzymes. Polygalacturonases (PGases) are pectinolytic enzymes that catalyze the hydrolytic cleavage of the polygalacturonic acid chain with the introduction of water across the oxygen bridge. The PGases involved in the hydrolysis of pectic substances are endo-PGase (E.C. 3.2.1.15) and exo-PGase (E.C. 3.2.1.67). Endo-PGases are widely distributed among fungi, bacteria and many yeasts. They are also found in higher plants and some plant parasitic nematodes. They have been reported from many microorganisms, including *Aureobasidium pullulans, Rhizoctonia solani* Kuhn, *Fusarium moniliforme, Neurospora crassa, Rhizopus stolonifer, Aspergillus* sp., Thermomyces lanuginosus, Peacilomyces clavisporus. Endo-PGases have also been cloned and genetically studied in a large number of microbial species. In contrast, exo-PGases occur less frequently. They have been reported in *Erwinia carotovora, Agrobacterium tumefaciens, Bacteroides thetaiotamicron, Alternaria mali Fusarium oxysporum, Ralstonia solanacearum, Bacillus* sp. For review see Jayani et al., 2005, Process Biochemistry, 40, 2931-2944.

Lignin degrading secretory enzymes such as lignin peroxidase are expressed by white and brown rot fungi, such as *Phanerochaete chrysosporium,* and degrade the polymeric lignin network by radical reactions catalyzed by peroxidases.

As indicated above with regard to cellulolytic enzymes, cell wall degrading enzymes from thermophilic microorganisms or other thermostable enzymes can be advantageous with regard to stability in the storing step of the invention as well as in the subsequent use for degrading plant material. This also applies to cell wall degrading enzymes other than cellulolytic enzymes. According to Vieille, Biotechnol Annu Rev. 1996;2:1-83, enzymes synthesized by thermophiles (organisms with optimal growth temperatures > 60°C) and hyperthermophiles (optimal growth temperatures > 80°C) are typically thermostable (resistant to irreversible inactivation at high temperatures) and thermophilic (optimally active at high temperatures, i.e., > 60°C). Such thermostable enzymes are referred to as "thermozymes". Some thermophiles can grow at 90°C or above and some have maxima above 100°C. Procaryotes that have growth optima between 80°C and about 113°C are called hyperthermophiles. They usually do not grow well below 55°C (Microbiology, 5th edition, Lansing M Prescott, Donald A Klein, John P Harley). Many important biotechnological processes utilize thermophilic enzymes because of their ability to withstand intense heat (Madigan MT, Martino JM (2006). Brock Biology of Microorganisms (11th ed.). Pearson. pp. 136. ISBN 0-13-196893-9).

The one or more than one enzyme used in the invention may be thermostable and/or thermostable. The enzymes used in the invention may have a temperature optimum of activity at >60°C. The temperature optimum of an enyzme can be determined by conducting activity assays at optimum pH at several temperatures to determine the temperature optimum, for example as described by Takashima et al., J. Biotechnol. 1996; 50(2-3):137-47.

Thermostable cellulases and hemicellulases are reviewed by Haki and Rakshit, Bioresource Technology, Vol. 89, Issue 1, 2003, pages 17-34. These enzymes, notably those listed in Tables 5 and 6 may be used in the present invention. Turner et al., Microbial Cell Factories 2007, 6:9 reviews thermophiles and thermostable enzymes, inter alia for biodegradation and modification of lignocellulose and for biofuel production. The enzymes mentioned in sections "Cellulose conversion by cellulases", "Hemicellulose conversions" and "Conversion of pectins" of Turner et al. may be used in the present invention. Taylor and Vaisman, BMC Structural Biology 2010, 10(Suppl 1):S5 describe discrimination of thermophilic and mesophilic proteins. Li et al., review structural features of thermoenzymes in Biotechnology Advances, 23 (2005) issue 4, 271-333. ΔG_{stab} of thermophilic proteins is 5 to 20 kcal/mol higher than that of mesophilic proteins.

When the plants or part thereof have expressed sufficient amounts of cell wall degrading enzyme in step (i) of the process of producing a preparation of cell wall degrading enzymes, they are harvested. Harvesting of said plants or plant parts can be done by conventional means as known in the art for the plants employed. Harvesting may comprise elimination of undesired plant parts. For example, it may be desired to remove leaf material from potato tubers or beetles. With other plants, roots may be removed. Harvesting may also comprise shredding, hackling, chipping or chopping of desired plants or plant parts, as is generally done with maize to be ensiled. Then, the harvest is ensiled in the storing step of the process.

Ensiling of harvests is widely used by farmers for the preservation of forages as an animal feed supply for periods where fresh forage is limited or unavailable. Thus, ensiling is a well established technique that can be performed according to known procedures for the plants employed in the process of the invention. The ensiling process comprises an anaerobic microbial fermentation of water-soluble sugars to lactic acid, lowering the pH to a point that inhibits further microbial fermentation. The goal is a rapid pH drop to minimize fermentation losses of feedstuff nutrients, especially protein. Literature on ensiling plant harvests is abundant and knowledge on conditions such as suitable moisture content of the material to be ensiled is known and can be tested experimentally, see e.g.: "Preparation, Maintenance and Utilisation of Maize Silage: A Review" by Castenada, C., Birch, C. J. and Dryden, G. (2003); in: Birch, C. J. and Wilson, S. R. Versatile Maize - Golden Opportunities: 5th Australian Maize Conference Proceedings, City Golf Club, Toowoomba, (67-75). 18 - 20 February, 2003; and "Troubleshooting Silage Problems: How to identify potential problems" by Robert J. Van Saun and A. Jud Heinrichs, Department of Dairy and Animal Science; the Pennsylvania State University, http://www.das.psu.edu/research-extension/dairy/nutrition/pdf/ troubleshootsilage08125.pdf). A selection of literature references on ensiling pant material is as follows: US 5,053,233 discloses inter alia use of additives. Other publications on ensiling plants and use of additives are: Adesogan et al., J. Dairy Sci. 86 (2003) 1789-1796; Conaghan et al., J. Dairy Sci. 93 (2010) 628-643; Kung et al., J. Dairy Sci. 87 (2004) 1310-1316; Woolford, M. K. (1984), Grass and Forage Science 39: 53-57; M.E. McCullough, Journal of Dairy Science, Vol. 52, Issue 10, October 1969, 1605-1608. Further, reference is made to the manual "Successful silage", eds.: Alan G. Kaiser, John W. Piltz, Helen M. Burns and Neil W. Griffiths, Top Fodder, published by Dairy Australia and New South Wales Department of Primary Industries, Australia, 2004. The manual is available online at: www.dpi.nsw.gov.au/agriculture/field/pastures-and-rangelands/silage/successful-silage.

Lactic acid fermentation generally starts automatically by lactic acid bacteria present in the environment or on the plant that is to be stored. It is, however, also possible to inoculate the harvest to be ensiled by additional lactic acid-producing organisms. Commercial products may be used for this purpose, such as Silo-King, Agri-King Inc., Fulton, USA containing *Lactobacillus plantarum, Pediococcus pentosaceus* and *Enterococcus faecium.*

Alternatively or additionally to achieving the pH drop by lactic acid-producing organisms, acids, preferably organic acids such as acetic acid, propionic acid or butyric acid may be added to the harvest. In this way, a rapid pH drop can be achieved.

The harvest, optionally after having dried the harvested plant material to a suitable moisture content, is put into a container or silo, and optionally compacted as required to avoid excessive air in the container or silo. As mentioned above, lactic acid-producing organisms may be added to the silage. Further or alternatively, it is possible to add silage additives to the silage material. Silage additives are known. Examples of silage additives are nitrites, hexamine, sodium benzoate, sodium propionate, formic acid, propionic acid and ammonia, and mixtures thereof. Mixtures of sodium benzoate and sodium propionate or mixtures of sodium benzoate and propionic acid are conventional silage additives. Detailed guidance on the use of silage additives is provided in chapter 7 of the manual "Successful silage" cited above.

The container or silo is then covered or closed, so as to allow anaerobic conditions to develop. Containers/silos can be solid silos as conventionally used in agriculture. Alternatively, flexible plastic bags that may be made of polyethylene can be used for ensiling the harvest. Excessive air may be removed from plastic bags before sealing using a vacuum pump to reduce oxygen content.

Upon storage, the silage undergoes various phases, including an aerobic phase, a fermentation phase and a stable phase. In the aerobic phase, aerobic microorganisms consume the remaining oxygen, whereby anaerobic conditions are reached. In the fermentation phase, growth of anaerobic bacteria produce lactic acid that lowers the pH. Low pH reduces the activity of proteolytic enzymes and stops the growth of some anaerobic bacteria. The fermentation phase can last from 7 to 30 days. This phase ceases when fermentation of sugars by lactic acid bacteria ceases either due to low pH that stops bacterial growth or depletion of low molecular sugar. Possibly after a secondary fermentation phase due to growth of clostridial spores, a stable phase is reached that is characterized by little biological activity. In the stable phase, the silage can be stored with little further change for extended periods of time of, for example, more than 100 days, preferably more than 200 days and even more than 300 days. Although there is no strict upper limit, 365 days may be mentioned as such upper limit for storage of the silage. The high flexibility of the storage time in the silage is ideal for storing the silage until the silage is used for degrading plant cell wall components of plant material. The minimum storage time in step (iii) or in the process of storing plant cell wall-degrading enzymes is 10 days, preferably 14 days, more preferably, 21 days. The silage can be stored at ambient temperature without external heating or cooling. The silage internally undergoes temperature changes in the course of the silage phases mentioned above.

Storage conditions in silage can be tested on a small laboratory scale before performing a process on a large industrial scale. A model system for laboratory-scale silage fermentation is described by Johnson et al., J. Appl. Microbiol. 98 (2005) 106-113. This model system can be employed for testing silage conditions such as packing density, moisture content of the material to be ensiled etc. for plant harvests for which there may be less experience from conventional ensiling of animal feed. As described by Johnson *et al*., silage conditions determined on a small scale can be used for determining silage conditions on larger scales.

In the process of storing plant cell wall-degrading enzymes of the invention, harvested plants or plant parts containing expressed one or more than one plant cell wall-degrading enzymes are stored in a silage as described above for step (iii) of the production process of the invention. Different plant or plant parts containing different expressed enzymes may be ensiled together to produce a silage containing two or more cell wall-degrading enzymes such as all enzymes required for degrading cellulose as described above.

The product obtained or obtainable in step (iii) of the production process or in the storing process of the invention is the silage of the invention. The silage of the invention contains one or more than one active plant cell wall-degrading enzymes. Activity of the enzyme can be determined according to generally known methods for the enzyme in question. Such activity tests for several enzymes are described in the examples. Generally, these methods require homogenization and/or extraction step to produce total soluble protein (TSP) from the silage. It is also possible to perform further purification steps before determination of activity of an enzyme in question.

The silage of the invention contains active enzymes capable of degrading plant cell wall components. Thus, the silage can be used in a process of degrading plant cell wall components of plant material. The main application of the silage of the invention is in a process of producing bioalcohol, notably bioethanol, from lignocellulosic material. There is abundant literature on processes for degrading plant cell wall components of various plant lignocellulosic materials to monosaccharides, a process also referred to as saccharification. Saccharification may be followed by fermentation, the process wherein monosaccharides and optionally small oligosccharides obtained in saccharification are fermented to alcohol. Yeasts such as *Saccharomyces cerevisiae* are typically used for alcohol fermentation. In one embodiment, the steps of saccharification and fermentation are combined in a simultaneous process known as "SSF" that stands for "simultaneous saccharification and fermentation". A review on SSF is found in Biotechnology for Biofuels 2008, 1:7.

Plant material to be treated in the degradation and alcohol production processes of the invention may be any plant agricultural waste material such as straw. Plant material rich in cellulose is preferred. Mostly, however, these processes are used for treating plant material from plants specifically grown for producing bioalcohol. There are no strict limits for the origin of the plant material to be employed. The plant material may be from annual, biannual or perennial plants. Both monocot and dicot plants may be used, whereby cellulose-rich plants is preferred. Examples of annual plants are cerial plants such as barley, wheat, oat. A preferred plant is maize. Examples of perennial plants are woody plants, notably trees. Plant material from different plant sources may be mixed and treated as a mixture. The plant material is preferably milled to comminuted before subjecting to the processes of the invention to increase the surface area and ease attack by the degrading enzymes.

In the process for degrading cell wall components or in the process for producing the bioalcohol of the invention, the step of treatment of plant material with the silage of the invention may be preceded by a pretreatment step for changing the physical and/or chemical structure of the cell walls of the plant material. Such pretreatment steps are known in the art. Most process concepts for bioethanol production from lignocellulose start with a thermo-chemical hydrolysis of the hemicellulose part (pretreatment), followed by an enzymatic hydrolysis of the cellulose part. According to Biotechnology for Biofuels 2008, 1:7, the purpose of the pretreatment is to alter the lignocellulosic structure and increase the rate of enzymatic hydrolysis of primarily the cellulose. This should be done with a minimum formation of compounds which inhibit the fermenting microorganisms. The accessible surface area is regarded as one of the most important factors affecting the effectiveness of enzymatic cellulose degradation. In native wood only a small fraction of the cell wall capillaries are accessible to the enzymes. Pretreatment, however, increases the available area in several ways: i) fragments and cracks are formed yielding increased area, ii) the hemicellulose fraction is hydrolysed which diminishes shielding effects, iii) the lignin also undergoes structural changes and the wood is delignified to various degrees, depending on the pretreatment technology. Thus, the shielding of microfibrils and occluding of pores, caused by lignin, can be removed. Other factors, believed to influence the digestibility in SSF, are the substrate crystallinity and the degree of polymerization.

The pretreatment methods can be divided into physical and chemical methods, and combinations of these two are commonly used (see e.g. the review written by Mosier et al. Bioresour Technol 2005, 96(6):673-686). The type of feedstock affects the choice of pretreatment method. The hemicellulose is, for instance, acetylated to a high degree in xylan-rich materials. Since acetate is liberated during hydrolysis, the pretreatment of these materials is to some extent autocatalytic and require less added acid and milder process conditions. However, the liberated acetate adds to the toxicity of the hemicellulose hydrolyzates. See: Biotechnology for Biofuels 2008, 1:7. Ammonia fiber/freeze explosion (AFEX) pretreatment is an attractive method for pretreatment of agricultural residues, yielding highly digestible cellulose. AFEX depolymerizes the lignin, removes the hemicellulose and decrystallizes the cellulose. The moderate temperature and pH also minimize formation of sugar degradation products. Another method of pretreatment is the lime method, based on calcium (or sodium) hydroxide. Alkali pretreatments are run at lower temperatures for long residence times, and as for the AFEX method, a delignification of the biomass is obtained.

After the optional pretreatment, the optionally pretreated plant material is subjected to treatment with the silage of the invention for saccharification. This step is done in a aqueous reaction medium. Temperature and pH of the reaction medium are set such the enzyme(s) contained in the reaction medium have sufficient activity. Silage containing at least one cell wall degrading enzyme is added. Other enzymes required for cell wall degradation may be added to the reaction medium from other sources. Preferably, silage containing multiple different enzymes involved in plant cell wall or cellulose degradation is added. The silage is added such that cell wall degrading enzymes contained therein easily enter the reaction medium. Since storage in silage partially destroys the structure of the plants or parts thereof containing the enzymes, the silage may be added to the reaction medium as it is, notably if the enzymes are targeted to the apoplast. Alternatively, the silage may be milled or homogenized before addition to the reaction medium in order to promote entry of the enzymes into the reaction medium. In the reaction medium, the cell wall material from the ensiled plant material is also degraded by the enzymes. Thus, the plant material originating from the plants having expressed the enzymes is also used for monosaccharide and bioalcohol production, which contributes to the excellent efficiency and cost-effectiveness of the present invention.

In the fermentation step (b) of the bioalcohol production process, monosaccharides produced in the saccharification step are converted to bioalcohol such as bioethanol. As mentioned above, saccharification and fermentation can be done simultaneously (see e.g. Biotechnology for Biofuels 2008, 1:7). For fermenting monosaccharides to alcohol, known microorganism as known from starch-based bioethanol production can be employed. Yeasts such as *Saccharomyces cerevisiae* may be used, and many suitable strains thereof for alcohol production are known. Conditions for alcohol fermentation are also known. Produced bioalcohol may then be isolated and purified, e.g. using distillation.

### EXAMPLES

The invention is further described using examples that do not limit the scope of protection.

### Reference Example 1: Determination of Agrobacterium cell concentration in liquid culture in terms of colony forming units (cfu)

The concentration of *Agrobacterium* cells in liquid suspension in terms of colony forming units per ml (cfu/ml) of liquid suspensions can be determined using the following protocol. Cells of *Agrobacterium tumefaciens* strain ICF 320 transformed with construct pNMD620 were grown in 7.5 ml of liquid LBS medium containing 25 mg/L kanamycin (AppliChem, A1493) and 50 mg/L rifampicin (Carl Roth, 4163.2). The bacterial culture was incubated at 28°C with continuous shaking. Absorbance or optical density of bacterial culture expressed in absorbance units (AU) was monitored in 1-ml aliquots of the culture using a spectrophotometer at 600 nm wavelength (OD600). The cell concentration estimated as a number of colony-forming units per milliliter of liquid culture (cfu/ml) can be analyzed at OD600 values 1; 1.3; 1.5; 1.7 and 1.8. For this purpose 250-µl aliquots of liquid culture were diluted with LBS-medium to achieve a final volume of 25 ml (dilution 1:100). 2.5 ml of such 1:100 dilution were mixed with 22.5 ml of LBS to achieve the dilution 1:1000. Liquid culture dilutions 1:100; 1:1,000; 1:10,000; 1:100,000; 1:1,000,000; 1:10,000,000 and 1:100,000,000 were prepared similarly. Aliquots of last three dilutions were spread on agar-solidified LBS medium supplemented with 25 mg/L kanamycin and 50 mg/L rifampicin (250 µl of bacterial culture per plate of 90 mm diameter). Plating of aliquots for each dilution was performed in triplicate. After 2 days incubation at 28°C, bacterial colonies were counted for each plate. Plating of 1: 1,000,000 and 1:10,000,000 dilutions resulted in few hundred and few dozen colonies per plate, respectively. So far as dilution 1:100,000,000 provided just few colonies per plate, this dilution was not used for calculation of cell concentration. The cell concentration was estimated according to the formula: cfu/ml = 4 x number of colonies per plate x dilution factor.

For transforming cell concentrations as measured by absorbance measurements at 600 nm (in LB medium) and in terms of cell-forming units, the following relation is used herein: an OD600 of 1.0 corresponds to 1.1 x10⁹ cfu/ml.

### Example 1: Vectors used in the following examples

Tobacco mosaic virus (TMV) based viral vectors with cell-to-cell movement ability designed as pro-vectors containing complementary parts of viral replicon and assembled vectors containing full viral replicon were made. They were created on the basis of vectors described in Marillonnet et al. (2004), Proc. Natl. Acad. Sci. USA 101 (18) 6852-685), WO 02/88369.

The 5'-module TMV pro-vectors have a *Arabidopsis* actin 2 promoter-driven TMV RNA-dependent RNA-polymerase (RdRp) containing 14 introns, TMV movement protein (MP) with 2 introns, and different fusion sequences followed by an intron 5' half part, an attP recombination site and a nopalin synthase (Nos) terminator. The entire fragment is cloned between the T-DNA left and right borders of binary vector. Fusion sequences include His(6)-tag with enterokinase cleavage site (His-EK) (pICH22455 construct, the nucleotide sequence of its T-DNA is given as SEQ ID NO: 1), rice α-amylase 3A apoplast targeting presequence (TP) (pICH20155), *Nicotiana plumbaginifolia* calreticulin apoplast TP (pICH20188), apple pectinase apoplast TP (pICH20388), barley α-amylase apoplast TP (pICH20999), *Nicotiana plumbaginifolia* calreticulin apoplast TP-His(6)-EK (pICH22464), artificial dicot chloroplast TP (pICH20030) and artificial dicot chloroplast TP-His-EK (pICH22474) (Fig. 2A).

In 3'-provector modules of TMV pro-vectors, an attB recombination site is followed by an intron 3' half part, the gene of interest coding sequences and a 3'-TMV non-translated region (3'NTR) as well as a nopalin synthase (Nos) terminator. The entire fragment is cloned between the T-DNA left and right borders of binary vector. Genes of interest used in these constructs encoded endoglucanase E1 from *Acidothermus cellulolyticus* (pNMD231; the nucleotide sequence of the T-DNA region is given as SEQ ID NO: 2), Exoglucanase 1 (CBHI) from *Trichoderma reesei* (pNMD241; the nucleotide sequence of the T-DNA region is given as SEQ ID NO: 3), exocellulase E3 from *Thermobifida fusca* (pNMD251; the nucleotide sequence of T-DNA region is given as SEQ ID NO: 4), β-glucosidase BGL4 from *Humicola grisea* (pNMD910; the nucleotide sequence of T-DNA region is given as SEQ ID NO: 5) and GFP (pICH7410) (Fig. 2B). Coding sequences of all genes of interest except GFP were codon-optimized for *Nicotiana benthamiana* and synthesized by Entelechon GmbH (Bad Abbach, Germany).

The integrase vector module pICH14011 harbors a heat-shock-promoter-driven *PhiC31* integrase gene with nuclear localization signal (NLS) and a Nos terminator inserted between left and right borders of the T-DNA (Fig. 2C). The nucleotide sequence of the T-DNA region is given as SEQ ID NO: 6.

Assembled TMV vectors contain Actin 2-promoter-driven TMV RdRp with 14 introns, TMV MP with 2 introns, fusion sequence, and coding sequence of gene of interest, 3'TMV non-translated region (3' NTR) as well as a Nos terminator. The entire fragment is inserted between the T-DNA left and right borders of binary vector. pNMD1201 (nucleotide sequence of its T-DNA is given as SEQ ID NO: 7), pNMD1213 and pNMD1192 constructs contain β-glucosidase BGL4 from *Humicola grisea* without any fusion sequence, BGL4 fused with N-terminal His-EK and BGL4 fused with artificial dicot chloroplast TP, respectively. The pNMD1231 construct bears endoglucanase E1 from *Acidothermus cellulolyticus* fused with rice α-amylase 3A apoplast TP. pNMD1181 contains Exoglucanase 1 (CBHI) from *Trichoderma reesei* fused with barley α-amylase apoplast TP. pNMD1161 (nucleotide sequence of its T-DNA is given as SEQ ID NO: 8), and pNMD1129 contain exocellulase E3 from *Thermobifida fusca* fused with apple pectinase apoplast TP and artificial dicot chloroplast TP-His-EK, respectively. The pNMD560 vector contains GFP ORF without any fusion sequences (Fig. 7).

### Details on the cellulolytic enzymes employed are as follows.

Endoglucanase E1 is from the thermophilic bacterium *Acidothermus cellulolyticus* 11B (Swiss-Prot: P54583; EC 3.2.1.4). It is a termostable enzyme with 81 °C temperature optimum and pH optimum from about 5 to about 6 (Himmel et., 1994; Sakon et al., 1996). It is a secreted protein with cleavable N-terminal signal peptide. The protein precursor consists of 562 amino acid residues; the signal peptide spans the first 41 amino acids; the 42-562 aa region corresponds to the mature protein. A nucleotide sequence corresponding to the mature part of the protein (42-662 aa) without native signal peptide was codon optimized for *Nicotiana benthamiana* and synthesized by Entelechon GmbH (Bad Abbach, Germany). This sequence was subcloned into TMV expression vectors.

Exoglucanase 1 (CBH1) is from the mesophilic fungus *Trichoderma reesei* (also known as *Hypocrea jecorina*) (Swiss-Prot: P62694; EC 3.2.1.91). It is a secreted protein with cleavable N-terminal signal peptide. The protein precursor consists of 513 amino acids, signal peptides spans first 17 amino acids. It is a mesophilic enzyme with 45-50°C temperature optimum and pH optimum 5 (Baker et al., 1998). The nucleotide sequence corresponding to the mature part of the protein (18-662 aa) without native signal peptide codon optimized for *Nicotiana benthamiana* and synthesized by Entelechon GmbH (Bad Abbach, Germany) was used for subcloning into TMV expression vectors.

Beta-1,4-exocellulase (E3) is from the thermophilic bacterium *Thermobifida fusca* (GenBank: AAA62211; EC 3.2.1.91). It is a secreted protein; the protein precursor consists of 596 amino acid residues, including 38 aa N-terminal signal peptide and the mature part (39-596 aa). E3 is thermostable, retaining full activity after 16 h at 55°C. It also has a broad pH optimum around 7-8, retaining 90% of its maximal activity between pH 6 and 10 (Zhang et al., 1995, 2000). The nucleotide sequence corresponding to the mature part of the protein (39-596 aa) without native signal peptide codon optimized for *Nicotiana benthamiana* and synthesized by Entelechon GmbH (Bad Abbach, Germany) was used for subcloning into TMV expression vectors.

β-glucosidase BGL4 is from the thermophilic fungus *Humicola grisea* (GenBank: BAA74958; EC 3.2.1.21). It is a 476 amino acids polypeptide. The literature describing BGL4 does not contain any indication about the presence of cleavable N-terminal signal peptide; in addition, TargetP 1.1 software (http://www.cbs.dtu.dk/services/TargetP/) did not identify any N-terminal signal peptide in this protein sequence. The BGL4 enzyme is thermostable, with a temperature optimum 60°C. The pH optima of this enzymes is 6.0, and it retains more than 80% of its relative activity within the range of pH 6.0-11.0 after 20 h at 4°C (Takashima et al. 1999). The nucleotide sequence corresponding to the full-length protein (39-596 aa) was codon optimized for *Nicotiana benthamiana* and synthesized by Entelechon GmbH (Bad Abbach, Germany). This sequence was used for subcloning into TMV expression vectors.

### Example 2: Targeting to the cell compartments improves the expression level and enzymatic activity of recombinant cell wall-degrading enzymes

Test on expression of cellulases in different cell compartments (cytosol, chloroplast and apoplast). For this purpose cellulolytic enzymes were expressed as translational fusions with corresponding targeting presequences using TMV pro-vector modules (Marillonnet et al., 2004, see above), WO 02/88369.

*Nicotiana benthamiana* plants were grown in the greenhouse (day and night temperatures of 19-23°C and 17-20°C, respectively, with 12 h light and 35-70% humidity). Six-week-old plants were used for inoculations.

For plant transfection, saturated *Agrobacterium* overnight cultures were adjusted to OD₆₀₀=1.3 (~1.2x10⁹ cfu/ml) with *Agrobacterium* inoculation medium (AIM: 10 mM MES pH 5.5, 10 mM MgSO₄). Further dilutions of cultures were done with AIM and inoculation was carried out by infiltration using a needleless syringe.

For screening of plant material expressing cellulase fusions to different targeting presequences for cellulase activity, a dilution mixture of *Agrobacterium* strains carrying 5'-TMV provectors (1 00-fold dilution with respect to the final mixture), 3'-TMV provectors (100-fold dilution) or integrase construct (10-fold dilution), respectively, was inoculated by needleless syringe into 3 leaves of different age of *Nicotiana benthamiana.* An overview over agrobacterial inoculations for the optimisation of transient expression of recombinant cellulases is shown in Fig. 3.

Inoculated *Nicotiana benthamiana* plants were incubated in a greenhouse under the conditions described above for 11 days. Variable phenotypes were observed in inoculation zones ranging from strong necrosis (*e.g*. endoglucanase E1 expressed in cytosol) to practically unaffected tissue (all β-glucosidase BGL4 fusions) (Fig. 4).

The leaf material from 3 leaves of different age was harvested using the cork borer at 7 to 11 dpi (days post inoculation) in triplicate samples, frozen in liquid nitrogen and stored at -80°C. Harvested plant material was analyzed using the SDS-PAGE and enzymatic activity assays.

### SDS-PAGE analysis with Coomassie staining

For SDS-PAGE, about 50 mg fresh weight *Nicotiana benthamiana* leaf material were ground in liquid nitrogen and crude protein extracts were prepared with 5 volumes of 2 x Laemmli buffer. Total soluble protein (TSP) was extracted in small-scale from -150 mg fresh weight plant material ground in liquid nitrogen with 5 volumes pre-chilled extraction buffer (EB: 50 mM sodium acetate pH 5.5, 100 mM NaCl, 10% (v/v) glycerol in water). For extraction, samples were vortexed after addition of EB and incubated on ice for 30 min with vortexing from time to time. Cell debris was separated by centrifugation for 10 min at 13000 rpm and 4°C twice.

The protein concentration of TSP extracts was determined by the Bradford assay using Bio-Rad Protein Assay (Bio-Rad Laboratories, GmbH, Munich, Germany) and Bovine Serum Albumin (Sigma-Aldrich, Co., St- Louis, USA) as a standard. For analysis by SDS-PAGE, Laemmli buffer was added to TSP samples with a final concentration of 1x.

Protein samples were denatured at 95°C for 5 min, separated in 10% polyacrylamide gel and stained using PageBlue™ Protein Staining Solution (Fermentas GmbH, St. Leon-Rot, Germany). An estimation of the percentage of recombinant cellulase fusion of TSP was done by comparison of TSP extracts to known amounts of BSA (Sigma-Aldrich) on Coomassie-stained polyacrylamide gels gels.

Fig. 5 summarizes the SDS-PAGE analysis of expression for different translational fusions of Endoglucanase E1 from *Acidothermus cellulolyticus,* Exoglucanase 1 (CBHI) from *Trichoderma reesei,* Exocellulase E3 from *Thermobifida fusca* and β-glucosidase BGL4 from *Humicola grisea.* Significant variation of the expression level depending on the targeting presequence was found. High expression was demonstrated for all β-glucosidase BGL4 fusions with highest accumulation in cytosol and chloroplast, although in the latter case the cleavage of the targeting presequence was not complete. In case of Endoglucanase E1, the highest accumulation was shown for apoplast targeted forms; accumulation in cytosol and chloroplast was lower. A similar expression pattern was found for Exoglucanase 1 (CBHI) with significant differences between tested apoplast targeting presequences. Surprisingly, CBHI expressed as a fusion with barley α-amylase apoplast TP was not detectable after the Coomassie staining. All Exoglucanase E3 fusions were well expressed with best accumulation in cytosol and chloroplast. Electrophoretic migration shift was found for apoplast targeted forms suggesting that these proteins are glycosylated.

### Enzymatic activity measurements

### Endoglucanase E1 and cellobiohydrolase CBHI cellulase activity assay

Enzyme activity was determined using p-nitrophenyl-β-D-cellobioside as substrate. 100 µg of plant TSP extracts were incubated in 50 mM NaAc pH 5.5, 100 mM NaCl, 5% (v/v) glycerol, supplemented with or without 5 mM p-nitrophenyl-β-D-cellobioside as enzyme blanks, respectively, in a final volume of 1 ml at 50°C for 1 to 24 hours. Aliquots of the reaction mixture were removed at different time points and diluted 1:10 with 0.15 M glycine pH 10.0 to terminate the reaction. The concentration of p-nitrophenol (pNP) released from p-nitrophenyl-β-D-cellobioside was determined by measurement of the absorbance at 405 nm. The commercial enzymes cellulase (endo-1,4-β-D-glucanase) from *Trichoderma* sp. (Megazyme, Bray, Ireland) and cellobiohydrolase (CBHI) from *Trichoderma* sp. (Megazyme) served as positive controls. Enzyme activities were expressed on a gram fresh weight plant material or a TSP basis.

### β-glucosidase Bgl4 cellulase activity assay

For determination of β-glucosidase activity, cellobiose was used as a substrate. Different protein amounts of plant TSP extracts ranging from 1-40 µg were incubated in 50 mM NaAc pH 5.5, 100 mM NaCl, 5% (v/v) glycerol supplemented with or without 5 mM cellobiose as enzyme blanks (mixture with substrate but without enzyme), respectively, in a final volume of 1 ml at 50°C for 30 min. The concentration of released glucose in the samples was determined by D-glucose (GOPOD-Format) Kit (Megazyme). The commercial enzyme cellobiase from *Aspergillus niger* (synonym: Novozyme 188; Sigma-Aldrich) served as positive control. β-glucosidase activity was calculated in cellobiase units (CBU) according to IUPAC or international units (IU) as described in Ghose *et al.,* 1987, and enzyme activities were expressed on a gram fresh weight plant material or a TSP basis.

### Exoglucanase E3 cellulase activity assay

For determination of exoglucanase activity, the insoluble microcrystalline cellulose substrate Avicel (Sigmacell cellulose type 20; Sigma-Aldrich) was used. 100 µg of plant TSP extracts were incubated in 50 mM NaAc pH 5.5, 50 mM NaCl, 5% (v/v) glycerol, 0.02% sodium azide, 1.2 mg/ml Novozyme 188 (β-glucosidase; Sigma-Aldrich), supplemented with or without 1% (w/v) Avicel as enzyme blanks, respectively, in a final volume of 3 ml at 50°C and 90 rpm for 120 hours. Aliquots of the reaction mixture were removed at different time points (2 h, 4 h, 24 h, 48 h, 72 h, and 120 h) for determination of the glucose concentration using D-glucose (GOPOD-Format) Kit (Megazyme). The commercial enzyme cellobiohydrolase (CBHI) from *Trichoderma* sp. (Megazyme) served as positive control. Enzyme activities were expressed on a gram fresh weight plant material or a TSP basis.

Analysis of the enzymatic activities of expressed translational fusions is summarized in Fig. 6. Highest β-glucosidase BGL4 activity was found in cytosol and chloroplast, which correlates with recombinant protein accumulation data (Fig. 6A). In the case of Endoglucanase E1, the highest activity was detected for apoplast targeted forms, again correlating with recombinant protein accumulation (Fig. 6B). The same was found for Exoglucanase CBHI (Fig. 6C). In contrast, no clear correlation with protein accumulation was found for Exoglucanase E3 suggesting that proper folding was critical for enzymatic activity (Fig. 6D). Cytosolic form with high expression provided relatively low enzymatic activity; highest activity was found for chloroplast targeted form with His-tag (high recombinant protein accumulation) and apoplast targeted form with presequence from apple pectinase (relatively low accumulation). Interestingly, poor enzymatic activity was found for chloroplast targeted form without His-tag.

Fusions providing highest enzymatic activity were selected for cloning of assembled vectors to be used for the preparative large-scale production of cell wall-degrading enzymes (Fig. 7). They include two forms of β-glucosidase BGL4 from *Humicola grisea* for cytosolic expression (without any presequence (pNMD1201) and with N-terminal His-EK (pNMD1213)) as well as BGL4 with chloroplast targeting presequence (pNMD1192). For Endoglucanase E1 from *Acidothermus cellulolyticus,* fusion with apoplast targeting presequence from rice α-amylase was selected (pNMD1231 construct). In the case of Exoglucanase 1 (CBHI) from *Trichoderma reesei,* it was the fusion with α-barley amylase apoplast TP (pNMD1181 construct). Two constructs were created for *Thermobifida fusca* exocellulase E3 containing fusions with apple pectinase apoplast TP and chloroplast TP followed by His-EK (pNMD1161 and pNMD1129, respectively). In addition, the pNMD560 construct containing the GFP coding sequence insertion was used as an agrobacterial transfection control.

### Example 3: Spraying with agrobacteria provides similar expression levels of cell wall-degrading enzymes as infiltration

We analysed two different inoculation methods for highest cellulase expression of selected cellulase fusions: syringe infiltration vs. spraying with suspensions of agrobacteria harboring same assembled TMV vectors. For plant transfection, *Agrobacterium* overnight cultures (OD₆₀₀=1.3) were diluted with *Agrobacterium* inoculation medium (AIM, 10 mM MES pH 5.5, 10 mM MgSO₄). For spraying, 0.1% Silwet L-77 (v/v) (Kurt Obermeier GmbH & Co. KG, Bad Berleburg, Germany) was added to the solution. Dilutions of agrobacterial cultures 1:100 and 1:1000 were used for syringe infiltration and spraying, respectively, as providing the highest expression (data not shown). Six week old *Nicotiana benthamiana* plants were used for inoculation with agrobacterial cultures. Plant material was harvested at 4, 7, 11 and 14 dpi (days post infection) and used for the SDS-PAGE analysis and evaluation of enzymatic activity as described in Example 2.

SDS-PAGE analysis (Fig. 8) demonstrated that for most cellulase fusions, syringe infiltration and spraying with agrobacteria provide comparable expression levels. Few days delay of recombinant protein accumulation was observed for spraying delivery, which can be explained by the lower number of leaf cells initially targeted by *Agrobacterium.* Cytosolic β-glucosidase BGL4 from *Humicola grisea* expressed from an assembled TMV vector upon syringe inoculation of a 1:100 dilution of *Agrobacterium* culture (7 dpi) and spray inoculation of a 1:1000 dilution (11 dpi) is estimated to form about 55% and 50% of TSP, respectively. Similarly, chloroplast targeted BGL4 formed about 75% TSP after the infiltration and 60% of TSP after the spraying. In case of Exoglucanase 1 (CBHI) from *Trichoderma reesei* expressed as a fusion with barley-α-amylase apoplast TP, corresponding values were 6.5% and 3.5% of TSP. Chloroplast TP-His-EK-*Thermobifida fusca* exocellulase E3 fusion formed about 30% and 25% of TSP, respectively; and apple pectinase apoplast TP-E3 fusion provided about 7% and 2,5% of TSP, respectively. More significant difference was found for apoplast targeted Endoglucanase E1 from *Acidothermus cellulolyticus* (fusion with rice α-amylase) where syringe infiltration resulted in recombinant protein accumulation about 15% of TSP and by spraying of about 2% TSP.

Enzymatic activities of all four enzymes calculated on the basis of g fresh weight of plant material correlated with recombinant protein expression data (Fig. 9). BGL4, CBHI and E1 provided comparable enzymatic activities after infiltration and spraying; E3 activity after the spraying was lower.

### Example 4: Storage of cellulase-containing plant biomass as a silage prevents degradation of recombinant cellulase proteins and maintains their enzymatic activity

Plant material inoculated with 1:1000 dilutions of *Agrobacterium* cultures carrying assembled TMV constructs was harvested 11 dpi as a pool of plants. Midribs of leaves were removed and leaf blades were chopped into pieces of about 5 cm² in size using a scalpel blade. Leaf pieces were spread out and dehydrated either at 55°C for 20 h for storage as dry mass, or for 22 h at room temperature (RT) for storage as silage. Dehydrated leaf material (45-58% of fresh weight) was supplemented with 2% (w/dry weight) propionic acid, sodium salt (AppliChem, Darmstadt, Germany) and packed into plastic bags using a commercial vacuum food sealer (Food Saver V2040-I, Sunbeam Products, Inc., Boca Raton, USA) for conservation. This plant material was stored as silage at RT for 1-4 months.

150 mg fresh weight aliquots (9 leaf discs of 1 cm in diameter pooled from 3 comparable leaves) were harvested in triplicates (3 samples from leaves of different age) from identical plant material used for the preparation of silage. Samples were frozen in liquid nitrogen and stored at -80°C.

Silage stored for up to 16 weeks looked brown and had a pH of about 5.3. The determination of protein concentration of TSP samples revealed that TSP samples prepared from silage or from plant material dried at 55°C contained a reduced amount of extractable protein compared to plant material stored at -80°C.

For the analysis of β-glucosidase activity, 1, 2.5, 5 and 25 µg of TSP samples were incubated with 50 mM NaAc pH 5.5, 100 mM NaCl, 5% (v/v) glycerol, 5 mM cellobiose at 50°C for 30 min. The release of glucose was quantified using the GOPOD Kit (Megazyme) and used to calculate enzymatic activity in cellobiase units (CBU) or international units (IU) per mg protein amount analyzed or in CBU and IU per g fresh weight plant material or g commercial enzyme. Due to the low protein amount extractable from dried plant material, only 5 µg TSP of these samples could be analyzed which show only weak enzymatic activities (data not shown).

β-glucosidase activities of TSP samples prepared from plant material upon a storage period of 4, 8 or 16 weeks are compared in Fig. 10A and are very similar, suggesting that the extension of the storage period seems to have no significant negative effect on recombinant BGL4 cellulases. SDS-PAGE demonstrated that recombinant BGL4 remained stable in silage (Fig. 10B and C); in the same time period, native plant protein degrades nearly completely. The recovery of BGL4 proteins from dry biomass was very low due to the poor degradation or poor extractability.

For cytosolic BGL4, the percentage content in TSP extracted from plant material stored at -80°C was estimated to 60%, whereas the percentage content in TSP extracted from silage was 95%. For chloroplast targeted BGL4, the corresponding values are 60% and 90%, respectively (Fig. 10D). Such increase in the content in TSP observed during silage storage can be explained by degradation of native plant proteins.

*Acidothermus cellulolyticus* Endoglucanase E1 and *Trichoderma reesei* Exoglucanase 1 (CBHI) fusions remain stable and retain their enzymatic activity during storage of plant biomass as a silage as shown in Fig. 11A-D. Again, the percentage content of CBHI in TSP extracted from silage (35%) is higher than in TSP extracted from material stored at -80°C (12.5%) (Fig. 11E).

Good protein stability during storage of plant biomass as a silage was observed also for recombinant exocellulase E3 from *Thermobifida fusca* (Fig. 12A-C). For E3 fusion with apple pectinase apoplast TP, the percentage content in TSP extracted from plant material stored at -80°C was estimated to 5% and 18% in TSP extracted from silage. For chloroplast TP-His-EK-E3 fusion these values are 25% and 15%, respectively (Fig. 12D).

### Example 5: Mixture of plant-produced recombinant cellulases efficiently convert cellulose to glucose

In order to analyze the total enzymatic activity of a mixture of different cellulase classes expressed *in planta* on the conversion of cellulose to glucose, *N. benthamiana* plants were inoculated for the production of cellulases by spraying with 1:1000 dilutions of *Agrobacterium* cultures carrying assembled TMV vectors. For this experiment, each one fusion for ß-glucosidase BGL4 (cytosolic), Endoglucanase E1 (fusion with rice α-amylase apoplast TP), exoglucanase CBHI (fusion with barley α-amylase apoplast TP) and exoglucanase E3 (chloroplast TP-His-EK fusion) was chosen on the basis of the following criteria: highest expression *in planta* and highest enzymatic activity (also upon storage of plant material).

To evaluate the total cellulase activity, the percentage of recombinant cellulases in TSP extracts was estimated and a mixture of 20 µg endo- and exocellulases (E1, CBHI and E3 in variable amounts) and 100-1000 µg β-glucosidase (Bgl4) was incubated in 50 mM NaAc pH 5.5, 80 mM NaCl, 8% (v/v) glycerol, 0.02% sodium azide, supplemented with or without 1% (w/v) Avicel as enzyme blanks, respectively, in a final volume of 5 ml at 50°C and 90 rpm in a shaker for 144 hours. Aliquots of the reaction mixture were removed at different time points (2 h, 24 h, 48 h, 72 h, and 144 h) for determination of the glucose concentration using D-glucose (GOPOD-Format) Kit (Megazyme). The activity of the cellulase mixtures was calculated as mg glucose release per mg TSP (Fig. 13A) or per a gram fresh weight plant material (Fig. 13B).

Samples containing 20 µg of a mixture of rice amylase apoplast TP-E1, barley α-amylase apoplast TP-CBHI and chloroplast-TP-His-EK-E3 and 500 µg Bgl4 (ratio (1:1:0):25; (2:1:1):25; and (2:2:1):25)) showed the highest total glucose release. Calculations of cellulase activity as glucose release in mg glucose/mg TSP or mg glucose/g fresh weight plant material indicates highest enzymatic activity for 20 µg of a mixture of rice amylase apo TP-E1, barley α-amylase apo TP-CBHI and chloroplast-TP-His-EK-E3 and 100 µg of Bgl4 (ratio (1:1:1):5) and no significant influence of the alteration of the ratios between endo- and exoglucanases on the amount of glucose production (ratio (1:1:1):25; (2:1:1):25; (1:2:1):25; (1:1:2):25 or (2:2:1):25). A 2:2:1 ratio for E1, CBHI and E3 gave highest sugar release in the analysis of Baker *et al.,* 1998, here a 2:1:1 ratio for E1, CBHI and E3 gives at least no significantly higher glucose production when calculated per g of fresh weight plant material or mg TSP. In summary for this experiment, using cellulases expressed *in planta,* the most efficient cellulose degradation is expected to be observed for a mixture of E1, CBHI, E3 and Bgl4 of (1:1:1):5 or (2:1:1):5 when referred to g of fresh weight plant material.

Concerning the amount of total glucose release, the cost effectiveness of a larger excess of Bgl4 should be taken into consideration, as the release of glucose is increased for samples with ratios (1:1:1):10, (1:1:1):25 and (1:1:1):50 compared to (1:1:1):5 (to minimize a putative inhibition of endo- and exoglucanases by cellobiose and for complete conversion of cellobiose to glucose). The concrete composition of a quarternary mixture of cellulases analyzed giving the highest glucose production will presumably vary with the degree of variation of enzyme expression *in planta* and the estimation of percentage of TSP.

### References

Baker, J. O., Ehrman, C.I., Adney, W.S., Thomas, S.R. & Himmel, M.E. (1998) Hydrolysis of cellulose using ternary mixtures of purified celluloses. Appl Biochem Biotechnol 70-72: 395-403.
Himmel, M. E., Adney, W. S., Grohmann, K., & Tucker, M. P. (1994) Thermostable purified endoglucanase from *Acidothermus cellulolyticus* ATCC 43068. U.S. Patent 5.275.944. Sakon, J., Adney, W.S., Himmel, M.E., Thomas, S.R. & Karplus, P.A.(1996) Crystal structure of thermostable family 5 endocellulase E1 from Acidothermus cellulolyticus in complex with cellotetraose. Biochemistry 35(33):10648-60.
Shoemaker, S., Schweickart, V., Ladner, M., Gelfand, D., Kwok, S., Myambo, K. & Innis, M. (1983) Molecular cloning of Exo-Cellobiohydrolase I derived from Trichoderma reesei strain L27. Nature Biotechnology, 1: 691-696.
Takashima S, Nakamura A, Hidaka M, Masaki H, Uozumi T. (1999) Molecular cloning and expression of the novel fungal beta-glucosidase genes from Humicola grisea and Trichoderma reesei. J. Biochem 125, 728-736.
Zhang, S., Lao, G. & Wilson D.B. (1995) Characterization of a Thermomonospora fusca exocellulase. Biochemistry, 34 (10): 3386-95.
Zhang, S., Barr, B.K., & Wilson, D.B. (2000) Effects of noncatalytic residue mutations on substrate specificity and ligand binding of Thermobifida fusca endocellulase cel6A. Eur J Biochem. 67(1): 244-52.
Ghose, T. K. (1987) Measurement of cellulase activities. Pure & Appl. Chem. 59: 257-268.

## Claims

1. A process of producing a preparation of one or more than one cell wall-degrading enzyme, comprising:
(i) expressing in plants or parts thereof one or more than one heterologous cell wall-degrading enzyme;
(ii) harvesting said plants or parts containing said one or more than one heterologous cell wall-degrading enzyme; and
(iii) storing the harvest as a silage.

2. A process of producing a preparation of cell wall-degrading enzymes, comprising:
(i) transfecting plants or parts thereof with a nucleic acid molecule comprising a nucleic acid construct containing a nucleotide sequence encoding one or more than one cell wall-degrading enzyme and expressing in said plants or parts thereof said one or more than one cell wall-degrading enzyme;
(ii) harvesting said plants or parts containing said one or more than one cell wall-degrading enzyme; and
(iii) storing the harvest as a silage.

3. The process according to claim 1 or 2, wherein said one or more than one cell wall-degrading enzyme is selected from cellulolytic enzymes such as an endoglucanase, an exoglucanase, an exocellulase, a β-glucosidase, or combinations thereof; from hemicellulases; pectinases; and/or lignin degrading enzymes.

4. The process according to any one of claims 1 to 3, wherein said one or more than one cell wall-degrading enzymes are secretory enzymes in the organism from which they are derived.

5. The process according to any one of claims 1 to 4, wherein step (iii) comprises storing said harvested plants or parts thereof for at least 1 month in an atmosphere of reduced oxygen.

6. The process according to any one of claims 1 to 5, wherein step (iii) comprises lactic acid bacteria fermentation and/or acidification by addition of an acid to produce acidic conditions in said silage.

7. The process according to any one of claims 1 to 6, wherein said cell wall-degrading enzymes are expressed in the cytosol of cells of said plants or parts thereof, or said cell wall-degrading enzymes are targeted to the apoplast or to plastids of said plant or plant parts.

8. The process according to any one of claims 1 to 7, wherein step (i) comprises spraying aerial parts of said plant with an aqueous suspension containing cells of an *Agrobacterium* strain comprising a DNA molecule comprising a nucleic acid construct containing a DNA sequence encoding said one or more than one cell wall-degrading enzyme.

9. The process according to any one of claims 1 to 7, wherein step (i) comprises spraying aerial parts of said plant with an aqueous suspension containing cells of an *Agrobacterium* strain comprising a DNA molecule comprising a nucleic acid construct encoding a DNA or RNA replicon encoding said one or more than one cell wall-degrading enzyme.

10. A process of storing cell wall-degrading enzymes, comprising storing, in a silage, plants or plant parts containing one or more than one expressed enzyme capable of degrading a plant cell wall component, said plant or plant parts preferably containing said enzyme(s) targeted to the apoplast or to plastids of said plant or plant parts.

11. Silage containing one or more than one active cell wall-degrading enzyme capable of degrading a plant cell wall component, said silage obtainable by fermenting plants or plant parts containing expressed one or more than one cell wall-degrading enzyme that may be heterologous to said plants or said part.

12. A process of degrading plant cell wall components of plant material, comprising treating said plant material in an aqueous reaction medium with the silage of claim 11 or with the preparation obtained or obtainable according to any one of claims 1 to 9.

13. The process according to claim 12, wherein said silage contains cell wall-degrading enzymes selected from endoglucanases, exoglucanases, exocellulases, hemicellulases, β-glucosidases, pectinases, lignin-degrading enzymes and combinations thereof.

14. The process according to claim 12 or 13, wherein a silage containing an endoglucanase, a silage containing an exoglucanase, a silage containing an exocellulase, and a silage containing a β-glucosidase are mixed in desired ratio before treatment of said plant material to obtain a desired ratio of cellulolytic enzymes, and treating said plant material in an aqueous reaction medium with the mixtures of silages.

15. A process of producing a bioalcohol such as bioethanol from plant material, comprising:
(a) treating said plant material, or a product obtained by pre-treatment of said plant material, in an aqueous reaction medium with the silage of claim 11 or with the preparation obtained or obtainable according to any one of claims 1 to 9 for producing glucose from cellulose contained in said plant material,
(b) fermenting the product obtained in the step (a) for producing said bioalcohol from said glucose,
wherein steps (a) and (b) may be performed simultaneously in the same reaction vessel or consecutively.
